(19) Europäisches Patentamt
European Patent Office
Office européen des brevets

(11) EP 4 206 327 A1

(12) **EUROPEAN PATENT APPLICATION**
published in accordance with Art. 153(4) EPC

(43) Date of publication:
**05.07.2023 Bulletin 2023/27**

(21) Application number: **21855623.1**

(22) Date of filing: **13.08.2021**

(51) International Patent Classification (IPC):
***C12N 15/863*** *(2006.01)* ***A61K 35/768*** *(2015.01)*
***A61P 35/00*** *(2006.01)*

(52) Cooperative Patent Classification (CPC):
**A61K 35/768; A61P 35/00; C12N 15/863**

(86) International application number:
**PCT/CN2021/112408**

(87) International publication number:
**WO 2022/033573 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.08.2020 CN 202010813096**

(71) Applicant: **Suzhou Prajna Biotech Co., Ltd.**
**Suzhou, Jiangsu 215000 (CN)**

(72) Inventor: **HU, Minjie**
**Suzhou, Jiangsu 215000 (CN)**

(74) Representative: **Finnegan Europe LLP**
**1 London Bridge**
**London SE1 9BG (GB)**

Remarks:
The complete document including Reference Table(s) and the Sequence Listing(s) can be downloaded from the EPO website

# (54) MUTANT OVIS SPP. INFECTIOUS PUSTULAR DERMATITIS VIRUS AND USE THEREOF

(57) Provided are the use of a mutant Ovis spp. infectious pustular dermatitis virus (ORFV) and a pharmaceutical composition thereof in the treatment of cancer.

EP 4 206 327 A1

## Description

RELATED APPLICATIONS

This application claims priority to China Provisional Application Ser. No. 202010813096.9 filed August 13, 2020.

FIELD

The present invention relates to the biotechnology field, more specifically, relates to mutant Orf viruses and uses thereof as pharmaceutical composition in treating cancers.

BACKGROUND OF THE INVENTION

The Orf virus (ORFV), is a member of the Parapoxvirus (PPV) genus in the Poxviridae family. The PPV genus includes other members, such as the Bovine papular stomatitis virus, Pseudocowpox virus, Parapoxvirus of red deer in New Zealand, Sealpox and other viruses. The ORFV can cause contagious, and epitheliotropic infections in sheep and goats. When animals are infected by an ORFV, erythema initially emerges on the lips, tongue, nose, breast et al, and subsequently progresses to papule, vesicle, pustule, and scab, which are often characterized by proliferative skin inflammation.

Patent CN 104017776 B discloses an attenuated ORFV , obtained by isolation and cell subculture, for vaccine development. Patent CN 108026542 A describes the use of the viral strain D1701 to make a recombinant ORFV vector for expressing viral and tumor antigens,etc., for the preparaton of vaccines.

The oncolytic viral therapy is a type of the systemic therapy that employs naturally existing or engineered viruses to treat cancers. The special gene mutations that originally promote the proliferation and survival of tumor cells, but also promote the growth of the viruses having oncolytic functionality (oncolytic virus, OV) within the tumor cells, which is one of the reasons an OV can precisely attacks tumor cells [1, 2, 3]. Following infection, an oncolytic virus promotes tumor cell lysis and death at a suitable time point within its viral life cycle, leading to a release of infectious viral virions, further infecting neighboring tumor cells. At the same time, neoantigens released during oncolysis activate the host immune system, and reattack the tumor the second time [4]. Currently, more than 90 ongoing or completed clinical trials using oncolytic viruses for the treatment of human malignancies have been documented worldwide. Among them, TVEC ((talimogene laherparepvec) has been successfully approved by FDA [5].

ORFV is a double-stranded DNA virus with a genome of 134-139kb [6]. It is an ovoid in shape, resembling a ball of yarn, and measures approximately 230-280mn (long) $\times$ 150-200nm (wide). ORFV has the following characteristics: 1) an ORFV, like other members of the poxviridae family, only replicates in the cytoplasm of a host cell and does not enter the nucleus, therefore (its DNA) will not integrates into the host cell's genome, so, its safety is high and its probability of carcinogenicity is extremely low. [7, 10]; 2) an ORFV could infect a human with broken or scarified skin through contact of the infected area of an animal, producing clinically mild pustules at the site of infection (usually on fingers) with basically no other adverse effects and life-threatening conditions, and resolving spontaneously within 6-8 weeks [8, 11]; 3) ORFV infection does not rely on specific cell surface receptors [9, 10], and therefore could provide a way to overcome the heterogenicity problem of a solid tumor; 4) an ORFV has a large genome, which is conducive to the insertion and efficient expression of foreign genes; 5) ORFV's viral genome is stable and the fidelity of replication is high; 6) little or no neutralizing antibodies against an ORFV are produced in the host, so multiple infections can occur[11], and repeated intravenous injections of an ORFV drug into cancer patients can be achieved; 7) ORFV infection can induce both innate, and adaptive anti-tumor immune responses within a host [12]; 8) Vaccinia, another member of the poxvirus family, has been used as a vaccine against smallpox infection in billions of people worldwide, setting the basis for low risks in future clinical applications of an ORFV drug; 9) an ORFV has the capability of turning a "Cold" tumor into a "Hot" one [18], which is ORFV's own properties. Based on these special characteristics discussed above, ORFV will likely become a new type of an oncolytic virus to treat solid tumors.

An ORFV viral strain usually has 130-134 (protein encoding) genes. The currently confirmed virulence factors include the orf virus interferon-resistance factor (OVIFNR, ORFV020) , the chemokine binding protein (CBP, ORFV112) , the inhibitor of granulocyte-monocyte colony-stimulating factor and IL-2 (GIF, ORFV117) , the viral interleukin 10 homologue (vIL-10, ORFV127) , the vascular endothelial growth factor (VEGF-like protein, ORFV132 etc [11]. These viral gene-encoded proteins could also modulate the host's immune response [13].

The ORFV002 gene is a late viral gene, and (its product) is localized in the nucleus following protein synthesis, inhibiting the NF-κB pathway in the nucleus. It is identified as the first NF-κB inhibitor produced by the ORFV virus. [19]

The ORFV005 gene encodes a hypothetical protein whose mechanism of action is currently unclear.

The ORFV007 gene is about 483bp and encodes deoxyuridine pyrophosphatase (dUTPase) [15]. Its protein is an important enzyme in the biosynthesis of dNTPs (deoxyribonucleoside triphosphate, dUTPase is involved in the major biosynthesis pathway to dTTP, a type of dNTP). In general, the concentration of dNTPs in normal cells is strictly regulated, which is not conducive to viral replication. In cancer cells, however, there are higher concentrations of dNTPs, which favors viral replication [16]. ORFV007 deletion will restrict viral replication in normal cells, but not in cancer cells, therefore achieving the goal of selective replication of an ORFV in cancer cells.

The ORFV111 gene encodes for a hypothetical protein whose mechanism of action is currently unclear.

The ORFV112 gene encodes the chemokine-binding protein (CBP) and is about 864 bp long. It interferes with the anti-viral mechanism of host immune cells [14]. This protein is similar in structure and function to the CBP-II protein of other pox viruses, which can not only inhibit the migration of DC cells to the inflammatory site, but also prevent DC cells from activating T cells [14].

Currently, the ORFV viral strains that have been disclosed and used in oncolytic virus research include NZ-2, NZ-7, D1701, NA1/11, etc. In prior art, due to its capacity for inserting a relatively large foreign DNA fragment, an ORFV is generally used as a vector to insert the genes encoding tumor-specific antigens or viral antigens, cytokines, etc., to create a recombinant virus for research.

Patent WO 2012122649A1 disclosed a recombinant ORFV virus with an insertion of the vaccinia virus E3L gene, infecting tumor cells containing specific host-range genes (SPI-1 , K1L, C7L, BSR, p28/N1R, E3L etc.) , for anti-cancer drug development. Patent CN 108220251A disclosed a recombinant ORFV and its preparation method and use, mainly knocking out the ORFV132 gene (VEGF) of the ORFV NA1/11 strain and inserting the P53-EGFP fusion protein gene into the same site, for cancer drug development. Neither the absence of the ORFV112 gene nor the absence of the ORFV007 gene have been reported in the strains of the two patents said above.

The present invention discloses the mutated ORFV viruses, characterized by the absence of the functionally expressed products of the ORFV112 and/or the ORFV111 gene, wherein absence of the functionally expressed products is due to a complete or partial deletion of the gene ORFV 112 encoding CBP and/or the gene ORFV111 encoding a hypothetical protein. The inventors (of the present disclosure) fortuitously discovered that complete or partial absence of the gene ORFV 112 and/or the gene ORFV111 said above resulted in distinctly enhanced antitumor activities by the virus. Furthermore, genome sequence comparison between these mutated type ORFV viruses and all other disclosed ORFV viral strains revealed a complete loss of the dUTPase-encoding gene ORFV 007 of the mutant ORFV viruses said above. Further, the comparison said above also confirmed the complete absence of the ORFV002 and ORFV005 genes of the said mutant ORFV viruses.

Therefore, the mutant Orf viruses in the present disclosure provide a guiding role in anti-cancer drug development for this kind of viruses.

## SUMMARY OF THE INVENTION

Inventors (of the present disclosure) fortuitously discovered that the complete or partial absence of gene ORFV112 (encoding CBP protein) and/or gene ORFV111 (encoding a hypothetical protein, from now on referred to as the hypothetical protein 111), especially the absence of the 5' end of ORFV112 and the 3' end of ORFV111, leads to enhanced antitumor activity of the mutant Orf viruses (ORFVs). Furthermore, the inventors also fortuitously discovered that the aforementioned ORFV viruses showed a complete absence of gene ORFV 007. Mutant Orf viruses without functional CBP and/or hypothetical protein 111 can be obtained using standard molecular biology methods (such as molecular cloning, DNA recombination, homologous recombination, PCR, restriction nuclease (digestion), gene knockout, gene silencing, etc.) or emerging molecular biology techniques (e.g., gene editing, etc.).

In one aspect, the present invention discloses a mutant Orf virus characterized by absence of the functionally expressed products of genes ORFV112 and/or ORFV111. In one embodiment, absence of the ORFV112 gene functionally expressed product is caused by complete or partial (e.g., the 5' end or 3' end, especially the 5' end) deletion of the gene ORFV112. In one embodiment, the mature protein sequence of the expressed product of gene ORFV112 is shown in SEQ ID NO:2. In one embodiment, the full sequence of gene ORFV112 is shown in SEQ ID NO:3. In one embodiment, the sequence of SEQ ID NO:3 is completely deleted. In one embodiment, the sequence of SEQ ID NO: 3 is partially deleted, for example, nucleotides 1-1161, 1-1140, 1-538, 539-1139, or 1141-1160 at the 5' end are deleted. In one embodiment, the partially deleted gene ORFV112 has a sequence of SEQ ID NO: 4 or SEQ ID NO: 57. In one embodiment, absence of the functionally expressed ORFV111 gene product is resulted from partial or complete sequence deletion of the ORFV111 gene (e.g., 5' end or 3' end, especially 3' end). In one embodiment, the sequence of the expressed product of gene ORFV111 is shown in SEQ ID NO: 58. In one embodiment, the full sequence of gene ORFV111 is shown in SEQ ID NO: 59. In one embodiment, the sequence of SEQ ID NO: 59 is completely deleted. In one embodiment, the sequence of SEQ ID NO: 59 is partially deleted. For example, nucleotides 1-793, 1-309, or 310-792 at the 3' end are deleted. In one embodiment, the partially deleted gene ORFV111 has a sequence of SEQ ID NO: 60. In one embodiment, the sequences of SEQ ID NO: 3 and SEQ ID NO: 59 are completely deleted. In one embodiment, the expressed product is a protein and/or a nucleic acid (especially a functional nucleic acid). In one embodiment, the present invention provides the genomes of the aforementioned ORFV viruses.

In one aspect, the present invention discloses a method to modify Orf viruses, comprising reducing or eliminating the expression and/or activity of the expressed products of gene ORFV112 and/or gene ORFV111. In one embodiment, the method includes complete or partial (e.g., at the 5' or 3' end, especially the 5' end) deletion of the ORFV112 gene. In one embodiment, the mature protein sequence of the expressed product of gene ORFV112 is shown in SEQ ID NO:2. In one embodiment, the full sequence of gene ORFV112 is shown in SEQ ID NO:3. In one embodiment, the method comprises completely deleting the sequence of SEQ ID NO: 3. In one embodiment, the method comprises partially deleting the sequence of gene ORFV112 (SEQ ID NO:3), such as deleting nucleotides 1-1161, 1-1140, 1-538, 539-1139, or 1141-1160 at the 5' end. In one embodiment, the method generates a partially deleted gene ORFV112 having a sequence of SEQ ID NO: 4 or SEQ ID NO: 57. In one embodiment, the method comprises complete or partial (e.g., the 5' or 3' end, especially the 3'end) deletion of gene ORFV111. In one embodiment, the sequence of the expressed product of gene ORFV111 is shown in SEQ ID NO: 58. In one embodiment, the full sequence of gene ORFV111 is shown in SEQ ID NO: 59. In one embodiment, the method comprises completely deleting the sequence of SEQ ID NO: 59. In one embodiment, the method comprises partially deleting the sequence of SEQ ID NO: 59, for example, deleting nucleotides 1-793, 1-309, or 310-792 at the 3' end. In one embodiment, the method generates a partially deleted gene ORFV111 of SEQ ID NO: 60. In one embodiment, the method comprises completely deleting the sequences of SEQ ID NO: 3 and SEQ ID NO: 59. In one embodiment, expression is transcription and/or translation. In one aspect, the present invention provides viruses obtained by the method described herein. In one embodiment, this invention provides the genomes of the ORFV viruses described herein. The methods described in the present invention could be carried out by conventional molecular biology techniques (e.g., molecular cloning, DNA recombination, homologous recombination, PCR, restriction nucleases, gene knockout, silencing, etc.) or by emerging molecular biology techniques (e.g., gene editing, etc.).

In another aspect, the present invention provides methods for identifying the aforementioned mutant Orf viruses and/or their genome, in particular methods for detecting gene ORFV112 and/or ORFV111 (its presence or sequence) and/or their expression products (presence and/or activities). The methods described in the present invention could be carried out by detecting the presence and/or activities of proteins and/or the presence and/or sequence of nucleic acids through standard molecular biology techniques. The standard techniques include activity assays, blottings (such as Southern blot, Northern blot, and Western blot), restriction endonuclease (digestion), PCR, electrophoresis (e.g., gel electrophoresis, including protein electrophoresis and nucleic acid electrophoresis, including agarose electrophoresis and PAGE electrophoresis, including reducing and non-reducing electrophoresis), sequencing (including protein sequencing and nucleic acid sequencing), or emerging molecular biology technologies (e.g., next-generation sequencing, etc.). In particular, the present invention provides methods for detecting the integrity of the gene ORFV112 and/or gene ORFV111. In one embodiment, the methods can be carried out by PCR and gel electrophoresis. In one embodiment, the methods can be carried out by hybridization or sequencing.

In another aspect, the present invention provides a method to treat cancers in subjects using the said mutant Orf viruses and/or their genomes. In another aspect, the present disclosure provides the use of the said mutant Orf viruses and/or their genomes to treat cancers in subjects. In another aspect, the present disclosure provides the use of the said mutant Orf viruses and/or their genomes to prepare a drug for treating cancers in subjects. In another aspect, the present disclosure provides the said mutant Orf viruses and/or their genomes for treating caners in subjects. In another aspect, the present disclosure provides the said mutant Orf viruses and/or their genomes for preparing cancer drugs for treating cancers in subjects. In one embodiment, the cancer is a solid tumor. In one embodiment, the said solid tumor is cervical cancer, bladder cancer, liver cancer, ovarian cancer, melanoma, colorectal cancer, lung cancer, breast cancer, gastric cancer, uterine cancer, head and neck cancer, thyroid cancer, esophageal cancer, prostate cancer, pancreatic cancer, sarcoma, a brain tumor, etc. In one embodiment, the said subject is mammal, including rodent (e.g., mice and rats), non-human primate (e.g., cynomolgus monkeys), and human.

## FIGURE DESCRIPTION

Figure 1 shows the results of agarose gel electrophoresis of PCR products after PCR amplification using ORFV112 gene-specific forward primer and reverse primer (SEQ ID NO: 5 and 6). In the figure, sample 1 is (from) the POV-601-1A1 viral strain with the ORFV112 gene deletion, sample 2 is (from) the POV-601-3F8 viral strain with the complete ORFV112 gene, and M is DNA size marker.

Figure 2 shows the anti-tumor inhibitory effects of the ORFV112 gene-deleted viral strains (viruses) of POV-601-1A1and POV-604-1D1 in the MB49 murine bladder cancer model.

Figure 3 compares the anti-tumor inhibitory effects of the ORFV112 gene-deleted viral strains (viruses), POV-601-1A1 and POV-604-1D1, with the ORFV112 gene-intact strain, POV-601-3F8, in the B16-F10 murine melanoma tumor model.

Figure 4 shows the influence of the POV-601-1A1 viral strain on mouse body weight under different doses and routes of administration. (i.v., intravenous, s.c., subcutaneous).

Figure 5 shows immune system changes in mice with human-derived C-33A cervical cancer bilateral tumors following administration of the POV-601-1A1 viral strain. In Figs. 5A and 5B, the squares represent the results of intratumoral administration on the right side, and the circles represent the results of unadministered mice on the left side. The activation ratio of CD45+ and NK cells in the tumor is increased in mice injected with POV-601-1A1. Figure 5C shows the activation of NK cells in the blood, and the ratio of activated NK cells is increased by intravenous administration.

Figure 6 shows a comparison of the gene ORFV112 of the present invention between the complete nucleotide sequences and that of the partial deletion.

Figure 7 shows the alignment of the complete sequences of the CBP protein of the present invention with that of the CBP proteins of other parapox virus strains, where "3F8" stands for POV-601-3F8 Strain, and "B029" for ORFV Strain B029, "GO" for ORFV Strain GO, "NA11" for ORFV Strain NA1/11, "NZ2" for ORFV Strain NZ2, "NA17" for ORFV Strain NA17, "OV-SA00" for ORFV Strain OV-SA00, "OV-IA82" for ORFV Strain OV-IA82, "SJ1" for ORFV Strain SJ1, "SY17" for ORFV Strain SY17, "OV-NH3_12" for ORFV Strain OV-HN3/12, "NP" for ORFV Strain NP, and "YX" for ORFV Strain YX.

Figure 8 shows the nucleotide sequence comparison of the ORFV112 gene of the ORFV viral strains POV-601-3F8 of the present invention with that of other parapox virus strains, where "3F8" stands for POV-601-3F8 Strain, "B029" for ORFV Strain B029, "GO" for ORFV Strain GO, "NA11" for ORFV Strain NA1/11, "NZ2" for ORFV Strain NZ2, "NA17" for ORFV Strain NA17, "OV-SA00" for ORFV Strain OV-SA00, "OV-IA82" for ORFV Strain OV-IA82, "SJ1" for ORFV Strain SJ1, "SY17" for ORFV Strain SY17, "OV-NH3_12" for ORFV Strain OV-HN3/12, "NP" for ORFV Strain NP, and "YX" for ORFV Strain YX.

Figure 9 shows a comparison of the gene ORFV111 of the present invention between the complete nucleotide sequences and that of the partial deletion.

Figure 10 shows the alignment of the complete sequences of the hypothetical protein 111 of the present invention with that of the hypothetical protein 111 of other parapox virus strains, where "3F8" stands for POV-601-3F8 Strain, "B029" for ORFV Strain B029, "GO" for ORFV Strain GO, "NA11" for ORFV Strain NA1/11, "NZ2" for ORFV Strain NZ2, "NA17" for ORFV Strain NA17, "OV-SA00" for ORFV Strain OV-SA00, "OV-IA82" for ORFV Strain OV-IA82, "SJ1" for ORFV Strain SJ1, "SY17" for ORFV Strain SY17, "OV-NH3_12" for ORFV Strain OV-HN3/12, "NP" for ORFV Strain NP, and "YX" for ORFV Strain YX.

Figure 11 shows the comparison of the complete sequence of the ORFV111 gene of the present invention with that of the ORFV111 gene of other parapox virus strains, where "3F8" stands for POV-601-3F8 Strain, "B029" for ORFV Strain B029, "GO" for ORFV Strain GO, "NA11" for ORFV Strain NA1/11, "NZ2" for ORFV Strain NZ2, "NA17" for ORFV Strain NA17, "OV-SA00" for ORFV Strain OV-SA00, "OV-IA82" for ORFV Strain OV-IA82, "SJ1" for ORFV Strain SJ1, "SY17" for ORFV Strain SY17, "OV-NH3_12" for ORFV Strain OV-HN3/12, "NP" for ORFV Strain NP, and "YX" for ORFV Strain YX.

Figure 12 shows a comparison of the gene ORFV111 of the present invention between the complete amino acid sequences and that of the partial deletion.

Figure 13 shows a comparison of the gene ORFV112 of the present invention between the complete amino acid sequences and that of the partial deletion.

Figure 14 compares the anti-tumor inhibitory effects between the viral strain POV-601-3F8 with a complete ORFV112 gene and the viral strain POV-601-1A1 with a deleted ORFV112 gene in a CT26 mouse colon cancer model.

Figure 15 compares the anti-tumor inhibitory effects between strains POV-601-1A1 and the v611a in the B16-F10 mouse melanoma tumor model.

Figure 16 compares the anti-tumor inhibitory effects among strains POV-601-1A1, v615a and v616a in the B16-F10 mouse melanoma tumor model.

Figure 17 compares the anti-tumor inhibitory effects among strains v601-1A1, v617a and v618a in the B16-F10 mouse melanoma tumor model.

DETAINED DESCRIPTION OF THE INVENTION

The present invention discloses the mutant Orf viruses and their medicinal compositions for use in cancer treatment.

According to the literature, the virulence genes of a wild-type Orf virus mainly include OVIFNR (orf virus interferon-resistance gene), CBP (chemokine-binding protein), GIF (GM-CSF/IL-2 inhibitory protein), vIL-10 (viral interleukin 10), and VEGF-like protein (vascular endothelial growth factor-like protein), etc [11,13]. In general, attenuated viruses can be obtained by removing a certain virulence gene using molecular and/or cellular biology methods. For example, patent CN 104878043 B disclosed a method by deleting the virulence gene OVIFNR of the ORFV SHZ1 strain to achieve a rapid reduction in virulence and obtaining an attenuated virus strain, used for preparation of attenuated vaccines.

Mutant Orf viruses disclosed in the present invention were obtained through modification and screening of the original POV-601 viral strain (Deposit No. V201713) deposited in the China Typical Culture Collection Center (CCTCC).

Based on the literature [17], the inventors' specific primers for the ORFV112 gene were designed. The forward primer (SEQ ID NO: 5) was designed on the coding region sequence of the ORFV111 gene, and the reverse primer (SEQ ID NO: 6) was designed on the coding region sequence of the ORFV112 gene, thereby establishing a PCR-based molecular biology identification technique to check the integrity of the ORFV112 gene of the attenuated POV-601 viruses. Afterwards, African green monkey kidney cells (CV-1) were infected with the parental virus strain POV-601; and the virus-infected cells were collected, sorted into single cell by a flow cytometer, and expanded in culture. Using the said ORFV112 gene-specific primer pairs, extracting viral genomic DNAs (used as the PCR templates) from the expanded and virus-infected cells, the said attenuated ORFV virus strain with a partially deleted ORFV112 gene was obtained by the established molecular biology identification technique, and named it POV-601-1A1. This strain has a 312 bp 5' deletion and remains 552 bp within the coding region of the ORFV112 gene, and keeps 72 bp in its noncoding region at the 3' end, a total of 624 bp remaining, see SEQ ID NO: 4). At the same time, POV-601-3F8, a virus having the intact ORFV112 gene with 1162 bp that include both the coding and non-coding regions, has also been obtained. The complete sequence of gene ORFV112 is shown in SEQ ID NO: 3.

When the attenuated POV-601-1A1 viruses were evaluated in an animal tumor efficacy model, it was unexpectedly found that the POV-601-1A1 virus had a better anti-tumor inhibitory effect than POV-601-3F8, the virus with a complete ORFV112 gene.

On the other hand, the inventors (of the present invention) further intentionally used a gene editing technique, in an attempt to knock out all the remaining coding regions of the gene ORFV112 of the POV-601-1A1 virus. In one embodiment, the remaining coding region of gene ORFV112 was completely knocked out (only 22 bp of the 3' noncoding region were retained, see sequence SEQ ID NO: 57), and (the knocking out result) was confirmed by DNA sequencing. The obtained virus was named POV-604-1D1. In one embodiment, it was unexpectedly found that this virus exhibited a better anti-tumor effect than POV-601-3F8 containing the complete ORFV112 gene, when evaluated in an animal tumor efficacy model. Furthermore, any methods, such as knocking out or changing certain sequences of the coding region and non-coding region of the gene ORFV112, that prevent the expression of the CBP protein, can also achieve the same effect.

The POV-601-1A1 virus was deposited in accordance with Budapest Treaty at China Center for Type Culture Collection (CCTCC) (Wuhan, China) on May 19, 2020, under the CCTCC Deposit No. V202029.

In an embodiment, the mutant Orf viruses of the present disclosure can selectively infect in melanoma cells (B16-F10), bladder cancer cells (MB49), liver cancer cells (Hepa1-6), colon cancer cells (CT26), human cervical cancer cells (C-33A), human ovarian cancer cells (SK-OV-3), etc, and replicate within them.

In an embodiment, the ORFV007 gene of the mutant Orf viruses of the present disclosure was unexpectedly found to be completely deleted, while all the published ORFV strains in NCBI, such as NZ -2, NZ-7, D1701, NA1/11 strains, etc., have the intact gene ORFV007. The ORFV007 gene encodes for the deoxyuridine pyrophosphatase (dUPTase). This protein is an important enzyme for dNTP synthesis. In general, the concentration of dNTP in normal cells is strictly regulated, which is not conducive to viral replication. In cancer cells, however, there are higher concentrations of dNTP, which favors viral replication [16]. The deletion of ORFV007 will restrict viral replication in normal cells, but not in cancer cells, therefore achieving the goal of selective replication of ORFV in cancer cells.

Wild-type ORFV virus can generally be cultured in primary cells from bovine and sheep animal tissues (CN 103952377 A). Patent WO2012122649 Alfirst disclosed that ORFV could be proliferated and expanded in the human cervical cancer cell line HeLa. Further, the present invention discloses a method to select out the said mutant Orf viruses using mammalian cells as the host, preferably using the African green monkey kidney cell (line) CV-1 for viral infection and proliferation.

Further, an application of the said viruses in treating individual cancer is provided. The said cancer is any type of a solid tumor. The types of the said solid tumor include: cervical cancer, bladder cancer, liver cancer, ovarian cancer, melanoma, colorectal cancer, lung cancer, breast cancer, gastric cancer, uterine cancer, head and neck cancer, thyroid cancer, esophageal cancer, prostate cancer, pancreatic cancer, sarcoma, brain tumor, etc. Furthermore, the subject is mammal, including rodent and human.

The mechanisms by which oncolytic viruses inhibit tumor growth can generally be classified into 1) replication and amplification in infected tumor cells, lysing cancer cells, and achieving the purpose of oncolysis; 2) Following oncolysis, tumor-specific molecules, such as tumor neoantigens, are released, activating the immune system to mount a systemic attack on remaining cancer cells.

In an embodiment, the mutant Orf viruses of the present disclosure can effectively induce the innate immune response , represented by NK cell activation. In the bilaterally inoculated human C-33A cervical cancer cell model, the proportion of CD45+ and NK cell activation in the tumor and/or blood can be increased through intratumoral and/or intravenous injection of these viruses.

**I. General Techniques**

Unless otherwise indicated, implementation of the present invention will the employ the conventional techniques of molecular biology (including recombinant techniques), virology, microbiology, cell biology, biochemistry, and immunology, which are within the scope of technology in the field.

II. **Definitions**

- The term "OV" stands for oncolytic virus, the abbreviation of oncolytic virus.
- The term "solid tumor" refers to a tumor entity composed of multiple cells, as distinguished from blood tumors; it refers to tumors for a variety of cancers including cervical cancer, bladder cancer, liver cancer, ovarian cancer, melanoma, colorectal cancer, lung cancer, breast cancer, gastric cancer, uterine cancer, head and neck cancer, thyroid cancer, esophageal cancer, prostate cancer, pancreatic cancer, sarcoma, brain tumor, etc. Cancers can be at an early or advanced stage.
- The term "continuous cell line" refers to a cell population obtained from the primary culture or cell line with special genetic characteristics and biochemical properties or specific markers are called cell lines. A cell line that can be continuously passaged is called a continuous cell line.
- The term "CPE" stands for a cytopathic effect, that is, the cellular degeneration following infection of cultured cells by a virus. In an in vitro experiment, cultured cells are inoculated with a cell-killing virus, and cell rounding, necrosis, and detachment can be observed under a microscope after a certain period of time, called cytopathic effects.
- The term "Orf', also known as "contagious pustular dermatitis virus of the sheep" "OVIS", "ORFV" and "orf virus," refers to a pox virus that can cause contagious and epitheliotropic diseases mainly in sheep and goats.
- The term "effective amount" refers to the quantity needed to achieve the desired therapeutic or preventive effects within the necessary dosage and time; it also refers to the quantity at which the therapeutic beneficial effect of a therapeutic agent outweighs any toxic or harmful consequences.
- The term "PBS" refers to phosphate buffer saline.
- The term "MOI" stands for multiplicity of infection and refers to the ratio between the number of the viral particles and the total number of target cells (pfu/cell) during a viral infection.
- "POV-601-1A1 viral strain", where "POV" represents "Prajna Oncolytic Virus", refers to the fact that this viral strain was originated from Suzhou Prajna Biotechnology Co., Ltd. In the internal experimental records of Suzhou Prajna Biotechnology Co., Ltd., POV-601-1A1, v601-1A1, v601-p0-1A1, and 1A1 all represent the same strain. The name of this viral strain is "ORFV mutational type POV-601-1A1" in the CCTCC registry form and belongs to a Orf virus of the the parapoxvirus genus of the poxvirus family.
- "POV-601-3F8 viral strain," where "POV" represents "Prajna Oncolytic Virus", refers to the fact that this viral strain was originated from Suzhou Prajna Biotechnology Co., Ltd. In the internal experimental records of Suzhou Prajna Biotechnology Co., Ltd., POV-601-1A1, v601-3F8, v601-p0-3F8, and 3F8 all represent the same strain.
- The term "POV-604-1D1 viral strain," where "POV" represents "Prajna Oncolytic Virus", refers to the fact that this viral strain was originated from Suzhou Prajna Biotechnology Co., Ltd. In the internal experimental records of Suzhou Prajna Biotechnology Co., Ltd., POV-604-1D1, v604-1D1, and 1D1 all represent the same strain.
- For "viral storage buffers": the preparation method is to aspirate 500 mL of PBS (i.e. phosphate buffer, CORNING, catalog number: 21-040-CVR), add 1.25mL of 1M MgC12.6H2O to make its final concentration 2.48 mM, and then add 2.5 mL of 1M Tris-HCl (pH 9.0) (Sangong Bioengineering (Shanghai) Co., Ltd., catalog number: B548128-0500) to make its final concentration 4.96mM, mix evenly to obtain the virus storage buffer( the theoretical final concentrations of MgCl2·6H2O is 2.5mM and Tris-HCl is 5 mM).

**III. Composition and methods**

1. ORFV112 gene expressed product, or CBP protein

The present invention relates to a (natural) CBP. In one embodiment, the (natural) CBP protein has or comprises the sequence shown in SEQ ID NO: 1 or from the same or similar biological source (e.g., strain, species, genus, family) and having at least 90%, 91%, 92%, 93%, 94%, 95%, 96%, 97%, 98%, 99% or 100% identity full-length sequence, or has or comprises a mature protein sequence shown in SEQ ID NO: 2 or from the same or similar biological source (e.g., strain, species, genus, family) having at least 90%, 91%, 92%, 93%, 94%, 95%, 96%, 97%, 98%, 99% or 100% identity of the mature sequence, or is (essentially) composed thereof. In another embodiment, the (natural) CBP proteins have or comprise the sequences shown in SEQ ID NO: 31-42 or are from the same or similar biological source (e.g., strain, species, genus, family) and having at least 90%, 91%, 92%, 93%, 94%, 95%, 96%, 97%, 98%, 99% or 100% full-length sequence identity and the corresponding mature sequence.

The present invention also relates to a mutant type CBP protein. In one embodiment, the mutations are the addition, deletion, or substitution of one or more amino acid residues or any combination thereof, relative to the natural protein sequences. In one embodiment, the function of the mutant type CBP proteins is reduced or lost.

The present invention also relates to a decrease or loss of the expression of the CBP proteins (e.g., natural CBP proteins or mutant type CBP proteins).

For example, the function and/or expression of the CBP proteins are reduced at least 50%, 60%, 70%, 75%, 80%, 90%, 91%, 92%, 93%, 94%, 95%, 96%, 97%, 98%, 99%, to 100%, relative to the natural protein.

2. The ORFV112 gene

The present invention relates to a kind of the (natural) ORFV112 genes. In one embodiment, the (natural) ORFV112 genes encode proteins having or comprising the sequence shown in SEQ ID NO: 1 or from the same or similar biological source (e.g., strain, species, genus, family) and having at least 90%, 91%, 92%, 93%, 94%, 95%, 96%, 97%, 98%, 99% or 100% identity full-length sequence, or having or comprising the sequence shown in SEQ ID NO: 2 or from the same or similar biological source (e.g., strain, species, genus, family) having at least 90%, 91%, 92%, 93%, 94%, 95%, 96%, 97%, 98%, 99% or 100% identity mature protein sequence, or is (essentially) composed thereof. In another embodiment, the (natural) CBP proteins have or comprise the sequences shown in SEQ ID NO: 31-42 or from the same or similar biological source (e.g., strain, species, genus, family) and having at least 90%, 91%, 92%, 93%, 94%, 95%, 96%, 97%, 98%, 99% or 100% identity full-length sequence and the corresponding mature sequence.

The present invention relates to a (natural) ORFV112 gene. In one embodiment, the (natural) ORFV112 gene has or comprises the sequence shown in SEQ ID NO: 3 (or its coding region), encodes the same amino acid sequence, or come from the same or similar biological source (e.g., strain, species, genus, family) and has at least 75%, 80%, 85%, 90%, 91%, 92%, 93%, 94%, 95%, 96%, 97%, 98%, 99% or 100% identity nucleotide sequence, or is (essentially) composed thereof. The sequence shown in SEQ ID NO: 3 contains 1162 nucleotides, of which nucleotides 1-226 belong to the 5' non-coding region, nucleotides 227-1090 cover the protein-coding region, and nucleotides 1091-1162 belong to the 3' non-coding region. In another embodiment, the (natural) ORFV112 genes have or comprise the nucleotide sequences shown in SEQ ID NO: 43-54 (or its coding region), encode the same amino acid sequence, or come from the same or similar biological source (e.g., strain, species, genus, family) and have at least 75%, 80%, 85%, 90%, 91%, 92%, 93%, 94%, 95%, 96%, 97%, 98%, 99% or 100% identity nucleotide sequences.

The present invention also relates to a type of the mutant ORFV112 genes. In one embodiment, the mutations are the addition, deletion, or substitution of one or more nucleotide or any combination thereof, relative to the natural nucleotide sequence. In one embodiment, the mutant type ORFV112 gene is the ORFV112 gene with a complete deletion. The full-deletion type ORFV112 gene refers to the absence of the entire ORFV112 gene. In one embodiment, the mutant type ORFV112 gene is a partially deleted ORFV112 gene. Partial deletion type ORFV112 gene lacks one or more but not all nucleotides of the ORFV112 gene. In one embodiment, the partial deletion type ORFV112 gene lacks one or more nucleotides in the 5' non-coding region, the coding region and/or the 3' non-coding region, or any combination thereof. In one embodiment, the partial deletion type ORFV112 gene is a 5' terminal deletion type ORFV112 gene, that is, one or more nucleotides at the 5' end are missing, such as missing the entire or part of the 5' non-translated region, missing the entire 5' non-translated region and all or part of the coding region, or missing the entire 5' non-translated region, the entire coding region, and all or part of the 3' non-translated region. In one embodiment, the ORFV112 gene deficiency extends to the upstream region (the ORFV111/112 intergenic region, if present, and/or the ORFV111 gene, specifically its 3' end) and/or the downstream region (the ORFV112/113 intergenic region, if present, and/or the ORFV113 gene, especially 5' end).

In the case where the natural ORFV112 gene has or comprises a nucleotide sequence (or its coding region) shown in SEQ ID NO: 3 or is (essentially) composed thereof, in one embodiment, the partial deletion type ORFV112 gene lacks one or more nucleotides of the sequence shown in SEQ ID NO: 3, such as the 1-1161 nucleotides. In the case where the natural ORFV112 gene has or comprises a nucleotide sequence (or its coding region) shown in SEQ ID NO: 3 or is (essentially) composed thereof, in one embodiment, the 5' end partial deletion type ORFV112 gene lacks one or more nucleotides of the sequence shown in SEQ ID NO: 3, in particular the 5' end 1-1161, 1-1140, 1-538, 539-1139, or 1141-1160 nucleotides. In one embodiment, the 5' end partial deletion type ORFV112 gene has or comprises the nucleotide sequence (or its coding region) shown in SEQ ID NO: 4 or SEQ ID NO: 57, or is (essentially) composed thereof. In the case where the natural ORFV112 gene has or comprises the nucleotide sequences (or its coding region) shown in SEQ ID NO: 43-54, or is (essentially) composed thereof, in one embodiment, the 5' end deletion type ORFV112 gene lacks one or more nucleotides at the 5' end of the sequences shown in SEQ ID NO: 43-54, especially one or more nucleotides of a segment corresponding to 1140 or 538 nucleotides at the 5' end of SEQ ID NO: 3. Due to the deletion of the 5' non-translated region and/or the initiation codon, these deletion type genes are unable to express proteins.

In one embodiment, the mutation type ORFV112 gene of the present invention results in a decrease or loss of the functional CBP protein, including a decrease and loss in expression and/or activity of CBP. For example, the decrease is of at least 50%, 60%, 70%, 75%, 80%, 90%, 91%, 92%, 93%, 94%, 95%, 96%, 97%, 98%, 99%, to 100%, while compared to the natural function and/or expression.

3. The expressed product of the ORFV111 gene, the hypothetical protein 111

The present invention relates to a (natural) hypothetical protein 111. In one embodiment, the (natural) hypothetical protein 111, having or comprising the sequence shown in SEQ ID NO: 58 or from the same or a similar biological source (e.g. strain, species, genus, family) and having at least 90%, 91%, 92%, 93%, 94%, 95%, 96%, 97%, 98%, 99% or 100% identity sequence, or is (essentially) composed thereof. In another embodiment, the (natural) hypothetical proteins 111 have or comprise the sequences shown in SEQ ID NO: 61-72 or are from the same or a similar biological source (e.g. strain, species, genus, family) and have at least 90%, 91%, 92%, 93%, 94%, 95%, 96%, 97%, 98%, 99% or 100% identity sequence.

The present invention also relates to a mutant type hypothetical protein 111. In one embodiment, the mutations are the addition, deletion, or substitution of one or more amino acid or any combination thereof relative to the natural sequences. In one embodiment, the function of the mutant type hypothetical protein 111 is reduced or lost.

The present invention also relates to a decrease or loss of the expression of the hypothetical protein 111 (e.g., the natural hypothetical protein 111 or the mutant type hypothetical protein 111).

For example, the function and/or expression of the hypothetical protein 111 are reduced at least 50%, 60%, 70%, 75%, 80%, 90%, 91%, 92%, 93%, 94%, 95%, 96%, 97%, 98%, 99%, to 100%, relative to the natural protein.

4. TheORFV111 gene

The present invention relates to a (natural) ORFV111 gene. In one embodiment, the (natural) ORFV111 genes encode proteins having or comprising the sequence shown in SEQ ID NO: 58 or from the same or similar biological source (e.g., strain, species, genus, family) and having at least 90%, 91%, 92%, 93%, 94%, 95%, 96%, 97%, 98%, 99% or 100% identity sequence, or the hypothetical protein 111 is (essentially) composed thereof. In another embodiment, the (natural) hypothetical protein 111 has or comprise the sequences shown in SEQ ID NO: 61-72 or from the same or similar biological sources (e.g., strain, species, genus, family) and has at least 90%, 91%, 92%, 93%, 94%, 95%, 96%, 97%, 98%, 99% or 100% identity sequence.

The present invention relates to a (natural) ORFV111 gene. In one embodiment, the (natural) ORFV111 gene has or comprises the sequence shown in SEQ ID NO: 59 and encodes the same amino acid sequence or come from the same or similar biological sources (e.g. strain, species, genus, family) and has at least 75%, 80%, 85%, 90%, 91%, 92%, 93%, 94%, 95%, 96%, 97%, 98%, 99% or 100% identity nucleotide sequences, or is (essentially) composed thereof. The sequence shown in SEQ ID NO: 59 contains 794 nucleotides, of which nucleotides 1-28 belong to the 5' non-coding region, nucleotides 29-568 cover the protein-coding region, and nucleotides 569-794 belong to the 3' non-coding region. In another embodiment, the (natural) ORFV111 genes have or comprise the nucleotide sequences shown in SEQ ID NO: 73-84 (or the coding region), encode the same amino acid sequence, or come from the same or similar biological sources (e.g., strain, species, genus, family) and have at least 75%, 80%, 85%, 90%, 91%, 92%, 93%, 94% %, 95%, 96%, 97%, 98%, 99% or 100% identity nucleotide sequences.

The present invention relates to a type of mutant ORFV111 genes. In one embodiment, the mutations are the addition, deletion, or substitution of one or more nucleotides or any combination thereof, relative to the natural nucleotide sequence. In one embodiment, the mutant type ORFV111 gene is the ORFV111 gene with a complete deletion. The full-deletion type ORFV111 gene refers to the absence of the entire ORFV111 gene. In one embodiment, the mutant type ORFV111 gene is a partially deleted ORFV111 gene. Partial deletion type ORFV111 gene lacks one or more but not all nucleotides of the ORFV111 gene. In one embodiment, the partial deletion type ORFV111 gene lacks one or more nucleotides in the 3' non-coding region, the coding region and/or the 5' non-coding region, or any combination thereof. In one embodiment, the partial deletion type ORFV 111 gene is a 3' terminal deletion type ORFV111 gene, that is, one or more nucleotides at the 3' end are missing, such as missing the entire or part of the 3' non-translated region, missing the entire 3' non-translated region and all or part of the coding region, or missing the entire 3' non-translated region, the entire coding region and all or part of the 5' non-translated region. In one embodiment, the ORFV111 gene deficiency extends to the downstream region (the ORFV111/112 intergenic region, if present, and/or the ORFV 112 gene, specifically its 5' end) and/or the upstream region (the ORFV 110/111 intergenic region, if present, and/or the ORFV110 gene, especially its 3' end).

In the case where the natural ORFV111 gene has or comprises a nucleotide sequence (or its coding region) shown in SEQ ID NO: 59 or is (essentially) composed thereof, in one embodiment, the partial deletion type ORFV111 gene lacks one or more nucleotides of the sequence shown in SEQ ID NO: 59, such as the 1-793 nucleotides. In the case where the natural ORFV111 gene has or comprises a nucleotide sequence (or its coding region) shown in SEQ ID NO: 59 or is (essentially) composed thereof, in one embodiment, the 3' end partial deletion type ORFV111 gene lacks one or more nucleotides at the 3' end of the sequence shown in SEQ ID NO: 59, especially the 3' end 1-793; 1-309 or 310-792 nucleotides. In one embodiment, the 3' end deletion type ORFV111 gene has or comprises the nucleotide sequence (or its coding region) shown in SEQ ID NO: 60 or is (essentially) composed thereof. In the case where the natural ORFV111 gene has or comprises the nucleotide sequences (or its coding region) shown in SEQ ID NO: 73-84 or is (essentially) composed thereof, in one embodiment, the 3' terminal deletion type ORFV111 gene lacks one or more nucleotides at the 3' end of the sequence shown in SEQ ID NO: 73-84, especially one or more nucleotides of a segment corresponding to 309 nucleotides at the 3' end of SEQ ID NO: 59. Due to the deletion of the 3' non-translated region, these deletion type genes are unable to express proteins.

In one embodiment, mutations of the ORFV111 gene of the present invention results in a decrease or loss of functional hypothetical protein 111, including a decrease or loss of the expression and/or activity of hypothetical protein 111. For example, the decrease is of at least 50%, 60%, 70%, 75%, 80%, 90%, 91%, 92%, 93%, 94%, 95%, 96%, 97%, 98%, 99%, to 100%, while compared to the natural function and/or expression.

5. The ORFV viral genome

The present invention relates to an ORFV virus genome. In one embodiment, the ORFV viral genome has the said above ORFV112 gene and/or ORFV111 gene, including the natural ORFV112 gene and/or ORFV111 gene and mutated types of the ORFV112 gene and/or ORFV111 gene. The present invention particularly relates to an ORFV virus genome with ORFV112 gene and/or ORFV111 gene deficiency. In one embodiment, the ORFV viral genome with ORFV112 gene and/or ORFV111 gene-deficient comprises the said mutant type ORFV112 gene and/or ORFV111 gene. In one embodiment, the ORFV viral genome (e.g., ORFV virus genome with ORFV112 gene and / or ORFV111 gene-deficient) completely loses the ORFV007 gene. In one embodiment, the 3' non-coding region of the ORFV111 gene overlaps with the 5' non-coding region of the ORFV112 gene.

6. The Orf virus

The present invention relates to a type of ORFV viruses. In one embodiment, the ORFV virus has the CBP protein and/or hypothetical protein 111 mentioned above, including the natural CBP protein and/or hypothetical protein 111 and the mutant type of the CBP protein and/or hypothetical protein 111. The present invention in particular relates to the ORFV virus with deficient CBP protein and/or hypothetical protein 111. In one embodiment, the ORFV virus with a deficient CBP protein and/or hypothetical protein 111 lowers or loses the functional CBP protein and/or hypothetical protein 111. Reduction or loss of the functional CBP protein and/or hypothetical protein 111 may result from reduction or loss of the expression and/or activity of the CBP protein and/or hypothetical protein 111.

The present invention relates to a type of ORFV viruses. In one embodiment, the ORFV virus contains the said ORFV112 gene and/or ORFV111 gene, including the natural ORFV112 gene and/or ORFV111 gene and the mutant type ORFV112 gene and/or ORFV111 gene. In one embodiment, the ORFV virus contains the ORFV viral genome said above, including the ORFV viral genome with deficient ORFV112 gene and/or the ORFV111 gene. In particular, the present invention relates to a type of ORFV virus with deficient CBP protein and/or hypothetical protein 111 , comprising the previously said mutated type ORFV112 gene and/or ORFV111 gene or the previously said ORFV112 gene and/or ORFV111 gene-defective ORFV viral genome.

In one embodiment, the ORFV virus (e.g. the ORFV viruses with CBP protein and/or hypothetical protein 111 deficiency) lacks the functional transcription and/or translational products of the gene ORFV007, including the loss of expression and/or activity.

7. Methods for modifying ORFV virus

The present invention relates to a type of methods for modifying the ORFV virus genome or the ORFV virus. In one embodiment, the methods comprise mutating the ORFV112 gene and/or the ORFV111 gene within the ORFV viral genome. In one embodiment, the methods comprise adding, deleting, or replacing, particularly deleting one or more nucleotides of the ORFV112 gene and/or ORFV111 gene, particularly (deleting) one or more nucleotides at the 5' end of the ORFV112 gene and/or one or more nucleotides at the 3' end of the ORFV111 gene. In the case where the natural ORFV112 gene has or comprises a nucleotide sequence (or its coding region) shown in SEQ ID NO: 3 or is (essentially) composed thereof, in one embodiment, the methods comprise deleting one or more nucleotides of the sequence shown in SEQ ID NO : 3, such as 1-1162 nucleotides. In the case where the natural ORFV112 gene has or comprises a nucleotide sequence (or its coding region) shown in SEQ ID NO: 3 or is (essentially) composed thereof , in one embodiment, the methods comprise deleting one or more nucleotides at the 5' end of the sequence shown in SEQ ID NO:3, especially 1-1161, 1-1140, 1-538, 539-1139, or 1141-1160 nucleotides at the 5' end, such as 538 or 1140 nucleotides. In the case where the natural ORFV112 gene has or comprises a nucleotide sequence (or its coding region) shown in SEQ ID NO: 3 or is (essentially) composed thereof , in one embodiment, the methods comprise the complete deletion of the entire sequence shown in SEQ ID NO: 3 or the deletion of 5' terminal 1140 or 538 nucleotides of SEQ ID NO: 3. In the case where the natural ORFV112 gene has or comprises a nucleotide sequence (or its coding region) shown in SEQ ID NO: 3 or is (essentially) composed thereof, in one embodiment, the methods comprise completely deleting the said nucleotide sequences or deleting a region of the said nucleotide sequence corresponding to 1140 or 538 nucleotides at the 5' end of SEQ ID NO: 3. In the case where the natural ORFV111 gene has or comprises a nucleotide sequence (or its coding region) shown in SEQ ID NO: 59 or is (essentially) composed thereof, in one embodiment, the methods comprise deleting one or more nucleotides of the sequence shown in SEQ ID NO: 59, such as 1-794 nucleotides. In the case where the natural ORFV111 gene has or comprises a nucleotide sequence (or its coding region) shown in SEQ ID NO: 59 or is (essentially) composed thereof, in one embodiment, the methods comprise deleting one or more nucleotides at the 3' end of the sequence shown in SEQ ID NO: 59, especially 1-793, 1-309 or 310-792 nucleotides at the 3' end, such as 309 nucleotides. In the case where the natural ORFV111 gene has or comprises a nucleotide sequence (or its coding region) shown in SEQ ID NO: 59 or is (essentially) composed thereof , in one embodiment, the methods comprise completely deleting SEQ ID NO: 59 or deleting the 309 nucleotides at the 3' end of the sequence shown in SEQ ID NO: 59. In the case where the natural ORFV111 gene has or comprises a nucleotide sequence (or its coding region) shown in SEQ ID NO: 59 or is (essentially) composed thereof , in one embodiment, the methods comprise completely deleting the said nucleotide sequences or deleting a region of the said nucleotide sequence corresponding to 309 nucleotides at the 3' end of SEQ ID NO: 59. In the case where the natural ORFV111 gene and the natural ORFV112 gene have or comprise other nucleotide sequences (or its coding regions) or are (essentially) composed thereof, in one embodiment, the methods comprise completely deleting the sequences shown in SEQ ID NO: 3 and SEQ ID NO: 59. In one embodiment, the methods also comprise mutating (e.g. deletion, including complete deletion or partial deletion) the ORFV007 gene in the ORFV viral genome (especially ORFV112 gene and/or ORFV111 gene-deficient ORFV viral genome). In one embodiment, the 3' non-coding region of the ORFV111 gene overlaps with the 5' non-coding region of the ORFV112 gene. The methods of the present invention can be carried out by conventional molecular biology techniques (such as molecular cloning, DNA recombination, homologous recombination, PCR, restriction nuclease (digestion), gene knockout, silencing, etc.) or emerging molecular biology techniques (such as gene editing, etc.). The present invention relates to obtaining the ORFV viral genome (such as ORFV112 gene and/or ORFV111 gene-deficient ORFV viral genome) and ORFV virus (such as CBP protein and/or hypothetical protein 111-deficient ORFV virus) through the utilization of the said methods.

8. Methods for identifying ORFV viral genomes and ORFV viruses

The present invention relates to a method for identifying the ORFV viral genome or the ORFV virus, in particular the method for detecting the (presence or activity) of the CBP protein and/or hypothetical protein 111 (especially mutant type CBP protein and/or hypothetical protein 111) and/or the (presence or sequence) of the ORFV112 and/or ORFV111 genes (especially mutant type ORFV112 and/or ORFV111 genes). The methods of the present invention can be carried out by the techniques for detecting the presence or activity of a protein or the presence or sequence of a nucleic acid, and these conventional molecular biology techniques include activity assay, hybridization (such as Southern hybridization, Northern hybridization, or Western hybridization), restriction nucleases, PCR, electrophoresis (such as gel electrophoresis, including protein electrophoresis and nucleic acid electrophoresis (agarose electrophoresis and PAGE electrophoresis, reducing and non-reducing electrophoresis), sequencing (including protein sequencing and nucleic acid sequencing), etc.) or emerging molecular biology techniques (such as next-generation sequencing, etc.). The present invention especially provides methods for detecting the integrity (or length) of gene ORFV112 and/or ORFV111. In one embodiment, the method is performed by PCR and gel electrophoresis. In one embodiment, the method can be performed by nucleic acid hybridization or sequencing.

The design of primers and probes is within the competence of those skilled in the art. Those skilled in the art know how to select primers and probes on the target area of the target sequence. For example, to detect the integrity of the gene ORFV112, on the one hand, the target region of the upstream primer can be set, but not limited to, at the 5' end of the gene ORFV112 or can extend upstream, for example, into the ORFV111/112 intergenic region, if any, or into gene ORFV111 (especially the 3' end of gene ORFV111, e.g., 3' non-translated region); on the other hand, the target region of the downstream primer can also be set at the 3' end of the gene ORFV 112 or can extend downstream, for example, into the ORFV 112/113 intergenic region, if any, or into gene ORFV113 (specifically the 5' end of the gene ORFV113, e.g., the 5' non-translated region). Similarly, primer/probes can be designed based on similar principles.

9. Uses of the ORFV viral genome and ORFV virus

The present invention relates to a pharmaceutical composition, which comprises a certain amount, especially an effective amount, such as a therapeutically effective amount or a preventive effective amount, of the ORFV viral genomes (such as the ORFV112 gene and/or ORFV111 gene-deficient ORFV viral genome) and/or the ORFV viruses (e.g., CBP protein and/or hypothetical protein 111-deficient ORFV viruses) of the present invention. In one embodiment, the pharmaceutical composition comprises a pharmaceutically acceptable carrier.

In one embodiment, the said composition is in a form of powder, solution, transdermal patch, ointment, or suppository. In one embodiment, the composition is administered through intravenous, intratumoral, intramuscular, subcutaneous, rectal, vaginal, or intraperitoneal routes.

The present invention relates to a method for treating a disease or delaying disease progression in subjects, which comprises administering a certain amount, particularly an effective amount, such as a therapeutically effective amount or a preventively effective amount of the ORFV viral genome (such as the ORFV112 gene and /or ORFV111 gene-deficient ORFV virus genome) and/or the ORFV virus (such as the CBP protein and/or hypothetical protein 111-deficient ORFV virus) of the present invention.

The present invention relates to a use of a certain amount, especially an effective amount, such as a therapeutically effective amount or a preventive effective amount of the ORFV viral genome (such as ORFV112 gene and/or ORFV111 gene-deficient ORFV viral genome) and/or ORFV virus (such as CBP protein and/or hypothetical protein 111-deficient ORFV virus) of the present invention in the preparation of drugs. In one embodiment, the said drug is used to treat the disease or delay disease progression in the subject.

The present invention relates to a certain amount, especially an effective amount, such as a therapeutically effective amount or a preventive effective amount of the ORFV viral genome (such as ORFV112 gene and/or ORFV111 gene-deficient ORFV viral genome) and/or ORFV virus (such as CBP protein and/or hypothetical protein 111-deficient ORFV virus) of the present invention, which are used to treat the disease or delay disease progression in the subject.

In one embodiment, the said disease is cancer. In one embodiment, the said cancer is a solid tumor. In one embodiment, the said solid tumor is cervical cancer, bladder cancer, liver cancer, ovarian cancer, melanoma, colorectal cancer, lung cancer, breast cancer, gastric cancer, uterine cancer, head and neck cancer, thyroid cancer, esophageal cancer, prostate cancer, pancreatic cancer, sarcoma, brain tumor, etc.

In one embodiment, the said subject is a mammal, including rodent (e.g., mouse and rat), non-human primate (e.g., cynomolgus monkey), and human.

## EXAMPLES

### Example 1: Screening and Purification Method of the ORFV Mutants (Strains)

- Virus source: POV-601 Viral Strain (China Center for Type Culture Collection (CCTCC), Deposit No. V201713)
- Host cell source : African green monkey kidney cell CV-1 (National Collection of Authenticated Cell Cultures/ Shanghai Institutes for Biological Sciences, CAS)
- Flow cytometry cell sorting and single clone picking methods:

  1) Infected the African green monkey kidney CV-1 cells with the POV-601 virus strain, followed by sorting of the viral infected cells with a flow cytometer (BD). Then, seeded the cells into 96-well plates, one cell each well;

2) Transferred the sorted 96-well plates into a 37°C, 5% CO2 incubator (Thermo, 160i) for culturing, and observed the infection daily;
3) When virus-infected cells displayed enough CPE, collected and cryopreserved the virus;
4) Extracted the viral genomic DNA from each collected monoclonal virus respectively using the QuickExtract™ DNA Extraction Solution (Lucigen, Cat#: QE09050);
5) Using the designed ORFV112 gene-specific forward and reverse primers (SEQ ID NO: 5 and 6) and the genomic DNA extracted from a single viral clone as the template, target fragment amplification and agarose gel electrophoresis were performed respectively. Figure 1 shows the electrophoresis results of the monoclonal viral strains(mutants) POV-601-1A1 and POV-601-3F8, and their band sizes are about 500bp and 1000bp respectively. Meanwhile, perform PCR amplification, using the ORFV112 gene-specific forward and reverse primers (SEQ ID NO: 5 and 6) and the POV-601-3F8 forward and reverse primers (SEQ ID NO: 55 and 56) respectively , and Sanger sequencing with the PCR products, confirming that the ORFV112 gene of POV-601-1A1 missed 538 bases at the 5' end, which contain 226 bases of the 5' end non-coding and 312 bases of the 5' end of protein-coding region (see SEQ ID NO: 4). POV-601-3F8 has a complete ORFV112 gene sequence (see SEQ ID NO: 3).

- The parameters of the PCR program: 94°C Pre-denaturation 5min; 94°C Denaturation 30s, 62°C Primer Annealing 30s, 68°C Extension lmin, 30 Cycles; 68°C Final Extension 7min.

**Example 2: Confirmation of the Species of the ORFV Mutant (strain)**

The viral genome (DNA) extracted from the POV-601-1A1 viral strain (using the kit (Mouse Tail Genomic DNA Kit, Cat#: CW2094S) ) was sent to a third-party sequencing company for next-generation sequencing (Genewiz Inc., Suzhou) . Sequencing results were assembled and analyzed. The complete B2L gene [21] (ORFV011) was compared to other published sequences using BLAST, the one showing higher similarity is the OV/HLJ/04 (strain), with a 99.91% similarity. Therefore, it was confirmed that the isolated virus was a ORFV (virus).

| No. | Description | Query Cover | Per.Ident | Accession |
|---|---|---|---|---|
| 1 | Orf virus strain OV/HLJ/04 | 99% | 99.91% | KU523790.1 |
| 2 | Orf virus isolate YL-3 | 100% | 99.82% | KF772211.1 |
| 3 | Orf virus strain China Vaccine | 100% | 99.82% | JQ904789.1 |
| 4 | Orf virus strain ORFV/Shaanxi/2015/China | 100% | 99.74% | KU194469.1 |
| 5 | Orf virus strain XD | 100% | 98.94% | KM675392.1 |

**Example 3: Construction of the ORF Mutants**

- Source of the viral strain: the POV-601-1A1 viral strain (China Center for Type Culture Collectio (CCTCC) , Deposit No: V202029)
  Source of the host cell: African Green Monkey Kidney Cell CV-1 (ATCC, No. CCL-70™)
- The method for constructing recombinant ORFV, mainly consisting of the following steps:

1) Using the specifically designed primers with the HindIII and EcoRI restriction endonuclease sites at the ends (SEQ ID NO: 7-12) , the flanking sequence of the ORFV112 gene (the left arm and right arm) and the EGFP report gene were cloned (amplified) by PCR;
2) Using HindIII and EcoRI to digest the pUC19 plasmid;
3) The PCR products in step 1) were ligated with the enzyme-cut pUC19 plasmid to obtain the CBP shuttle plasmid;
4) Using the ligated CBP shuttle plasmid obtained from 3) as a template, and the specific primers (SEQ ID NO: 13-14) with the EcoRI and AgeI restriction endonuclease sites at the ends, a linearized CBP shuttle vector was obtained by PCR amplification;
5) A new Cas9 plasmid vector (fragment) without a nuclear localization signal (NLS) and a new px330 plasmid vector backbone (fragment) were amplified by PCR amplification using the px330 plasmid vector as a template and the specific primers (SEQ ID NO: 15-18) with the EcoRI and AgeI restriction endonuclease sites at their ends respectively;

6) Digested the new Cas9 fragment and px330 backbone from step 5 with the EcoRI and AgeI enzymes and ligated them by the T4 DNA ligase. The ligated products were transformed into a host bacteria strain DH5α to obtain positive (desired) clones. Individual clones were expanded in culture and their plasmid (DNA) was extracted. After Sanger sequencing verification, the recombinant plasmid without a nuclear localization signal, px330-ΔNLS, was obtained;

7) Synthesized the CBP gRNAs, digested the px330-ΔNLS recombinant plasmid with the BbSI enzyme, and then ligated the CBP gRNAs and the digested px330-ΔNLS. The ligated products were transformed into a host bacteria strain DH5α to obtain positive (desired) clones. Individual (positive) clones were expanded in culture, its plasmid (DNA) was extracted, and the px330-ΔNLS-CBP gRNA expression plasmid was obtained;

8) Transfected the selected parapoxvirus host cell CV-1 with the obtained px330-ΔNLS-CBP gRNA plasmid, followed by infecting the transfected CV-1 cells with the POV-601-1A1 viral strain. Transfected the linear CBP shuttle vector obtained in step (4) into CV-1 cells that have been transfected with px330-ΔNLS-CBP gRNA plasmid and infected with the POV-601-1A1 virus;

9) Enriched and cultured the target cells having the EGFP fluorescent virus, and then sorted the cells with a flow cytometer into 96-well plates (1 cell per well), followed by culture expansion. Harvested the target cells from the well containing rich EGFP fluoresce virus, extracted the viral genomic DNA and used the PCR method for identification (the primers, SEQ ID NO: 19-30). Infected CV-1 cells with the virus showing the target bands again, and culturally expanded (the infected cells);

10) Through multiple rounds of virus inoculation, sorting, virus collection, and PCR identification, a pure monoclonal virus with a complete deletion of the ORFV112 gene was obtained, named as POV-604-1D1.

**Example 4: Culture of the ORFV mutants**

Source of the host cell: African Green Monkey Kidney Cell CV-1 (ATCC No.CCL-70™) Source of the viral strains (mutants): POV-601, POV-601-1A1, POV-604-1D1 and POV-601-3F8 viral strains (mutants)

- Viral infection of the cell:

1) Subcultured CV-1 cells according to the required amount. Cells in flasks could be infected when about 90% of cell confluence was reached;

2) The viral solution was appropriately diluted with a 2% FBS/DMEM complete medium

3) Discarded the used media from flasks and replenished with appropriate amount of the fresh 2% FBS/DMEM complete medium.

4) Added an already diluted viral solution at a MOI of 0.5. Gently shook the flasks to let the virus distribute evenly, labelled the flasks and transferred them into a 37oC, 5% CO2 incubator;

- Cryopreservation of the virus-infected cells

1) When more than 90% of the virus-infected cells displayed CPE, the virus could be harvested;

2) Took out the virus-infected cells from the cell culture incubator, collected the supernatant; trypsinized the adherent cells and combined them with the collected supernatant;

3) Centrifuged (the cell/supernatant mix from 2)) at 3000rpm, at 4°C for 10min;

4) Rinsed (the pellet from 3)) with PBS once;

5) Resuspended the pellet (cell) with an appropriate amount of PBS;

6) Transferred (the suspension) into a -80°C freezer for storage;

- Cell lysis to release the virus

1) Transferred the virus-infected cells from -80°C to a 37°C water bath for thawing ;

2) Sonicated the cells three times (Thermo, Model # FB120) : Set "Amplitude" to 100%, "Time" to 1 min;

3) Added Benzonase (25U/ml), gently mixed, and incubated at 37°C for 30min;

4) Centrifuged at 1300rpm for 10min;

5) Collected the viral supernatant and transferred it to -80°C for storage.

**Example 5: Evaluation of Antitumor Activity of the ORFV Mutants in A Mouse Bladder Cancer Model**

A. Experimental design

Table 1

| Group | Animal Number | Virus Titer | Route of Administration | Dosage and Frequency |
|---|---|---|---|---|
| Control (PBS) | 6 | / | Intra-tumoral | First injection upon |
| POV-601-1A1 | 6 | 2.07*$10^8$pfu/ml | injection | the day of grouping, followed by one injection every 72h, 40 μL/mouse |
| POV-604-1D1 | 6 | 1.9*$10^8$pfu/ml | | |

B. Experimental animals: Male C57BL/6 mice, 6W, (Zhejiang Vital River Laboratory Animal Technology Co., Ltd.)
C. Procedures:

Implanted a male C57BL/6 mouse subcutaneously on the right back with MB49 cells (GuangZhou Jennio Biotech Co.,Ltd) resuspended in PBS; the cell density was 2×$10^6$ /ml and the inoculation dose was 0.1ml/mice. The date of cell implantation was defined as Day 0. When the average volume of the tumors in the control group reached about 100mm$^3$, mice were randomly assigned to different groups based on the tumor size. Injection was performed according to Table 1, and all the mice were euthanized on day 30;
Weighed mice and measured tumor volumes twice a week throughout the entire study. The tumor volume was measured using a vernier caliper (Mahr GmbH, Model 16ER), and the results are shown in Figure 2.

Calculated the tumor volume by the following formula: tumor volume (mm$^3$) =a*b$^2$/2, wherein a is the tumor length (mm) , b is the tumor width (mm) . Length and width were measured vertically.
Calculated the relative mean of tumor volumes by the following formula: RTV (Relative Tumor Volume) =$V_t/V_0$, wherein, $V_0$ is the average tumor volume measured during group assignment, and $V_t$ is the average tumor volume at each measurement. Relative tumor proliferation rate T/C (%) =$T_{RTV}$ / $C_{RTV}$*100%。($T_{RTV}$: treatment group RTV; Curv: control group RTV) .

$$\text{Tumor growth inhibition rate TGI\%=（1-T/C）}\times 100\%$$

ANOVA for statistical analysis: ANOVA analysis was used to compare whether there was a significant difference in tumor volumes between the experimental and control groups. All the data were analyzed with SPSS 17 , and the statistical significance was defined as P<0.05.

D. The result

Table 2

| Day 30 | Average Tumor Size | TGI (%) | TGI Analysis (TGI>60%) | P-value (ANOVA) |
|---|---|---|---|---|
| Control (PBS) | 3066.11 | - | - | - |
| POV-601-1A1 | 558.53 | 82 | 5/6 | 0.008** |
| POV-604-1D1 | 102.85 | 100 | 6/6 | 0.002** |

**Example 6: Evaluation of Antitumor Activity of the ORFV Mutants in a Mouse Melanoma Model**

A. Experimental design

| Table 3 | | | | |
|---|---|---|---|---|
| Group | Number of Mice | Virus Titer | Route of Administration | Dosage and Frequency |
| Control (PBS) | 5 | / | Intratumoral injection | First injection upon the day of grouping, followed by one injection every 72h, 40 μL/ mouse |
| POV-604-1D1 | 5 | $1.90*10^8$pfu/ml | | |
| POV-601-3F8 | 5 | $4.31*10^8$pfu/ml | | |
| POV-601-1A1 | 5 | $2.98*10^8$pfu/ml | | |

B. Experimental animals: Female C57BL/6 mice, 6-8W, Zhejiang Vital River Laboratory Animal Technology Co., Ltd).

C. Procedures

Implanted female C57BL/6 mice subcutaneously on their right back with B16-F10 cells (Cell Bank of Type Culture Collection Committee of Chinese Academy of Sciences ) resuspended in PBS; the cell density was $5\times10^6$ /ml and the inoculation dose was 0.1ml/mice. The date of cell inoculation was defined as Day 0. When the average volume of the tumors in the control group reached about $100mm^3$, mice were randomly assigned to different groups based on the tumor size. Injection was performed according to Table 3, and all the mice were euthanized on day 15;

Weighed mice and measured tumor volumes twice a week throughout the entire study. The tumor volume was measured using a vernier caliper (Mahr GmbH, Model 16ER), and the results are shown in Figure 3.

Calculated the tumor volume by the following formula: tumor volume ($mm^3$) =$a*b^2/2$, wherein a is the tumor length (mm) , b is the tumor width (mm) . Length and width were measured vertically.

Calculated the relative mean of tumor volumes by the following formula: RTV (Relative Tumor Volume) =$V_t/V_0$, wherein, $V_0$ is the average tumor volume measured during group assignment, and $V_t$ is the average tumor volume at each measurement. Relative tumor proliferation rate T/C (%) =$T_{RTV}$ / $C_{RTV}$*100% 。($T_{RTV}$: treatment group RTV; Curv: control group RTV) .

$$\text{Tumor growth inhibition rate TGI\%=}\ (1\text{-}T/C)\times100\%$$

ANOVA for statistical analysis: ANOVA analysis was used to compare whether there was a significant difference in the tumor volume between the experimental and control groups. All the data were analyzed with SPSS 17 , and the statistical significance was defined as P<0.05.

D. The result

| Table 4 | | | | |
|---|---|---|---|---|
| Day 15 | Average Tumor Size | TGI (%) | TGI Analysis (TGI>60%) | P-value (ANOVA) |
| Control (PBS) | 3253.76 | - | - | - |
| POV-604-1D1 | 1524.13 | 53 | 2/5 | 0.023** |
| POV-601-3F8 | 2065.03 | 37 | 1/5 | 0.156 |
| POV-601-1A1 | 1357.58 | 58 | 3/5 | 0.012** |

**Example 7: Safety Evaluation of the ORFV Mutant**

A. Experimental design

| Table 5 | | |
|---|---|---|
| Group | Animal Number | Dosage and Frequency |
| Control (PBS) | 3 | 400ul/mouse s.c. |
| POV-601-1A1 | 3 | 400ul/mouse s.c. |
| Control (PBS) | 3 | 0.1ml/mouse i.v., 2 doses within 24h |
| POV-601-1A1 | 3 | 0.1ml/mouse i.v., 2 doses within 24h |
| Control (PBS) | 2 | 300ul/mouse s.c., 3 doses, within 24h, one dose every 8h |
| POV-601-1A1 | 2 | 300ul/mouse s.c., 3 doses, within 24h, one dose every 8h |
| s.c.= subcutaneous injection; i.v.= intravenous injection (tail) | | |

B. The testing substance and control
Testing substance: oncolytic virus ORFV POV-601-1A1; Control: PBS
C. Experimental animals: Female C57BL/6 mice, 6-8W, Zhejiang Vital River Laboratory Animal Technology Co., Ltd).
D. Procedures
Female C57BL/6 mice were injected subcutaneously on their right back or through the tail vein with the testing substance or control. The date of the first injection was defined as Day 0. Mice were randomly assigned to groups and injection was performed according to Table 5. The experiment was completed on day 15. Mice were weighed daily for the first week and every two to three days thereafter for the duration of the study
Mice were weighed using the ML1602T electric balance (Mettler). The formula below was used to calculate the relative weight change:

$$\text{Relative weight change (\%)} = [\text{weight}_{day_i} / \text{weight}_{day_0}] \text{ x } 100。$$

The results are shown in Figure 4

**Example 8: Modulation of the Immune System by the ORFV Mutants**

A. Experimental Design

| Table 6 | | | |
|---|---|---|---|
| Group | Animal Number | Route of Administration | Dosage and Frequency |
| Control (PBS) | 3 | - | - |
| POV-601-1A1 | 3 | i.v. | 40ul/mouse, a total of 4 doses |
| POV-601-1A1 | 3 | i.t. | 40ul/mouse, a total of 4 doses |
| POV-601-1A1 | 3 | i.t. + i.v. | First i.t. 40ul/mouse, followed by i.v., 0.1ml/mouse |
| POV-601-1A1 | 3 | i.v. + i.t. | First i.v. 0.1ml/mouse, followed by i.t. , 1h after i.v., 40ul/ mouse |
| i.t.= intratumoral injection; i.v.= intravenous injection (tail) | | | |

E. The testing substance and control:

Testing substance: oncolytic virus POV-601-1A1; Control: PBS

B. Experimental animals: female Balb/c-nude, 6W, Beijing Huafukang Biotechnology Co., Ltd

C. Procedure

Implanted female Balb/c-nude mice subcutaneously on their bilateral back with C-33A cells (Cell Bank of Type Culture Collection Committee of Chinese Academy of Sciences ) resuspended in PBS; the cell density was $1\times10^8$ /ml and the implantation dose was 0.1ml/mice. The date of cell implantation was defined as Day 0. When the average volume of the tumors reached about $100mm^3$, injection was performed according to Table 6. The experiment was completed 24h after the last injection. Upon completion of the experiment, blood and tumors were collected from mice. Tumors were cut, digested and resuspended in PBS into a single cell suspension. Cells were then labeled with fluorescent antibodies (Anti-mouse CD45 APC-eFluor 780, Anti-mouse CD49b-PE, Anti-mouse CD69 APC) , and analyzed by a flow cytometer (Thermo, Model AFC2) , see Figure 5 (sub panels A-C) .

**Example 9 *In Vitro* Cell Infection Effects of the ORFV Mutant**

Viral strain: POV-601-1A1

1) Trypsinized the cultured tumor cells and counted them. Cells were seeded into 96-well plates at $1.5\times10^4$cell/well and were cultured for 24 hours before exposing to the virus;

2) Diluted the viral solution with a 2% FBS complete medium to 4 different concentrations: $1.5\times10^8$ pfu/ml, $1.5\times10^7$ pfu/ml, $1.5\times10^6$pfu/ml, $1.5\times10^5$pfu/ml;

3) Added the diluted viral solutions into the 96-well cell plates, 100ul/well, and the final MOI of the added virus for each well is of 1000, 100, 10, 1, respectively, and three duplicates were set for each MOI;

4) Set three wells and added the 2% FBS complete medium as the negative control;

5) Rocked gently to let the viral solution cover the surface of the monolayer cell evenly, and ensured that every conners were covered;

6) Transferred the plates seeded with the control or the viral solution back into a 37°C, 5% CO2 incubator, and cultured cells for 72h;

7) Took out the 96-well plates from the incubator, and added 10ul of Alamar Blue Cell Viability Reagent (Invitrogen, Cat#: 2072060) into each well, rocked the plates gently to let the reagent (dye) disperse evenly. Covered the plates with aluminum foils, transferred them back into the 37°C incubator, and incubated without light;

8) After the incubation was complete, took off the lids, and put the 96-well plates into a microplate reader (Molecular Devices, model SPECTRAMAX M4). Set the excitation wavelength at 560nm and the emission wavelength at 590nm, carried out the absorbance detection, calculated the cell death ratio using the following formula, and analyzed the result: :

$$\left(1-\frac{Absorbance\ of\ a\ particular\ MOI}{Absorbance\ of\ MOI\ 0}\right)\times 100\%;$$

9) The result has shown that the POV-601-1A1 virus had an infectivity to melanoma cancer cell (B16-F10) , bladder cancer cell (MB49) , liver cancer cell (Hepa1-6) , colon cancer cell (CT26) , human cervical cancer cell (C-33A) , human ovarian adenocarcinoma (SK-OV-3) , and etc, and viral infection was increased with a increase in MOI. However, it barely infected Vero cells.

Table 7

The Result of *In Vitro* Infection of the POV-601-1A1 Virus to Tumor Cells

| MOI | B 16-F10 Melano -ma Cancer Cell | MB49 Mouse Bladder Cancer Cell | C-33A Human Cervical Cancer Cell | Hepa1-6 Mouse Liver Cancer Cell | CT26. WT Mouse Colon Cancer Cell | SK-OV-3 Human Ovarian Cancer Cell | LLC Mouse Lung Cancer Cell | Vero African Green Monkey Kidney Cell |
|---|---|---|---|---|---|---|---|---|
| 0 | 0.00% | 0.00% | 0.00% | 0.00% | 0.00% | 0.00% | 0.00% | 0.00% |
| 1 | 12.39% | 4.49% | -6.06% | 5.84% | 9.71% | 2.11% | 2.46% | 11.13% |
| 10 | 14.16% | 29.77% | 25.05% | 25.55% | 14.51% | 25.06% | 7.10% | 12.48% |
| 100 | 40.14% | 74.78% | 78.49% | 69.20% | 65.32% | 58.22% | 81.70% | 11.51% |

(continued)

| Table 7 | | | | | | | | |
|---|---|---|---|---|---|---|---|---|
| The Result of *In Vitro* Infection of the POV-601-1A1 Virus to Tumor Cells | | | | | | | | |
| MOI | B 16-F10 Melano -ma Cancer Cell | MB49 Mouse Bladder Cancer Cell | C-33A Human Cervical Cancer Cell | Hepa1-6 Mouse Liver Cancer Cell | CT26. WT Mouse Colon Cancer Cell | SK-OV-3 Human Ovarian Cancer Cell | LLC Mouse Lung Cancer Cell | Vero African Green Monkey Kidney Cell |
| 1000 | 85.60% | 82.94% | 88.88% | 75.29% | 85.53% | 65.16% | 95.04% | -36.81% |

**Example 8: Evaluation of Antitumor Activity of the ORFV Mutants in A Mouse Colon Cancer Model**

A. Experimental design

| Table 8 | | | | |
|---|---|---|---|---|
| Group | Animal Number | Virus Titer | Route of Administration | Dosage and Frequency |
| Control (PBS) | 6 | / | Intratumoral injection (i.t) | First injection upon the day of grouping, followed by one injection every 72h, 40 μL/mouse |
| POV-601-3F8 | 6 | $2.5*10^8$pfu/ml | | |
| POV-601-1A1 | 6 | $2.5*10^8$pfu/ml | | |

B. Experimental animals: Female Balb/c mice, 6-8W, Zhejiang Vital River Laboratory Animal Technology Co., Ltd).
C. Procedures

Implanted female Balb/c mice subcutaneously on their right back with CT-26 cells (Cell Bank of Type Culture Collection Committee of Chinese Academy of Sciences) resuspended in PBS; the cell density was $5\times10^6$ /ml and the implantation dose was 0.1ml/mice. The date of cell implantation was defined as Day 0. When the average volume of the tumors in the control group reached about 100mm$^3$, mice were randomly assigned to different groups based on the tumor size. Injection was performed according to Table 8, and all the mice were euthanized on day 25;
Weighed mice and measured the tumor volumes twice a week throughout the entire study. The tumor volume was measured using a vernier caliper (Mahr GmbH, Model 16ER), and the results are shown in Figure 14.

Calculated the tumor volume by the following formula: tumor volume (mm$^3$) =$a*b^2/2$, wherein a is the tumor length (mm) , b is the tumor width (mm) . Length and width were measured vertically.

Calculated the relative mean of tumor volumes by the following formula: RTV (Relative Tumor Volume) =$V_t/V_0$. Wherein, $V_0$ is the average tumor volume measured during group assignment, and $V_t$ is the average tumor volume at each measurement. Relative tumor proliferation rate T/C (%) =$T_{RTV}$ / $C_{RTV}$*100% 。($T_{RTV}$: treatment group RTV; Curv: control group RTV) .

$$\text{Tumor growth inhibition rate TGI\%=（1-T/C）}\times100\%$$

ANOVA for statistical analysis: ANOVA analysis was used to compare whether there is a significant difference in tumor volumes between the experimental and control groups. All the data were analyzed with SPSS 17 , and the statistical significance was defined as P<0.05.

D. Result

| Table 9 | | | | |
|---|---|---|---|---|
| Day 25 | Average tumor size | TGI (%) | TGI analysis (TGI>60%) | P-value (ANOVA) |
| Control (PBS) | 3587.22 | - | - | - |
| POV-601-3F8 | 2136.75 | 39 | 1/6 | 0.075 |
| POV-601-1A1 | 1757.35 | 51 | 4/6 | 0.028* |

**Example 9 Evaluation of Antitumor Activity of The ORFV Mutants in A Mouse Melanoma Model**

Constructed the ORFV mutants with complete or partial deletions of the coding and/or non-coding regions of the ORFV111 and/or ORFV112 genes with recombinant methods and the (deletion) effects were tested using the methods recorded (described) in Example 6, unless otherwise specified.

| Table 10 (see Figure 15) | | | | | |
|---|---|---|---|---|---|
| Day 18 | Animal Number | Average Tumor Size | TGI (%) | TGI Analysis (TGI>60%) | P-value (ANOVA) |
| Control (PBS) | 6 | 6420 | - | - | - |
| v611a (2.0*$10^8$pfu/ml) | 6 | 2306 | 64 | 3/6 | 0.002** |
| POV-601-1A1 (v601-1A1) (2.0*$10^8$pfu/ml) | 6 | 2812 | 56 | 1/6 | 0.007** |
| v611a: the mutant with a complete deletion of the ORFV112 gene | | | | | |

| Table 11 (see Figure 16) | | | | | |
|---|---|---|---|---|---|
| Cell density 3×$10^6$ /ml<br>Day 19 | Animal Number | Average tumor size | TGI (%) | TGI Analysis (TGI>60%) | P-value (ANOVA) |
| Control (PBS) | 6 | 3983.35 | - | - | - |
| v615a (1.37*$10^8$pfu/ml) | 6 | 1688.99 | 58 | 2/6 | 0.002** |
| v616a (2.0*$10^8$pfu/ml) | 6 | 1747.31 | 56 | 3/6 | 0.002** |
| POV-601-1A1 (v601-1A1) (2.0*$10^8$pfu/ml) | 6 | 1576.46 | 60 | 4/6 | 0.001** |
| v615a: the mutant with a complete deletion of the coding regions of the ORFV111 and ORFV112 genes<br>v616a: the mutant with a complete deletion of the ORFV111 and ORFV112 genes | | | | | |

| Table 12 (see Figure 17) | | | | | |
|---|---|---|---|---|---|
| Cell Density 3×$10^6$ /ml<br>Day 20 | Animal Number | Average Tumor Size | TGI (%) | TGI analysis (TGI>60%) | P-value (ANOVA) |
| Control (PBS) | 6 | 4128.23 | - | - | - |
| v617a (1.0*$10^8$pfu/ml) | 6 | 1552.86 | 62 | 4/6 | 0.008** |
| v618a (1.0*$10^8$pfu/ml) | 6 | 2494.45 | 40 | 3/6 | 0.078 |
| POV-601-1A1 (v601-1A1) (1.0*$10^8$pfu/ml) | 6 | 2092.23 | 50 | 3/6 | 0.030* |
| v617a: the mutant with a complete deletion of the ORFV111 gene<br>v618a: the mutant with a complete deletion of the coding region in the ORFV111 gene | | | | | |

## MATERIAL DEPOSIT

The following material has been deposited with the China Center for Type Culture Collection in accordance with the provisions of the Budapest Treaty (CCTCC) (Wuhan University, Wuhan, China, 430072):

| Material | CCTCC Deposit Number | Deposit Date |
|---|---|---|
| The ORFV Mutant Virus POV-601-1A1 (v601-1A1) | V202029 | 2020-05-19 |

## REFERENCES

1. Hanahan, D., Weinberg, R.A. (2011). Hallmarks of cancer: the next generation. Cell, 144(5):646-74.
2. Miest, T.S., Cattaneo, R. (2014). New viruses for cancer therapy: meeting clinical needs. Nat Rev Microbiol, 12(1):23-34.
3. Burke, J., Nieva, J., Borad, M.J., Breitbach, C.J. (2015). Oncolytic viruses: perspectives on clinical development. Curr Opin Virol, 13:55-60.
4. Seymour, L.W., Fisher, K.D. (2016). Oncolytic viruses: finally delivering. Br J Cancer, Feb 16; 114(4):357-361.
5. Harrington, K.J., Puzanov, I., Hecht, J.R., Hodi, F.S., Szabo, Z., Murugappan, S., Kaufman, H.L. (2015). Clinical development of talimogene Laherparepvec (T-VEC): a modified herpes simplex virus type-1-derived oncolytic immunotherapy. Expert Rev Anticancer Ther, 15(12): 1389-1403.
6. Yin, Z. and Liu, J.H. (1997) Animal Virology (Second Edition), Science Press, 977-978
7. Wang, Z.J. (2018) Research, development and quality control of Biopharmaceuticals (third edition), Science Press.
8. CDC (2006). Orf Virus Infection in Humans-New York, Illinois, California, and Tennessee, 2004-2005. Morbidity and Mortality Weekly Report, 55(3):65-68.
9. Kirn, D.H., Thorne, S.H. (2009). Targeted and armed oncolytic poxviruses: a novel multimechanistic therapeutic class for cancer. Nat Rev Cancer, 9:64-71.
10. McFadden, G. (2005). Poxvirus tropism. Nat Rev Microbiol, 3(3):201-213.
11. Wang, R., Wang, Y., Liu, F., Luo, S. (2018). Orf virus: A promising new therapeutic agent. Rev Med Virol, e2013.
12. Rintoul, J.L., Lemay, C.G., Tai, L.H., Stanford, M.M., Falls, T.J., Bridle, B.W., Souza, C.T., Daneshmand, M., Ohashi, P.S., Wan, Y., Lichty, B.D., Mercer, A.A., Auer, R.C., Atkins, H.L., Bell, J.C. (2012). ORFV: a novel oncolytic and immune stimulating parapoxvirus therapeutic. Mol Ther, 20(6):1148-1157.
13. Seet, B.T., McCaughan, C.A., Handel, T.M., Mercer, A., Brunetti, C., McFadden, G., Fleming, S.B. (2003). Analysis of an orf virus chemokine-binding protein: shifting ligand specificities among a family of poxvirus viroceptors. Proceedings of the National Academy of Sciences, 100(25):15137-15142.
14. Bergqvist, C., Kurban, M., Abbas, O. (2017). Orf virus infection. Rev Med Virol, 27(4).
15. Liu, W., Yang, K.K., Yin, D.D., Wang, Y.H., Yu, Z.R., Jiang, S.D., Li, C.F., Li, Y.D., Wang, Y. (2018). Prokaryotic expression and subcellular localization of dUTPase gene of orf virus, Chinese Veterinary Science, 7:818-823
16. Irwin, C.R., Hitt, M.M., Evans, D.H. (2017). Targeting Nucleotide Biosynthesis: A Strategy for Improving the Oncolytic Potential of DNA Viruses. Front Oncol, 7:229.
17. Fleming, S.B., McCaughan, C., Lateef, Z., Dunn, A., Wise, I.M., Real, N.C., Mercer, A.A. (2017). Deletion of chemokine binding protein gene from the parapoxvirus orf virus reduces virulence and pathogenesis in sheep. Front Microbiol, 8:46.
18. Twumasi-Boateng, K., Jessica, L.P., Eunice Kwok, Y.Y., John, C.B., Nelson, B.H. (2018). Oncolytic viruses as engineering platforms for combination immunotherapy. Nat Rev Cancer, 18:419-432.
19. Diel, D.G., Lou, S., Delhon, G., Peng, Y., Flores, E.F., Rock, D.L. (2011). A nuclear inhibitor of NF-kappaB encoded by a poxvirus. J Virol, 85(1):264-275.
20. Son, S.J., Harris, P.W., Squire, C.J., Baker, E.N., Kent, S.B., Brimble, M.A. (2014). Total Chemical Synthesis of an Orf Virus Protein, ORFV002, an Inhibitor of the Master Gene Regulator NF-κB. Biopolymers (Peptid Science), 102(2):137-144.
21. Wang, G.X., Shang, Y.J., Chen, J.T., Lu, Z.L., Zhang, K.S., Liu, X.T. (2012). Isolation and identification of orf virus in Hubei Province, Advances in Animal Medicine, 033 (011): 37-40.


## SEQUENCEAS

| SEQ ID NO: | Description | Sequence |
|---|---|---|
| 1. | Complete sequence of the CBP protein (287 aa), underlined: the signal peptide | **MKAVLLLALLGAFTNA**APLLSNQRLGSAEEEKFCSTHQGEVHARFWLQMRVGVRHSPLYTPSNMCMMDIEDSTDTEDSTMEKEYTSTATGDADGLNVSVALIGEGVSIPLSYIGLRFNPSLTDGYLYVNVSSRAPWDQQTLDLSANDGWGIKQVLEKEILAIQIGCDNQKFPEEPTTTQPPSPVTTTLSSTTLDPNDENTDTTPTTTGDSVDGKRNPDDFDFSLIVDPRCVTSVNLHFEIKDACMDHKESSPLSLKGEYGDGELVRKEIKNVGKDHNMCSLNLSPGH |
| 2. | Mature sequence of the CBP protein (271 aa) | APLLSNQRLGSAEEEKFCSTHQGEVHARFWLQMRVGVRHSPLYTPSNMCMMDIEDSTDTEDSTMEKEYTSTATGDADGLNVSVALIGEGVSIPLSYIGLRFNPSLTDGYLYVNVSSRAPWDQQTLDLSANDGWGIKQVLEKEILAIQIGCDNQKFPEEPTTTQPPSPVTTTLSSTTLDPNDENTDTTPTTTGDSVDGKRNPDDFDFSLIVDPRCVTSVNLHFEIKDACMDHKESSPLSLKGEYGDGELVRKEIKNVGKDHNMCSLNLSPGH |
| 3. | Complete sequence of the ORFV112 gene (1162 nt) | ttgctgctgctatgtcgtgcccgactctgtgcgacaaagaagcagcttaccagacgactcttcgttcatgttctctaagcaagaacactggagtgagttgccatttccgtctccaaccatataattagcatccttgtttttatcctgtatttttatcagtttttagctagttaaaacataaatagtaaagctaaaaagaggagttctggaatcttgcaacgacaggatgaaggcggtgttgttgctagcgctactgggagcgttcaccaacgcagcgcctttgttaagcaaccagcgtcttggcagtgcagaagaagaaaaattctgctcgacgcatcagggcgaagtgcacgccaggttctggcttcagatgcgcgtgggtgtacgacacagtccgctctacactcccagcaacatgtgcatgatggacatagaagactctacggacacagaagactccacgatggaaaaagaatacacgtctacggcaacgggtgatgcggacggattgaacgtgtccgtagcactaattggagaaggcgtgagcataccgctaagttacataggccttagatttaacccgtcgcttacagatggctacctgtacgtcaacgtctcgtcacgggctccttgggatcaacagactctggacctatccgcgaacgacggctggggtatcaaacaggttctagaaaaagagatactggccatccagatagggtgcgacaaccaaaaatttcccgaagaacccacaactacccaaccccccctcacctgtcacaacaacgctttcctcaacaactctagatccgaatgacgaaaacacagacactacgccgacaaccaccggcgacagtgtagacggaaagcgcaatccagatgactttgacttctcgctgatcgtggacccccgatgcgtgacctctgtaaacctgcactttgagattaag |

| | | |
|---|---|---|
| | | gacgcgtgcatggaccacaaagaatcgtcgccgttgtcgctgaagggggaatatggagacggcgaactagtaagaaaagaaatcaaaaacgtgggaaaggatcacaatatgtgcagtcttaacctcagccctggccattgagctgtttttattcggcaatataataggtgattattgaacattaaacaaaacttatcccacaacgccgcaaca |
| 4. | 5' end partially deleted ORFV112 gene (624 nt) | ggcgtgagcataccgctaagttacataggccttagatttaacccgtcgcttacagatggctacctgtacgtcaacgtctcgtcacgggctccttgggatcaacagactctggacctatccgcgaacgacggctggggtatcaaacaggttctagaaaaagagatactggccatccagatagggtgcgacaaccaaaaatttcccgaagaacccacaactacccaaccccccctcacctgtcacaacaacgctttcctcaacaactctagatccgaatgacgaaaacacagacactacgccgacaaccaccggcgacagtgtagacggaaagcgcaatccagatgactttgacttctcgctgatcgtggacccccgatgcgtgacctctgtaaacctgcactttgagattaaggacgcgtgcatggaccacaaagaatcgtcgccgttgtcgctgaagggggaatatggagacggcgaactagtaagaaaagaaatcaaaaacgtgggaaaggatcacaatatgtgcagtcttaacctcagccctggccattgagctgtttttattcggcaatataataggtgattattgaacattaaacaaaacttatcccacaacgccgcaaca |
| 5. | ORFV112 gene-specific forward primer | catgcccaac tgctacttcg |
| 6. | ORFV112 gene-specific reverse primer | acctgtttga taccccagcc |
| 7. | Forward primer for amplification of the ORFV112 gene's left arm | gaccatgattacgccaagcttcgtgatttgggaatatgcacc |
| 8. | Reverse primer for | aaaataaaatttcaatttttataacttcgtatagcatacattatacgaagttatgcagcattaactgcgcgctggg |

| | | |
|---|---|---|
| | amplification of the ORFV112 gene's left arm | |
| 9. | Forward primer for amplification of the ORFV112 gene's right arm | tttttttggaatataaatagctagcttaattaacttatcccacaacgccgcaac |
| 10. | Reverse primer for amplification of the ORFV112 gene's right arm | aaaacgacggccagtgaattcgtatctatcgtcgggaccag |
| 11. | Forward primer for amplification of the EGFP gene | aaaaattgaaattttattttttttttttggaatataaataaccggtcgccaccatggtgagcaag |
| 12. | Reverse primer for amplification of the EGFP gene) | tatttatattccaaaaaaaaaaaataaaatttcaatttttataacttcgtatagcatacattatacgaagttatacgcgttaagatacattgatgagttt |
| 13. | Forward primer for | gaccatgattacgccaagcttcgtgatttgggaatatgcacc |

| | | |
|---|---|---|
| | amplification of the linear CBP shuttle vector | |
| 14. | Reverse primer for amplification of the linear CBP shuttle vector | aaaacgacggccagtgaattcgtatctatcgtcgggaccag |
| 15. | Forward primer for amplification of the px330 backbone | gtaagaattcctagagctcgctgatcagcc |
| 16. | Reverse primer for amplification of the px330 backbone | tggcaccggtccaacctgaaaaaaagt |
| 17. | Forward primer for amplification of Cas9 without the NLS (sequence) | ttggaccggtgccaccatggacaagaagt |
| 18. | Reverse primer for | ggctgatcagcgagctctaggaattcttac |

| | | |
|---|---|---|
| | amplification of Cas9 without the NLS (sequence) | |
| 19. | PCR identification primer 1 (OVM38F/R) – Forward primer | gagacgccgccgagcaactt |
| 20. | PCR identification primer 1 (OVM38F/R) – Reverse primer | tgcagcacttcctggacatcg |
| 21. | PCR identification primer 2 (OVM43F/R) – Forward primer | ggacgatcctgtgcggtag |
| 22. | PCR identification primer 2 (OVM43F/R) – Reverse primer | ttgtcggcgtagtgtctgtg |

| | | |
|---|---|---|
| 23. | PCR identification primer 3 (OVM44F/R) – Forward primer | gatgactttgacttctcgctgat |
| 24. | PCR identification primer 3 (OVM44F/R) – Reverse primer | cgacagatccatttcccaat |
| 25. | PCR identification primer 4 (OVM45F/R) - Forward primer | tgcccaactgctacttcgc |
| 26. | PCR identification primer 4 (OVM45F/R) - Reverse primer | caccttgatgccgttcttct |
| 27. | PCR idenitification primer 5 (OVM46F/R) - Forward | agccataccacatttgtagagg |

| | | |
|---|---|---|
| | primer | |
| 28. | PCR identification primer 5 (OVM46F/R) -Reverse primer | agacgatcaccagcacttcc |
| 29. | PCR identification primer 6 (OVM3F2/4R2) – Forward primer | cgtgatttgggaatatgcacc |
| 30. | PCR identification primer 6 (OVM3F2/4R2) – Reverse primer | gcagcattaactgcgcgctggg |
| 31. | Orf virus strain YX AKU76602.1 | MKVVLLLVLLGALTNAAPVGNQRIDSEEKANFCSTHQNEVYARFRLQMRVGVRHSPLYVPSNMCMMDIEDSVMDNEYMSAATGDADGVNVSVALIGEGVSIPLSYIGLGFNPSLADGYLYVNVSSRAPWVQQTQDLSANGSWGIKQVLEQEMLAIQIGCDNQKFPEEPTTTQPPSPVTTTLSSTTLDSNDENADTTPPTTTSASVNRKRNSDDFDFSLLVDPRCVTSVDLHVELRDACIDYKEASLLSLKGKYGDGELVKKEIKDVGKDHNMCSLNLSPGH |
| 32. | Orf virus strain NA1/11 AHZ33810.1 | MKAVLLLALLGAFTNAAPLLESQRSNSEEKANFCSTHNNEVYARFRLQMRVGVRHSPFYTPSNMCMMDIEDSVEDIEESTEKEYASTATGEAAGVNVSVALVGEGVSIPFSYIGLGFNPSLEDSYLYVNVSSRAPWVKQTSDLSANGGWGIKQVLEKELLAIQIGCDNQKFPEEPTTTPPSPVTTTLSSTTPDLNEENTNTTPTTTSASVDRRRNLDDI |

| | | |
|---|---|---|
| | | DFSLLVDPRCVTSVDLHVELRDACMDYKQESPLSLKGKYGDG ELVKKEIKDVGKNYNMCSLNLNPGN |
| 33. | Orf virus strain NZ2 ABA00630.1 | MKAVLLLALLGAFTNAAPLLESQRSNSEEKANFCSTHNDEVYA RFRLQMRVGVRHSPLYTPSNMCMLDIEDSVEDIEESTEKEYAST ATGEAAGVNVSVALVGEGVSIPFSYIGLGFNPSLEDSYLYVNVS SRAPWVKQTSDLSANGGWGIKQVLEKELLAIQIGCDNQKFPEE PTTTPPSPVTTTLSSTTPDLNEENTENTPTTTGASVDRKRNPADD FSLLVDPRCVTSVDLHVELRDAICIDYKQESPLSLKGKYGDGEL VKKEIKDVGKNHNMCSLNLNPGN |
| 34. | Orf virus strain NA17 AYM26053.1 | MKVVVLLALLGALTNAAPVGNQRLNSKEKEDFCSTHQNEVYA RFRLQMRVGVRHSPLYVPSNMCMMDIEDSVDDIEEDSIIVKEFT STATGEAAGVNVSVALVGEGVSIPFSYIGLGFNPSLEDSYLYVN VSSRAPWVQQTPDLSANDSWGIKQVLEKELLAIQIGCDNQKFP EEPTTTQPPSLVTTTLSSTTLDLNDENTDTTPPTTTSASVNKKRN PDDFDFSLLVDPRCVTSVDLHVELRDACIDYKETSQLSLKGEYG DGELIKKEIKDVGKDHNMCSLNLNPGN |
| 35. | Orf virus strain OV-SA00 AAR98337.1 | MKVVLLLVLLGAFTNAAPLVGNQRLDSEEKANFCSTHQNEVY ARFRLQMRVGVRHSPLYTPSNMCMMDIEDSIMDIENSTEKEYT SAATGDANGVNVSVALIGEGVSIPLSYIGLGFNPSLADGYLYVN VSSRAPWVQQTLDLSANGGWGINQILEKELLAIQIGCDNQKFPE EPTTTQPPSPVTTTLSSTTLDLTDENTDTTPLTTTSASVNRKRNP DDFDFSLLVDPRCVTSVDLHVELRDACIDYKEASQLSLKGKYG DGELVKKEIKDVGKDHNMCSLNLSPGH |
| 36. | Orf virus strain OV-IA82 AAR98207.1 | MKAAAVLLLALLGAFTNAAPVSNQRLGSEEKEKFCSTHHDEV YARFRLQMRVGVRHSPLYVPSNMCMMDIEDSTDDIEESTEKEY TSTATGEAAGVNVSVALVGEGVKIPFSYIGLGFNPSTDGYLYV NVSSRAPWVQQTPDLSANSGWGIKQVLEKELLAIQIGCDNQKF PEEPTTTPSLVTTTLSPTTTLNPNNENTDTTPTPTGASVDGKRNP DDIDFSLIVDPRCVTSVNLHFELKDACMDYKKESPLSLKGKYG DSELVKQEIKDVGKNHNMCSLNLSPGH |
| 37. | Orf virus strain GO | MKVVLLLVLLGALTNAAPVGNQRIDSEEKANFCSTHQNEVYA RFRLQMRVGVRHSPLYTPSNMCMMNIEDSMMDNEYTSTATGD ADGVNVSVALMGKGVSIPLSYIGLGFNPLLADGYLYVNVSSRA |

| | | |
|---|---|---|
| | AKU76734.1 | PWVQQTPDLSANGGWGIKQVLEEEILAIQIGCDNQKFLEEPTTTQPPSLVTTTLSSTTLGLTDENTDTTPTTTSASVDRKRNLDDIDFSLLVDTRCVTSVNLHFEIKDACMDYKKESPLMMKGGYGDGELVRKEIKYVGTNHTMCSLNLSPGY |
| 38. | Orf virus strain SJ1 AKU76990.1 | MKVVVLLALLGALTNAAPVGNQRLNSKEKEDFCLTHPDEVYARFRLHMRVGVRHSPLYTPSNMCMMNIEDSMMDNEYTSTATGDADGVNVSVALMGKGVSIPLSYIGLGFNPLLADGYLYVNVSSRAPWVQQTPDLSANGGWGIKQVLEEEILAIQIGCDNQKFLEEPTTPQPPSLVTTTLSSTTLGLTDENTDTTPTTTSASVDRKRNLDDIDFSLLVDTRCVTSVNLHFEIKDACMDYKKESPLMMKGGYGDGELVRKEIKYVGTNHTMCSLNLSPGY |
| 39. | Orf virus strain SY17 AYN61060.1 | MKAVLLLALLGAFTNAAPVGNQRLDSGEKEKFCSTHQDEVYARFRLQMRVGVRHSPFYTPSNMCMMDIEDSVDDIEEDSIIVKEFTSTATGEADGVNVSVALVGEDVKIPLSYIGLGFNPSTDGYLYVNVSSRAPWVQQTLDLSANDSWGIKQVLEKELLAIQIGCDNQKFPEEPTTTQPPSLVTTTLSSTTPDLNDENTDTTPTTTGASVDRKRNPADFSFSLLVDPRCVTSVDLHVELRDACMEYKETSPLSLKGEYGDGELVKKEIKDVGKNHNMCSLNLNPGN |
| 40. | Orf virus strain OV-HN3/12 ASY92409.1 | MKAVLLLALLGAFTNAAPLLESQRSNSEEKANFCSTHNNEVYARFRLQMRVGVRHSPFYTPSNMCMMDIEDSVEDIEESTEKEYASTATGEAAGVNVSVALVGEGVSIPFSYIGLGFNPSLEDSYLYVNVSSRAPWVKQTSDLSANGGWGIKQVLEKELLAIQIGCDNQKFPEEPTTTPPSPVTTTLSSTTPDLNEENTDTTPTTTSASVDRRRNLDDIDFSLLVDPRCVTSVDLHVELRDACMDYKQESPLSLKGKYGDGELVKKEIKDVGKNYNMCSLNLNPGN |
| 41. | Orf virus strain NP AKU76866.1 | MKVVVLLALLGALTNAAPVGNQRLNSKEKEDFCSTHPDEVYARFRLQMRVGVRHSPLYVPSNMCMMDIEDSVDDIEEDSIIVKEFTSTATGEAAGVNVSVALVGEGVSIPFSYIGLGFNPSLEDSYLYVNVSSRAPWVQQTPDLSANDSWGIKQVLEKELLAIQIGCDNQKFPEEPTTTQPPSLVTTTLSSTTLDLNDENTDTTPPTTTSASVNKKRNPDDFDFSLLVDPRCVTSVDLHVELRDACIDYKETSQLSLKGEYGDGELIKKEIKDVGKDHNMCSLNLNPGN |

| | | |
|---|---|---|
| 42. | Orf virus strain B029 AHH34297.1 | MKAVLLLALLGAFTNAAPLLSNQRLGSAEEEKFCSTHHDKVYARFWLQMRVGVRHSSLYAPSNMCMMDIEDSTVDIEGSTETEDSTVEKEYTSAATGDANGVNVSVALMGEGVSIPLSYIGLGFNPLLKDGYLYVNVSSRAPWDQQTLDLSANDGWGIKQVLEKEILAIQIGCDNQKFPEEPTTTQPPSPVTTTLSPTTTLNPNNENTDTTPTPTGASVDGKRNPDDIDFSLIVDPRCVTSVNLHFEIKDACMDYKQESPLSLKGKYGDGELVKEEIKDVGKNHNMCSLNLSPGH |
| 43. | Orf virus strain YX KP010353.1 | ttgctgctgctatatcgtacctgactctgtgttttaaagacagcagctcaccgactcttcgtccctgttctccaagcaagaacactggagtgatttgccatttccgtctccaacatataattagcatccttgtttttatcttgtctttttatcagtttttatgttagttaaaacataaatagtaaagctaaaaagagtactctggaatcttgcaacagtcaggatgaaggtggtgttgttgctagtgctactgggggcgttaaccaacgcagcgcccgtcggcaaccagcgtattgacagtgaggagaaagcaaatttctgctcgacgcatcaaaatgaagtgtacgccaggttccggcttcagatgcgcgtgggtgtacgacacagtccgctctacgttcccagcaacatgtgcatgatggacatagaagactccgtaatggataatgaatacatgtctgcggccacgggtgatgcggacggagtgaacgtgtccgtggcactaataggagaaggcgtgagcataccgctaagttacataggcctcggatttaacccatcgcttgcagatggctacctgtacgtcaacgtctcgtcacgggctccttgggttcaacagactcaagacttatctgcgaacggcagctggggtattaaacaggttctagaacaagagatgctggccatccagatagggtgcgacaaccaaaaatttcctgaagaacccacaactacccaacctccctcacctgtcacgacaacgctttcctcaacaactctagattcgaatgacgaaaacgcagacactacgccgccgaccaccaccagcgccagtgtaaacagaaagcgcaattcagatgactttgacttctcgctgctcgtagaccccccgatgcgtgacctctgtagacctgcacgtcgagctcagggacgcgtgcatagactacaaagaagcgtcgctgttgtcgctgaaggggaaatatggagacggcgaactagtaaaaaaggagatcaaagacgtgggaaaggatcacaatatgtgcagtcttaacctcagccctggccattgagctgtttttattcggcaatataataggtgattattgaacattaaacaaaacttatcccacaacgccgcaaca |
| 44. | Orf virus strain NA1/11 KF234407.1 | tcaacatccttgtttttatcctgtctttttatcagtttttagctagttaaaacataaatagtaaagctaaaaagaggagttctggagtcttgcaataaccaggatgaaggcggtgttgttgctggcgttactgggagcgttcaccaacgcagcgcctttgttagaaagccagcgttctaacagtgaggagaaagcaaatttctgctcgacgcataataatgaagtgtacgccaggttcaggcttcagatgcgcgtgggtgtacgacacagtcctttctacactcccagcaacatgtgcatgatggacatagaagactccgttgaggacatagaagagtccacagagaaagaatacgcgtctacggccacgggtgaggcggccggagtgaacgtgtcagtggcactagtgggagaaggcgtgagcataccgtttagttacataggccttggattcaacccgtcgcttgaagatagctacctgtacgttaacgtctcgtcacgagctccttgggttaaacagacttcggacctatccgcgaacggc |

| | | |
|---|---|---|
| | | ggctggggtattaaacaggttctagaaaaagagttactggccatccaaatagggtgcgacaaccaaaaatttcccgaagaacccacaactacaccccccctcacctgtcacgacaacgctttcctcaacaactccagatctgaatgaagaaaacacaaacactacgccgacgaccaccagcgccagtgtagacagaaggcgcaatctagatgacattgacttctcgctgctcgtggacccccgatgcgtgacctctgtagacctacacgtcgagctcagggacgcgtgcatggactacaaacaagagtcgccgttgtcgctgaaggggaaatatggagacggcgagctagtaaaaaaggagatcaaagacgtgggaaagaattacaatatgtgcagtcttaacctcaaccctggcaattgagatgtttttattcggcaatataatagggattattgaacattaaacaaaacttatcccacaacgccgcaaca |
| 45. | Orf virus strain NZ2 DQ184476.1 | tcagcatccttgtttttatcctgtctttttatcagtttttagctagttaaaacataaatagtaaagctaaaaagaggagttctggagtcttgcaacaaccaggatgaaggcggtgttgttgctggcgttactgggagcgttcaccaacgcagcgcctttgttagaaagccagcgttctaacagtgaggaaaaagcaaatttctgctcgacgcataatgatgaagtgtacgccaggttcaggcttcagatgcgcgtgggtgtacgacacagtccgctctacactcccagcaacatgtgcatgctggacatagaagactccgttgaggacatagaagagtccacagaaaaagaatacgcgtctacggccacgggtgaggcggccggagtgaacgtgtcagtggcactagtgggagaaggcgtgagcataccgtttagttacataggccttggattcaacccgtcgcttgaagatagctacctgtacgtcaacgtctcgtcacgagctccttgggttaaacagacttcggacctatccgcgaacggcggctggggtatcaaacaggttctagaaaaagagttactggccatccaaatagggtgcgacaaccaaaaatttcccgaagaacccacaactacaccccccctcacctgtcactacaacgctttcctcaacaactccagatctgaatgaagaaaacacagaaaatacgccgacgaccaccggcgccagtgtagacagaaagcgcaatccagctgacattgacttctcgctgctcgtggacccccgatgcgtgacctctgtagacctgcacgtcgagctcagggacgcgtgcatagactacaaacaagagtcgccgttgtcgctgaaggggaaatatggagacggcgaactagtaaaaaaggagattaaagacgtgggaaagaatcacaatatgtgcagtcttaacctcaaccctggcaattgagctgtttttattcggcaatataataggtgattattgaacattaaacaaaacttatcccacaacgccgcaac |
| 46. | Orf virus strain NA17 MG674916.2 | ttgctgctgctatatcgtacctgactctgtgttacaaagacagcagctcaccagactcttcgtccctgttctctaagcaagaacactggagtgagttgccatttccgtctccaacatataattagcatccttgtttttatcttgtctttttatcagtttttatgctagttaaaacataaatagtaaagctaaaaagagtactctggaatcttgcaacaatcaggatgaaggtggtggtgttgctggcgctactgggagcgttaaccaacgcagcgcccgtcggcaaccagcgtcttaacagtaaagagaaagaagatttctgctcgacgcatcaaaatgaagtgtacgccaggttccggcttcagatgcgcgtgggtgtacgacacagtccgctctacgttcccagcaacatgtgcatgatggacatagaagactctgttgatgacatagaagaagactccattatagtaaaagaattcacgtctacagccacgggtgaggcggccggagtgaacgtgtccgtggcactagtgggagaaggcgtgagcataccgtttagttacataggccttggattcaacccgtcgcttgaagatagctacttgtacgtcaacgtct |

| | | |
|---|---|---|
| | | cgtcacgagctccttgggttcaacagactccagacttatctgcaaacgacagctggggtattaaacaggttctagaaaaagagttactggccatccaaatagggtgcgacaaccaaaaatttcccgaagaacccacaaccacccaacccccctcacttgtcacgacaacgctttcctcaacaactctagatctgaatgacgaaaacacagacactacgccgccaaccaccaccagcgccagcgtaaacaaaaagcgcaatccagatgactttgacttctcgctgctcgtggacccccgatgcgtgacctctgtagacctgcacgtcgagctcagggacgcgtgcatagactacaaagaaacgtcgcagttgtcgctgaagggggaatatggagacggcgaactaataaaaaaggagatcaaagacgtgggaaaggatcacaatatgtgcagtcttaacctcaaccctggcaattgagctgtttttattcggcaatataataggtgattattgaacattaaacaaaacttatcccacaacgccgcaaca |
| 47. | Orf virus strain OV-SA00 AY386264.1 | ttgctgcgctatgtcgtgcccgactctgtgcgacaaagacagaagctcaccaaactcttcgtctctgttctccaagcaagaacactggagtgatttgccatttccgtctccaacatataattagcatccttgtttttatcttgtctttttatcagtttttatgttagttaaaacataaatagtaaagctaaaaagagtactctggaatcttgcaacagtcaggatgaaggtggtgttgttgctagtgctactgggagcgtttaccaacgcagcgcctttggtcggcaaccagcgtcttgacagtgaggagaaagcaaatttctgctcgacacatcaaaatgaagtgtacgccaggttccggcttcagatgcgcgtgggtgtacgacacagtccgctctacactcccagcaacatgtgcatgatggacatagaagactccattatggacatagaaaactccacagaaaaagaatacacgtctgcagccacgggtgatgcgaacggagtgaacgtgtccgtggcactaataggagaaggcgtgagcataccgctaagttacataggcctcggattcaacccatcgcttgcagatggctacctgtacgtcaacgtctcgtcacgagctccttgggttcaacagactctagacctatccgcgaacggcggctggggtattaatcagattctagaaaaagagttactggccatccaaatagggtgcgacaaccaaaaatttcccgaagaacccacaactacccaacctccctcacctgtcacgacaacgctttcctcaacaacgctagatctgactgacgaaaacacagacactacgccgctgaccaccaccagcgccagtgtaaacagaaagcgcaatccagatgactttgacttctcgctgctcgtggacccccgatgcgtgacctctgtagacttgcacgtcgagctcagggacgcgtgcatagactacaaagaagcgtcgcagttgtcgctgaaggggaaatatggagacggcgaactagtaaaaaaggagatcaaagacgtgggaaaggatcacaatatgtgcagtcttaacctcagccctggccattgagatgtttttattcggcaattaataggtgattattgaacattaaacaaaacttatcccacaacgccgcaaca |
| 48. | Orf virus strain OV-IA82 AY386263.1 | ttgctgctgctatgtcgtgcccgactctgtgcgacaaagacagcggctaaccagactcttcgtccctgttctccaagcaaaaaactggagtgagttgccatttccgtctccaaccatataattagcatccttgtttttatcctgtatttttatcagtttttatgctagttaaaacataaatagtaaggctaaaaagaagagttctagaatcttgcaacaaccaagatgaaggcggcggcggtgttgttgctagcgctactgggagcgttcaccaacgcagcgcccgtcagcaaccagcgtcttggcagtgaggagaaagaaaaattctgctcgactcatcatgacgaagtgtacgccaggttccggcttcagatgcgcgtgggtgtacgacacagtccgctctacgttccca |

| | | |
|---|---|---|
| | | gcaacatgtgcatgatggacatagaagactctacggatgacatagaagagtccacggagaaagaatacacgtctacggctacgggtgaggcggccggagtgaacgtgtccgtggcactagtgggagaaggcgtgaaaataccgtttagttacataggccttggattcaacccatctacagatggctacctgtacgtcaacgtctcgtcacgagctccttgggttcaacagactccagacctatccgcgaacagcggctggggtattaaacaggttctagaaaaagagttactggccatccagatagggtgcgacaaccaaaaatttcccgaagaacccacaactacccccctcacttgtcacgacaacgctttccccaacaacgactttaaatccgaataacgaaaacacagacactacgccgacgcccaccggcgccagtgtagacggaaagcgcaatccagatgacattgacttctcgctgatcgtggacccccgatgcgtgacctctgtaaacctgcacttcgagctcaaggacgcgtgcatggactacaaaaaagagtcgccgttgtcgctgaaggggaaatatggagacagtgaactagtaaaacaggagattaaagacgtgggaaagaatcacaatatgtgcagtcttaacctcagccctggccattgagctgtttttattcggcaatataataaggtgattattgaacattaaacaaaacttatcccacaacgccgcaaca |
| 49. | Orf virus strain GO KP010354.1 | ttgctgcgctatgtcgtgcccgactctgtgcgacaaagacagaagctcaccaaactcttcgtctctgttctccaagcaagaacactggagtgatttgccatttccgtctccaacatataattagcatccttgtttttatcttgtctttttatcagtttttatgttagttaaaacataaatagtaaagctaaaaagagtactctggaatcttgcaacagtcaggatgaaggtggtgttgttgctagtgctactgggggcgttaaccaacgcagcgcccgtcggcaaccagcgtattgacagtgaggagaaagcaaatttctgctcgacgcatcaaaatgaagtgtacgccaggttccggcttcagatgcgcgtgggtgtacggcacagtccgctctacactcccagcaacatgtgcatgatgaacatagaagactccatgatggataatgaatacacgtctacggccacaggtgatgcggacggagtgaacgtgtctgtggcactaatgggaaaaggcgtgagcataccgttaagttacataggcctcggattcaacccattgcttgcagatggctacctgtacgtcaacgtctcgtcacgagctccttgggttcaacaaactccggacctatccgcgaacggcggctggggtattaaacaggttctagaagaagagatactggccatccagatagggtgcgacaaccaaaaatttctagaagaacccacaacaacccaacccccctcacttgtcacgacaacgctttcctcaacaacgctaggtctgactgacgagaacacagacactacgccgacgaccaccagcgccagtgtagacagaaagcgcaatctagatgacattgacttctcgctgctcgtagacacccgatgcgtgacctcggtaaacctgcacttcgagattaaggacgcgtgtatggactacaaaaaagagtcgccgttgatgatgaagggggggatatggagacggcgaactagtaagaaaagagatcaaatacgtgggaacgaatcacactatgtgcagtctcaacctcagccctggctattgatttgtttttattcggcaatataataggtgattattgaacattaaacaaaacttatcccacaacgcctcaaca |
| 50. | Orf virus strain SJ1 KP010356. | ttgctgcgctatgtcgtgcccgactctgtgcgacaaagacagaagctcaccaaactcttcgtctctgttctccaagtaagaacactggagtgatttgccatttccgtctccaacatataattagcatccttgtttttatcttgtctttttatcagtttttatgctagttaaaacataaatagtaaagctaaaaagagtactctggaatcttgcaacagtcaggatgaaggtggtggtgttgctggcgctactgggagcgttaaccaacgcagcgcccgtt |

| | | |
|---|---|---|
| | 1 | ggcaaccagcgtcttaacagtaaagagaaagaagatttctgcttgacgcatccggatgaagtgtatgccaggttccggcttcatatgcgcgtgggtgtacgacacagtccgctttacactcccagcaacatgtgcatgatgaacatagaagactccatgatggataatgaatacacgtctacggccacgggtgatgcggacggagtgaacgtgtctgtggcactaatgggaaaaggcgtgagcataccgttaagttacataggcctaggattcaacccattgcttgcagatggctacttgtacgtcaacgtctcgtcacgagctccttgggttcaacagactccggacctatccgcgaacggcggctggggtattaaacaggttctagaagaagagatactggccatccagatagggtgcgacaaccaaaaatttctagaagaacccacaactccccaaccccccctcacttgtcacgacaacgctttcctcaacaacgctaggtctgactgacgagaacacagacactacgccgacgaccaccagcgccagtgtagacagaaagcgcaatctagatgacattgacttctcgctgctcgtagacacccgatgcgtgacctcggtaaacctgcacttcgagatcaaggacgcgtgtatggactacaaaaaagagtcgccgttgatgatgaagggggggatatggagacggcgaactagtaagaaaagagatcaaatacgtgggaacgaatcacactatgtgcagtctcaacctcagccctggctattgatttgtttttattcggcaatataataggtgattattgaacattaaacaaaacttatcccacaacgccgcaaca |
| 51. | Orf virus strain SY17 MG712417. 1 | tcagcatccttgtttttatcctgtctttttatcagtttttagctagttaaaacataaatagtaaagctaaaaagaggagttctggagtcttgtaacaaccaggatgaaggcggtgttgttgctggcgttactgggagcgttcactaacgcagcgcccgtcggcaaccagcgtctcgatagtggggagaaagaaaaattctgctcgacgcatcaggatgaagtgtacgctaggttcaggcttcagatgcgcgtgggtgtacgacacagtcctttctacactcccagcaacatgtgcatgatggacatagaagactccgttgatgacatagaagaagactccattatagtaaaagaatttacgtctacggccacgggtgaggcggacggagtgaacgtgtccgtggcactagtgggagaagacgtgaaaataccgttaagttacataggccttggattcaacccatctacagatggctacctgtacgtcaacgtctcgtcacgagctccttgggttcaacagactctagacctatctgcgaacgacagctggggtattaaacaggttctagaaaaagagttactggccatccaaatagggtgcgacaaccaaaaatttcctgaagaacccacaaccacccaacctccctcacttgtcacgacaacgctttcctcaacaactccagatctgaatgacgaaaacacagacactacgccgacgaccactggcgccagtgtagacagaaagcgcaatccagctgactttagtttctcgctgctcgtggacccccgatgcgtgacttctgtagacctgcacgtcgagctcagggacgcgtgcatggagtacaaagaaacgtcgccgttgtcgctgaagggggaatatggagacggcgaactagtaaaaaaggagatcaaagacgtgggaaagaatcacaatatgtgcagtcttaacctcaaccctggcaattaagctgtttttattcggcaatataataggtgattattgaacattaaacaaaacttatcccacaacgccgcaaca |
| 52. | Orf virus strainOV-HN3/12 KY053526. | tcaacatccttgtttttatcctgtctttttatcagtttttagctagttaaaacataaatagtaaagctaaaaagaggagttctggagtcttgcaataaccaggatgaaggcggtgttgttgctggcgttactgggagcgttcaccaacgcagcgcctttgttagaaagccagcgttctaacagtgaggagaaagcaaatttctgctcgacgcataataatgaagtgtacgccaggttcaggcttcagatgcgcgtgggtgtacgacacagtcctttc |

| | | |
|---|---|---|
| | 1 | tacactcccagcaacatgtgcatgatggacatagaagactccgttgaggacatagaagagtccacagagaaagaatacgcgtctacggccacgggtgaggcggccggagtgaacgtgtcagtggcactagtgggagaaggcgtgagcataccgtttagttacataggccttggattcaacccgtcgcttgaagatagctacctgtacgttaacgtctcgtcacgagctccttgggttaaacagacttcggacctatccgcgaacggcggctggggtattaaacaggttctagaaaaagagttactggccatccaaatagggtgcgacaaccaaaaatttcccgaagaacccacaactacaccccccctcacctgtcacgacaacgctttcctcaacaactccagatctgaatgaagaaaacacagacactacgccgacgaccaccagcgccagtgtagacagaaggcgcaatctagatgacattgacttctcgctgctcgtggacccccgatgcgtgacctctgtagacctacacgtcgagctcagggacgcgtgcatggactacaaacaagagtcgccgttgtcgctgaaggggaaatatggagacggcgagctagtaaaaaaggagatcaaagacgtgggaaagaattacaatatgtgcagtcttaacctcaaccctggcaattgagatgtttttattcggcaatataatagggattattgaacattaaacaaaacttatcccacaacgccgcaaca |
| 53. | Orf virus strain NP KP010355. 1 | ttgctgcgctatgtcgtacctgactctgtgtacaaagacagcagctcaccagactcttcgtccctgttctctaagcaagaacactggagtgagttgccatttccgtctccgacatataattagcatccttgtttttatcctgtctttttatcagtttttatgctagttaaaacataaatagtaaagctaaaaagagtactctggaatcttgcaacaatcaggatgaaggtggtggtgttgctggcgctactgggagcgttaaccaacgcagcgcccgtcggcaaccagcgtcttaacagtaaagagaaagaagatttctgctcgacgcatccggatgaagtgtacgccaggttccggcttcagatgcgcgtgggtgtacgacacagtccgctctacgttcccagcaacatgtgcatgatggacatagaagactctgttgatgacatagaagaagactccattatagtaaaagaattcacgtctacagccacgggtgaggcggccggagtgaacgtgtccgtggcactagtgggagaaggcgtgagcataccgtttagttacataggccttggattcaacccgtcgcttgaagatagctacttgtacgtcaacgtctcgtcacgagctccttgggttcaacagactccagacttatctgcaaacgacagctggggtattaaacaggttctagaaaaagagttactggccatccaaatagggtgcgacaaccaaaaatttcccgaagaacccacaaccacccaacccccctcacttgtcacgacaacgctttcctcaacaactctagatctgaatgacgaaaacacagacactacgccgccaaccaccaccagcgccagcgtaaacaaaaagcgcaatccagatgactttgacttctcgctgctcgtggacccccgatgcgtgacctctgtagacctgcacgtcgagctcagggacgcgtgcatagactacaaagaaacgtcgcagttgtcgctgaagggggaatatggagacggcgaactaataaaaaaggagatcaaagacgtgggaaaggatcacaatatgtgcagtcttaacctcaaccctggcaattgagctgtttttattcggcgtataataggtgattattgaacattaaacaaaacttatcccacaacgccgcaaca |
| 54. | Orf virus strain | ttgctgctatgtcgtacccgactctgtgctacaaagacagcagctcaccagactcttcgtccctgttctccaagcaagaacactggagtgagttgccatttccgtctccaaccatataattagcatccttgtttttatcct |

| | | |
|---|---|---|
| | B029 KF837136.1 | gtatttttatcagtttttatgctagttaaaacataaatagtaaggctaaaaagaagagttctagaatcttgcaacaaccaagatgaaggcggtgttgttgctagcgctactgggagcgttcaccaacgcagcgcctttgttaagcaaccagcgtcttggcagtgcagaggaagaaaaattctgctcgacgcatcatgacaaagtgtacgccaggttctggcttcagatgcgtgtgggtgtacgacacagttcgctctacgctcccagcaacatgtgcatgatggacatagaagactctacggtggacatagaaggctccacggaaacagaagactccacggtggaaaaagaatacacgtctgcggcaacgggtgatgcgaacggagtgaacgtgtccgtggcactaatgggagaaggcgtgagcataccgctaagttacataggcctcggattcaacccattgcttaaagatggctacctgtacgtcaacgtctcgtcacgggctccttgggatcaacagactctggacctatccgcgaacgacggctggggtattaaacaggttctagaaaaagagatactggccatccagatagggtgcgacaaccaaaaatttcccgaagaacccacaactacccaaccccccctcacctgtcacaacaacgctttccccaacaacgactttaaacccgaataacgaaaacacagacactacgccgacgcccaccggcgccagtgtagacggaaagcgcaatccagatgacattgacttctcgctgatcgtggacccccgatgcgtgacctctgtaaacctgcacttcgagatcaaggacgcgtgcatggactacaaacaagagtcgccgttgtcgctgaaggggaaatatggagacggtgaactagtaaaagaggagattaaagacgtgggaaagaaccacaatatgtgcagtcttaacctcagccctggccattgagctgtttttattcggcaatataataggtgattattgaacattaaacaaaacttatcccacaacgccgcaaca |
| 55. | Forward primer | cgtgatttgggaatatgcacc |
| 56. | Reverse primer | gtatctatcgtcgggaccag |
| 57. | 5' truncated ORFV112 gene | cttatcccacaacgccgcaaca |
| 58. | Full peptide sequence of the hypothetical protein 111 (179 aa) | MSRLQILTSFGQIYAPDEARLREIARDLGICTIKRAFGDMLYGFIDFNPVPLTQVNMLMPNCYFAVNGNLLPCTEDFRLRLPATEISAAYLTKTGRTILCGRDFNIVAPSGFKPSMRLRNLSHVSALVEILELYDESGEYQFVLGPSAQLMLRLMEKENVCLFGRGWCIVDLRKLDVAI* |
| 59. | Full DNA sequence of the | aaatatactgtttgaacgcaaagacgccatgtcgcgacttcaaatactgacctcatttggacaaatctacgcacctgacgaagctcggctgcgagagatcgcgcgtgatttgggaatatgcaccataaaacgcgcattcggcgacatgctgtacggctttatagacttcaacccggtgcccctgacccaagtaaacatgctcat |

| | | |
|---|---|---|
| | ORFV111 gene (794 nt) | gcccaactgctacttcgcggtcaacggcaacctgcttccgtgcacggaggacttccggctcagactcccggcaacggagatctctgcggcctacctgacgaaaacgggacggacgatcctgtgcggtagagacttcaacatagtagcgccgtcggggttcaagccgtccatgcggctgcgcaacctcagtcacgtgtctgcgcttgtagagattttagagctttacgacgagtccggagagtaccaattcgtgctcggccccagcgcgcagttaatgctgcggctaatggagaaggagaatgtctgtctgtttggcagaggttggtgcatagtggacctgcgcaagctggacgtagccatataattgctgctgctatgtcgtgcccgactctgtgcgacaaagaagcagcttaccagacgactcttcgttcatgttctctaagcaagaacactggagtgagttgccatttccgtctccaaccatataattagcatccttgtttttatcctgtatttttatcagtttttagctagttaaaacataaatagtaaagctaaaaagaggagttctggaatcttgcaacgacagg |
| 60. | the 3' truncated type of the ORFV111 gene (485 nt) | aaatatactgtttgaacgcaaagacgccatgtcgcgacttcaaatactgacctcatttggacaaatctacgcacctgacgaagctcggctgcgagagatcgcgcgtgatttgggaatatgcaccataaaacgcgcattcggcgacatgctgtacggctttatagacttcaacccggtgcccctgacccaagtaaacatgctcatgcccaactgctacttcgcggtcaacggcaacctgcttccgtgcacggaggacttccggctcagactcccggcaacggagatctctgcggcctacctgacgaaaacgggacggacgatcctgtgcggtagagacttcaacatagtagcgccgtcggggttcaagccgtccatgcggctgcgcaacctcagtcacgtgtctgcgcttgtagagattttagagctttacgacgagtccggagagtaccaattcgtgctcggccccagcgcgcagttaatgctgc |
| 61. | Orf virus strain YX AKU76601.1 | MSQLQILTSFGQIYAPDEARLREIARDLGICTIKRAFGDMLYGFIDFNPVPLTQVNMLMSDFYFAVNGNLLPCTEDFRLRLPATDISAAYLTKTGRTILCGKDFNIVAPSGFKPSMRLRNLSHVSALVEILEFYSESGEYQFVLGPSAQFMLRLMEKENVCLFGSGWCIVDLRKLDVTI |
| 62. | Orf virus strain NA1/11 AHZ33809.1 | MSRLQILTSFGQIYAPDEARLREIARDLGVCTIKRAFGDMLYGFIDFDPVPLTQVNMLMPNCYFAVNGNLLPCTEDFRLRLPATEISAAYLTRTGRTILCGKDFNIVAPSGFKPSMRLRDLSHVSALVEILEIYNESGEYQFVLGPSAQFMLRLMEKENVCLFGSGWCIVDLRKLDVPI |
| 63. | Orf virus strain NZ2 ABA00629.1 | MSRLQILTSFGQIFAPDEARLREIARDLGICTIKRAFGDMLYGFIDFDPVPLTQVNMLMSNCYFAVNGNLLPCTEDFRLRLPATEISAAYLTRTGRTILCGKDFNIVAPSGFKPSMRLRDLSHVSALVEILEVYDESGEYQFVLGPSAQFMLRLMEKENVCLFGSGWCIVDLRKLDVPI |

| | | |
|---|---|---|
| 64. | Orf virus strain NA17 AYM26052 .1 | MSRLQILTSFGQIYAPDEARLREIARDLGICTIKRAFGDMLYGFIDFNPVPLTQVNMLMSDCYFAVNGNLLPCTEDFRLRLPATEISAAYLTKTGRTILCGKDFNIIAPSGFKPSMRLRNLSHVSALVEILEFYSESGEYQFVLGPSAQFMLRLMEKENVCLFGSGWCIVDLRKLDVTI |
| 65. | Orf virus strain OV-SA00 AAR98336. 1 | MSRLQILTSFGQIYAPDEARLREIARDLGICTIKRAFGDMLYGFIDFNPVPLTQVNMLMSNCYFAVNGNLLPCTEDFRLRLPATEISAAYLTKTGRTILCGKDFNIIAPLGFKPSMRLRNLSHVSALVEILEFYSESGEYQFVLGPSAQFMLRLMEKENVCLFGSGWCIVDLRKLDVTI |
| 66. | Orf virus strain OV-IA82 AAR98206. 1 | MHEGDARITENILFERKDAMSRLQILTSFGQIYAPDEARLREIARDLGICTIKRAFGDMLYGFIDFNPVPLTQVNMLMSNCYFAVNGNLLPCTEDFRLRLPATEISAAYLTRTGRTILCGKDFNIVAPSGFKPSMRLRDLSHVSALVEILELYDESGDYQFVLGPSAQFMLRLMEKENVCLFGNGWCIVDLRKLDVTI |
| 67. | Orf virus strain GO AKU76733. 1 | MSRLQILTSFGQIYAPDEARLREIARDLGICTIKRAFGDMLYGFIDFNPVPLTQVNMLMSDCYFAVNGNLLPCTEDFRLRLPATEISAAYLTKTGRTILCGKDFNIIAPSGFKPSMRLRNLSHVSALVEILEFYSESGEYQFVLGPSAQFMLRLMEKENVCLFGSGWCIVDLRKLDVTI |
| 68. | Orf virus strain SJ1 AKU76989. 1 | MSQLQILTSFGQIYAPDEARLREIARDLGICTIKRAFGDMLYGFIDFNPVPLTQVNMLMSNCYFAVNGNLLPCTEDFRLRLPATEISAAYLTKTGRTILCGKDFNIVAPSGFKPSMRLRNLSHVSALVEILEFYSESGEYQFVLGPSAQFMLRLMEKENVCLFGSGWCIVDLRKLDVTI |
| 69. | Orf virus strain SY17 AYN61059. 1 | MSRLQILTSFGQIYAPDEARLREIARDLGICTIKRAFGDMLYGFIDFNPVPLTQVNMLMPNCYFAVNGNLLPCTEDFRLRLPATEISAAYLTRTGRTILCGKDFNIVAPSGFKPSMRLRDLSHVSALVEILEIYNESGEYQFVLGPSAQFMLRLMEKENVCLFGSGWCIVDLRKLDVTI |
| 70. | Orf virus strain OV-HN3/12 | MSRLQILTSFGQIYAPDEARLREIARDLGVCTIKRAFGDMLYGFIDFDPVPLTQVNMLMPNCYFAVNGNLLPCTEDFRLRLPATEISAAYLTRTGRTILCGKDFNIVAPSGFKPSMRLRDLSHVSALVEILEI |

| | | |
|---|---|---|
| | ASY92408.1 | YNESGEYQFVLGPSAQFMLRLMEKENVCLFGSGWCIVDLRKLDVPI |
| 71. | Orf virus strain NP AKU76865.1 | MSRLQILTSFGQIYAPDEARLREIARDLGICTIKRAFGDMLYGFIDFNPVPLTQVNMLMSDFYFAVNGNLLPCTEDFRLRLPATDISAAYLTKTGRTILCGKDFNIVAPSGFKPSMRLRNLSHVSALVEILEFYSESGEYQFVLGPSAQFMLRLMEKENVCLFGSGWCIVDLRKLDVTI |
| 72. | Orf virus strain B029 AIO03573.1 | MSRLQILTSFGQIYAPDEARLREIARDLGICTIKRAFGDMLYGFIDFNPVPLTQVNMLMSNCYFAVNGNLLPCTEDFRLRLPATEISAAYLTKTGRTILCGKDFNIVAPSGFKPSMRLRDLSHVSALVEILELYDESGEYQFVLGPSAQFMLRLMEKENVCLFGNGWCIVDLRKLDVTI |
| 73. | Orf virus strain YX KP010353.1 | ctgaaaatatactgtttgaatgcaaagacgccatgtcgcaacttcaaatactgacctcatttggacaaatctacgcacccgacgaagctcggctgcgcgagatcgcgcgtgatttgggaatatgcaccataaaacgcgcattcggcgacatgctgtacggctttatagacttcaacccggtgccccttacccaagtaaacatgctcatgtccgacttctacttcgcggtcaacggcaacctgcttccgtgcacggaggacttccggctcagactcccggcgacggatatctctgcggcctacctgacgaaaacgggacggacgatcctgtgcggcaaagacttcaacatagtggcgccgtcagggttcaagccgtccatgcggctgcgcaacctcagtcacgtgtctgcgcttgtagagatcttggagttttacagcgagtccggggagtaccaattcgtgctcggccccagcgcgcagttcatgctgcggctgatggagaaggagaacgtctgtctgttcggcagcggatggtgcatagtggacctgcgcaagctggacgtaaccatataattgctgctgctatatcgtacctgactctgtgttttaaagacagcagctcaccgactcttcgtccctgttctccaagcaagaacactggagtgatttgccatttccgtctccaacatataattagcatccttgtttttatcttgtctttttatcagtttttatgttagttaaaacataaatagtaaagctaaaaagagtactctggaatcttgcaacagtcagg |
| 74. | Orf virus strain NA1/11 KF234407.1 | ctgaaaatatactgtttgaacgcaaagacgccatgtcgcgacttcaaatactgacctcatttggacaaatctacgcacccgacgaagctcggctgcgcgagatcgcgcgtgatttgggagtatgcaccataaaacgcgcattcggcgacatgctgtacggctttatagacttcgacccggttcccctgacccaagtaaacatgctcatgcccaactgctacttcgcggttaacggcaacctgcttccgtgcacggaggacttcaggctcagactcccggcaacggagatctccgcggcctacctgacgagaacgggacggacgatcctgtgcggcaaagacttcaacatagtggcgccgtcagggttcaagccgtccatgcggctgcgcgaccttagtcacgtgtctgcgcttgtagagatcctggaaatctacaacgagtccggggagtaccaattcgtgctcggccccagcgcgcagttcatgctgcggctgatggagaaggaaaacgtctgtctgttcggcagcgggtggt |

| | | |
|---|---|---|
| | | gcatagtggacctgcgcaagctggacgtacccatatatcaacatccttgtttttatcctgtctttttatcagtttttagctagttaaaacataaatagtaaagctaaaaagaggagttctggagtcttgcaataaccagg |
| 75. | Orf virus strain NZ2 DQ184476.1 | ctgaaaatatactgtttgaacgcaaagacgccatgtcgcgacttcaaatactgacctcatttggacaaatcttcgcacccgacgaagctcggctgcgcgagatcgcgcgtgatttgggaatatgcaccataaaacgcgcattcggcgacatgctgtacggctttatagacttcgacccggtgcccctgacccaagtaaacatgctcatgtccaactgctacttcgcggtcaacggcaacctgcttccgtgcacggaggacttccggctcagactcccggcaacggagatctctgcggcctacctgacgagaacgggacggacgatcctgtgcggcaaagacttcaacatagtagcgccgtcggggttcaagccgtccatgcggctgcgcgacctcagtcacgtgtctgcgcttgtagagatcctggaagtctacgacgagtccggggagtaccaattcgtgctcggccccagcgcgcagttcatgctgcggctgatggagaaggagaacgtctgtctgttcggcagcgggtggtgcatagtggacctgcgcaagctggacgtacccatataatcagcatccttgtttttatcctgtctttttatcagtttttagctagttaaaacataaatagtaaagctaaaaagaggagttctggagtcttgcaacaaccagg |
| 76. | Orf virus strain NA17 MG674916.2 | ctgaaaatatactgtttgaacgcaaagacgccatgtcgcgacttcaaatactgacctcatttggacaaatctacgcacccgacgaagctcggctgcgcgagatcgcgcgtgacttgggaatatgcaccataaaacgcgcattcggcgacatgctgtacggctttatagacttcaacccggtgccccttacccaagtaaacatgctcatgtccgactgctacttcgcggtcaacggcaacctgcttccgtgcacggaggacttccggctcagactcccggcgacggagatctccgcggcctacctgacgaaaacgggacggacgatcctgtgcggcaaagacttcaacataatagcgccgtcgggtttcaagccgtccatgcggctgcgcaacctcagtcacgtgtctgcgcttgtagagatcttggagttttacagcgagtccggggagtaccaattcgtgctcggccccagcgcgcagttcatgctgcggctgatggagaaggaaaacgtctgtctgtttggcagcggttggtgcatagtggacctgcgcaagctggacgtaaccatataattgctgctgctatatcgtacctgactctgtgttacaaagacagcagctcaccagactcttcgtccctgttctctaagcaagaacactggagtgagttgccatttccgtctccaacatataattagcatccttgtttttatcttgtctttttatcagtttttatgctagttaaaacataaatagtaaagctaaaaagagtactctggaatcttgcaacaatcagg |
| 77. | Orf virus strain OV-SA00 AY386264.1 | aaatatactgtttgaacgcaaagacgccatgtcgcgacttcaaatactgacctcatttggacaaatctacgcacccgacgaagctcggctgcgcgagatcgcgcgtgacttgggaatatgcaccataaaacgcgcattcggcgacatgctgtacggctttatagacttcaacccggtgcccctgacccaagtaaacatgctcatgtccaactgttacttcgcggtcaacggcaacctgcttccgtgcacggaggacttccggctcagactcccggcgacggagatctctgcggcctacctgacgaaaacgggacggacgatcctgtgcggcaaagacttcaacataatagcgccgttggggttcaagccgtccatgcggctgcgcaacctcagtcacgtgtctgcgcttgtagagatcttggagttttacagcgagtccggggagtaccaatttgtgctcggccccagcgcgcagttcatgctgcgtctgatggagaaggagaacgtctgtctgttcggcagcggttggtgcatagt |

| | | |
|---|---|---|
| | | ggacctgcgcaagctagacgtaactatataattgctgcgctatgtcgtgcccgactctgtgcgacaaagacagaagctcaccaaactcttcgtctctgttctccaagcaagaacactggagtgatttgccatttccgtctccaacatataattagcatccttgtttttatcttgtctttttatcagtttttatgttagttaaaacataaatagtaaagctaaaaagagtactctggaatcttgcaacagtcagg |
| 78. | Orf virus strain OV-IA82 AY386263.1 | atgcatgaaggagatgcacggataactgaaaatatactgtttgaacgcaaagacgccatgtcgcgacttcaaatactgacctcatttggacaaatctacgcacccgacgaagctcggctgcgcgagatcgcgcgtgatttgggaatatgcaccataaaacgcgcattcggcgacatgctgtacggctttatagacttcaacccggtgcccctgacccaagtaaacatgctcatgtccaactgctacttcgcggtcaacggcaacctgcttccgtgcacggaggacttccggctcagactcccggcaacggagatctctgcggcctacctgacgagaacgggacggacgatcctgtgcggcaaagacttcaacatagtggcgccgtcagggttcaagccgtccatgcggctgcgcgaccttagtcacgtgtctgcgcttgtagagatcctggagctctacgacgagtccggggattaccaattcgtgctcggccccagcgcgcagttcatgctgcggctgatggagaaggagaatgtctgtctgttcggcaacggttggtgcatagtggacctgcgcaagctagacgtaaccatataattgctgctgctatgtcgtgcccgactctgtgcgacaaagacagcggctaaccagactcttcgtccctgttctccaagcaaaaaactggagtgagttgccatttccgtctccaaccatataattagcatccttgtttttatcctgtatttttatcagtttttatgctagttaaaacataaatagtaaggctaaaaagaagagttctagaatcttgcaacaaccaag |
| 79. | Orf virus strain GO KP010354.1 | aaatatactgtttgaacgcaaagacgccatgtcgcgacttcaaatactgacctcatttggacaaatctacgcacccgacgaagctcggctgcgcgagatcgcgcgtgacttgggaatatgcaccataaaacgcgcattcggcgacatgctgtacggctttatagacttcaacccggtgccccttacccaagtaaacatgctcatgtctgactgctacttcgcggtcaacggcaacctgcttccgtgcacggaggacttccggctcagactcccggcgacggagatctccgcggcctacctgacgaaaacgggacggacgatcctgtgcggcaaagacttcaacataatagcgccgtcgggtttcaagccgtccatgcggctgcgcaacctcagtcacgtgtctgcgcttgtagaaatcttggagttttacagcgagtccggggagtaccaattcgtgctcggccccagcgcgcagttcatgctgcggctgatggagaaggagaatgtctgtctgttcggcagcggttggtgcatagtggacctgcgcaagctagacgtaaccatataattgctgcgctatgtcgtgcccgactctgtgcgacaaagacagaagctcaccaaactcttcgtctctgttctccaagcaagaacactggagtgatttgccatttccgtctccaacatataattagcatccttgtttttatcttgtctttttatcagtttttatgttagttaaaacataaatagtaaagctaaaaagagtactctggaatcttgcaacagtcagg |
| 80. | Orf virus strain SJ1 KP010356. | ctgaaaatatactgtttgaatgcaaagacgccatgtcgcaacttcaaatactgacctcatttggacaaatctacgcacccgacgaagctcggctgcgcgagatcgcgcgtgatttgggaatatgcaccataaaacgcgcattcggcgacatgctgtacggctttatagacttcaacccggtgccccttacccaagtaaacatgctcatgtccaactgctacttcgcagtcaacggcaacctgcttccgtgcacggaggacttccggctcaga |

| | | |
|---|---|---|
| | 1 | ctcccggcgacggagatctctgcggcctacctgacgaaaacgggacggacgatcctgtgcggcaaagacttcaacatagtggcgccgtcggggttcaagccgtccatgcggctgcgcaacctcagtcacgtgtctgcgcttgtagagatcttggagttttacagcgagtccggggagtaccaattcgtgctcggccccagcgcgcagttcatgctgcggctgatggagaaggagaatgtctgtctgttcggcagcggttggtgcatagtggacctgcgcaagctagacgtaaccatataattgctgcgctatgtcgtgcccgactctgtgcgacaaagacagaagctcaccaaactcttcgtctctgttctccaagtaagaacactggagtgatttgccatttccgtctccaacatataattagcatccttgtttttatcttgtctttttatcagtttttatgctagttaaaacataaatagtaaagctaaaaagagtactctggaatcttgcaacagtcagg |
| 81. | Orf virus strain SY17 MG712417.1 | ctgaaaatatactgtttgaacgcaaagacgccatgtcgcgacttcaaatactgacctcatttggacaaatctacgcacccgacgaagctcggctgcgcgagatcgcgcgtgatttgggaatatgcaccataaaacgcgcattcggcgacatgctgtacggctttatagacttcaacccggtgcccctgacccaagtaaacatgctcatgcccaactgctacttcgctgtcaacggcaacctgcttccgtgcacggaggacttccggcttagactcccggcaacggagatctctgcggcctacctaacgagaacgggacggacgatcctgtgcggcaaagacttcaacatagtggcgccgtcagggttcaagccgtccatgcggctgcgcgacctcagtcacgtgtctgcgcttgtagagatcctggaaatctacaacgagtccggggagtaccaattcgtgctcggccccagcgcgcagttcatgctgcggctgatggagaaggaaaacgtctgtctgttcggcagcgggtggtgcatagtggacctgcgcaagctggacgtaaccatataatcagcatccttgtttttatcctgtctttttatcagtttttagctagttaaaacataaatagtaaagctaaaaagaggagttctggagtcttgtaacaaccagg |
| 82. | Orf virus strainOV-HN3/12 KY053526.1 | ctgaaaatatactgtttgaacgcaaagacgccatgtcgcgacttcaaatactgacctcatttggacaaatctacgcacccgacgaagctcggctgcgcgagatcgcgcgtgatttgggagtatgcaccataaaacgcgcattcggcgacatgctgtacggctttatagacttcgacccggttcccctgacccaagtaaacatgctcatgcccaactgctacttcgcggttaacggcaacctgcttccgtgcacggaggacttcaggctcagactcccggcaacggagatctccgcggcctacctgacgagaacgggacggacgatcctgtgcggcaaagacttcaacatagtggcgccgtcagggttcaagccgtccatgcggctgcgcgaccttagtcacgtgtctgcgcttgtagagatcctggaaatctacaacgagtccggggagtaccaattcgtgctcggccccagcgcgcagttcatgctgcggctgatggagaaggaaaacgtctgtctgttcggcagcgggtggtgcatagtggacctgcgcaagctggacgtacccatataatcaacatccttgtttttatcctgtctttttatcagtttttagctagttaaaacataaatagtaaagctaaaaagaggagttctggagtcttgcaataaccagg |
| 83. | Orf virus strain NP KP010355.1 | ctgaaaatatactgtttgaacgcaaagacgccatgtcgcgacttcaaatactgacctcatttggacaaatctacgcacccgacgaagctcggctgcgcgagatcgcgcgtgacttgggaatatgcaccataaaacgcgcattcggcgacatgctgtacggctttatagacttcaacccggtgccccttacccaagtaaacatgctcatgtccgacttctacttcgcggtcaacggcaacctgcttccgtgcacggaggacttccggctcagactcccggcgacggatatctctgcggcctacctgacgaaaacgggacggacgatcctgtgcggca |

| | | |
|---|---|---|
| | | aagacttcaacatagtggcgccgtcagggttcaagccgtccatgcggctgcgcaacctcagtcacgtgtctgcgcttgtagagatcttggagttttacagcgagtccggggagtaccaatttgtgctcggccccagcgcgcagttcatgctgcggctgatggagaaggagaacgtctgtctgttcggcagcggttggtgcatagtggacctgcgcaagctagacgtaaccatataattgctgcgctatgtcgtacctgactctgtgtacaaagacagcagctcaccagactcttcgtccctgttctctaagcaagaacactggagtgagttgccatttccgtctccgacatataattagcatccttgttttatcctgtctttttatcagtttttatgctagttaaaacataaatagtaaagctaaaaagagtactctggaatcttgcaacaatcagg |
| 84. | Orf virus strain B029 KF837136.1 | aaatatactgtttgaacgcaaagacaccatgtcgcgacttcaaatactgacctcatttggacaaatctacgcacccacgaagctcggctgcgcgagatcgcgcgtgatttgggaatatgcaccataaaacgcgcattcggcgacatgctgtacggctttatagacttcaacccggtgcccctgacccaagtaaacatgctcatgtccaactgctacttcgcggtcaacggcaacctgcttccgtgcacggaggacttccggctcagactcccggcaacggagatctctgcagcctacctgacgaaaacgggacggacgatcctgtgcggcaaagacttcaacatagtggcgccgtcggggttcaagccgtccatgcggctgcgcgacctcagtcacgtgtctgcgcttgtagagatcctggagctctacgacgagtccggggagtaccaattcgtgctcggccccagcgcgcagttcatgctgcggctgatggagaaggagaatgtctgtctgttcggcaacgggtggtgcatagtggacctgcgcaagctagacgtaaccatataattgctgctatgtcgtacccgactctgtgctacaaagacagcagctcaccagactcttcgtccctgttctccaagcaagaacactggagtgagttgccatttccgtctccaaccatataattagcatccttgttttatcctgtatttttatcagtttttatgctagttaaaacataaatagtaaggctaaaaagaagagttctagaatcttgcaacaaccaag |
| 85. | Amino acid sequence of the ORFV111 gene of the POV-601-1A1 strain (mutant) （154aa） | MSRLQILTSFGQIYAPDEARLREIARDLGICTIKRAFGDMLYGFIDFNPVPLTQVNMLMPNCYFAVNGNLLPCTEDFRLRLPATEISAAYLTKTGRTILCGRDFNIVAPSGFKPSMRLRNLSHVSALVEILELYDESGEYQFVLGPSAQLMLRR |
| 86. | Amino acid sequence of the ORFV112 | GVSIPLSYIGLRFNPSLTDGYLYVNVSSRAPWDQQTLDLSANDGWGIKQVLEKEILAIQIGCDNQKFPEEPTTTQPPSPVTTTLSSTTLDPNDENTDTTPTTTGDSVDGKRNPDDFDFSLIVDPRCVTSVNLHF |

| | | |
|---|---|---|
| | gene of the POV-601-1A1 strain (mutant) （183aa） | EIKDACMDHKESSPLSLKGEYGDGELVRKEIKNVGKDHNMCSLNLSPGH |

**Claims**

1. A mutant orf virus, wherein the functionally expressed product of the ORFV112 gene and/or the ORFV111 gene is absent.

2. The virus of claim 1, wherein the absence of the functionally expressed product of the ORFV112 gene is a result of the complete or partial deletion of the ORFV112 gene.

3. The virus of claim 1, wherein the absence of the functionally expressed product of the ORFV111 gene is a result of the complete or partial deletion of the ORFV111 gene.

4. The virus of any one of claims 1-3, wherein the expressed product is protein and/or nucleic acid.

5. A mutant orf virus, **characterized by** complete or partial deletion of the ORFV112 gene, and/or complete or partial deletion of the ORFV111 gene.

6. A method for modifying an orf virus comprising reducing or suppressing the expression and/or activity of the ORFV112 gene product, and/or reducing or suppressing the expression and/or activity of the ORFV111 gene product.

7. The method of claim 6, wherein the ORFV112 gene is completely or partially deleted.

8. The method of claim 6, wherein the ORFV111 gene is completely or partially deleted.

9. The method of any one of claims 6-8, wherein the expression is transcription and/or translation.

10. A method for modifying an orf virus comprising deleting the ORFV112 gene completely or partially, and/or deleting the ORFV111 gene completely or partially.

11. A virus produced by the method of any one of claims 6-10.

12. The virus of any one of claims 1-5 and 11, wherein the ORFV002 gene (NF-κB inhibitor), the ORFV005 gene (hypothetical protein), and the ORFV007 gene (dUTPase) are completely deleted.

13. The virus of any one of claims 1-5, 11, and 12, wherein the virus has replicating and oncolytic capabilities.

14. An orf virus deposited at China Center for Type Culture Collection (CCTCC) with the accession number V20202.

15. A genome of the virus of any one of claims 1-5 and 11-14.

16. A composition comprising the virus of any one of claims 1-5 and 11-14, and a pharmaceutically acceptable carrier.

17. The composition of claim 16, wherein the composition is in a form of powder, solution, transdermal patch, ointment, or suppository.

18. The composition of claim 16, wherein the composition is administered through intravenous, intratumoral, intramuscular, subcutaneous, rectal, vaginal, or intraperitoneal routes.

**19.** A use for preparing a drug for cancer treatment with the virus of any one of claims 1-5 and 11-14.

**20.** The use of claim 19, wherein the cancer is a solid tumor.

**21.** The use of claim 20, wherein the said solid tumor is cervical cancer, bladder cancer, liver cancer, ovarian cancer, melanoma, colorectal cancer, lung cancer, breast cancer, stomach cancer, uterine cancer, head and neck cancer, thyroid cancer, esophagus cancer, prostate cancer, pancreatic cancer, sarcoma, or a brain tumor.

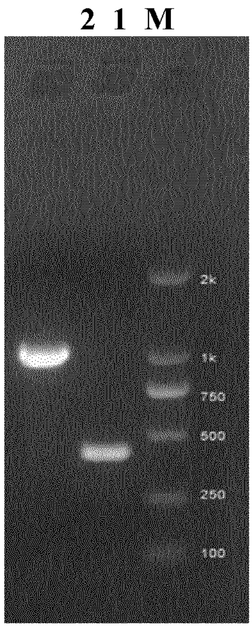


Figure 1

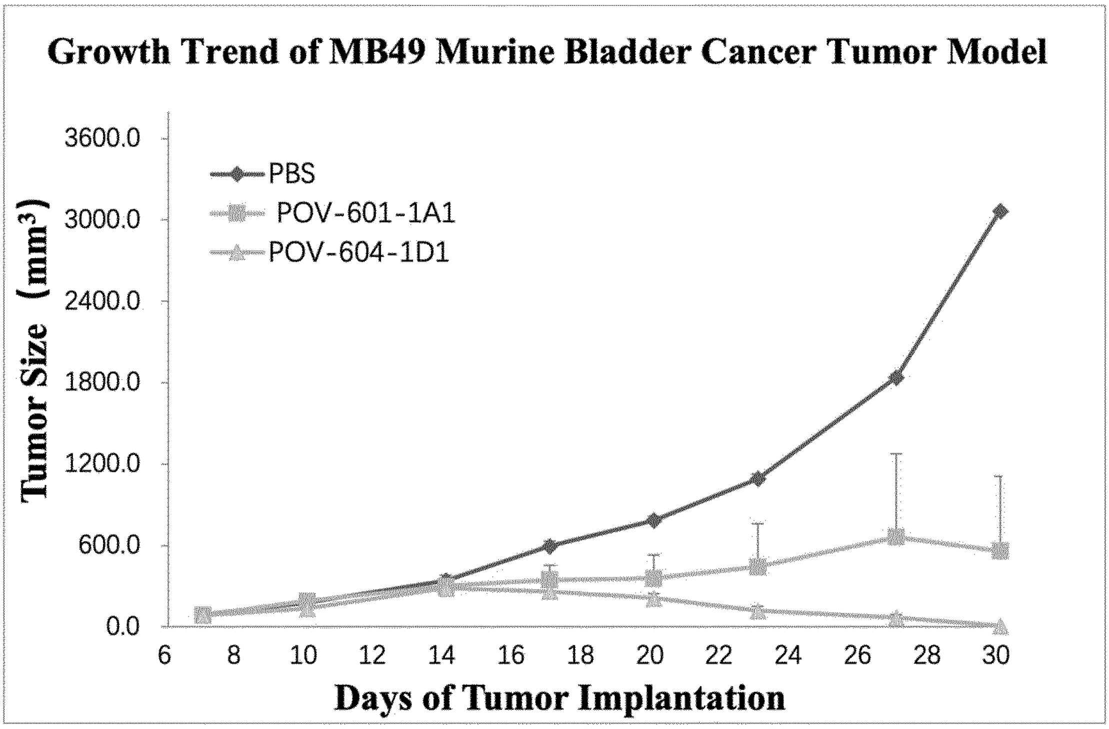


Figure 2

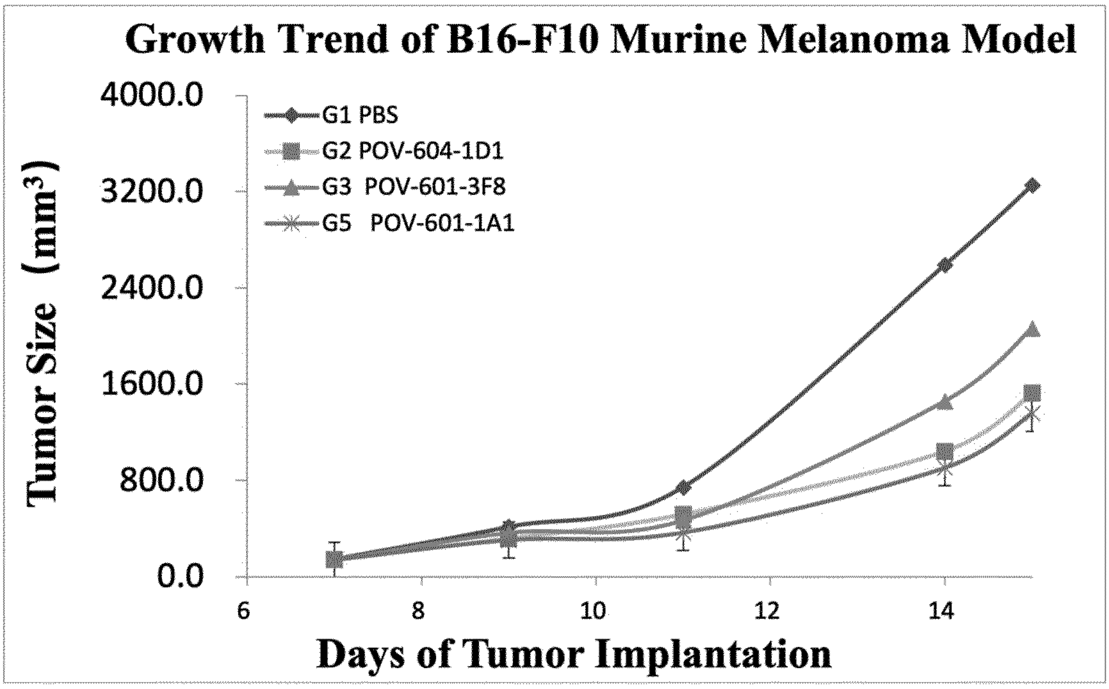


Figure 3

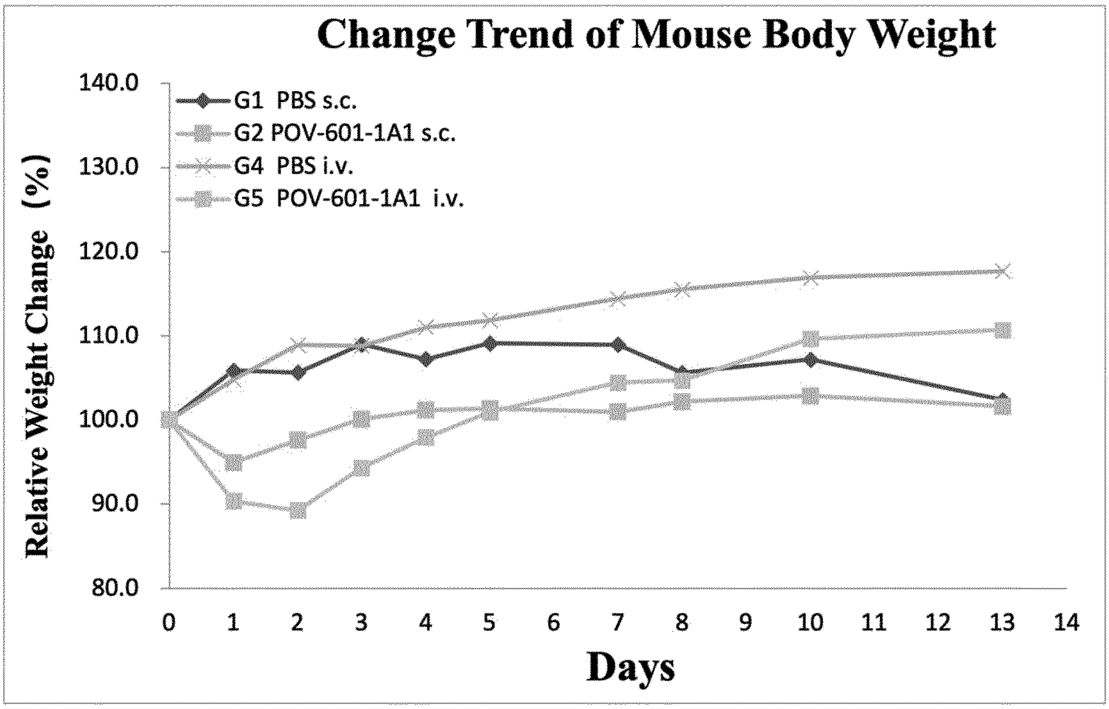


Figure 4

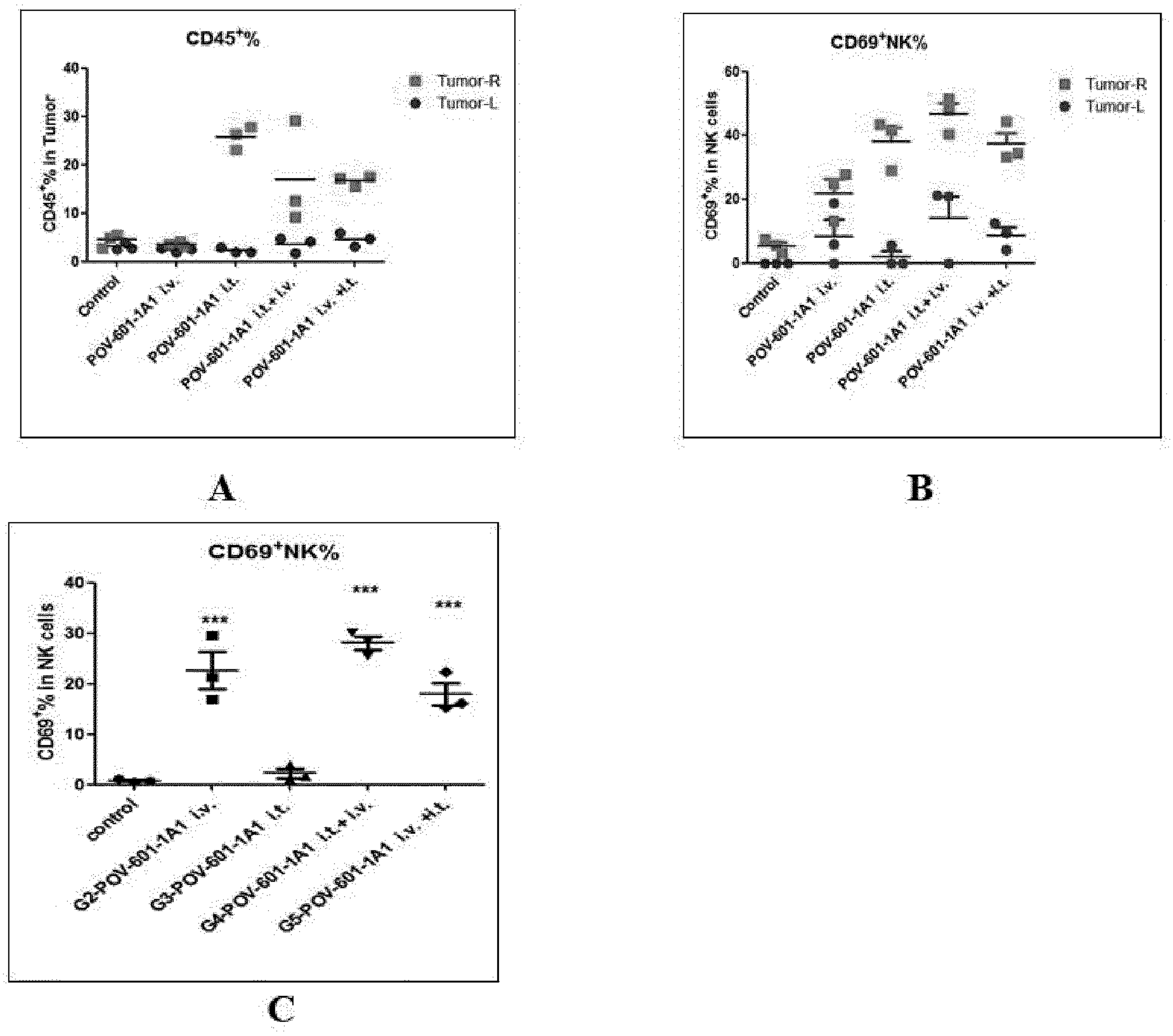
CD45+%
CD45+% in Tumor
Tumor-R
Tumor-L
Control
POV-601-1A1 i.v.
POV-601-1A1 i.t.
POV-601-1A1 i.t.+ i.v.
POV-601-1A1 i.v. +i.t.
A
CD69+NK%
CD69+% in NK cells
Tumor-R
Tumor-L
B
CD69+NK%
CD69+% in NK cells
control
G2-POV-601-1A1 i.v.
G3-POV-601-1A1 i.t.
G4-POV-601-1A1 i.t.+ i.v.
G5-POV-601-1A1 i.v. +i.t.
C

Figure 5

```
POV-601-1A1    0>~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~>0
POV-601-3F8    1>ttgctgctgctatgtcgtgcccgactctgtgcgacaaagaagcagcttaccagacgactcttcgttcatgttctctaagcaagaacactggagtgagttg>100

POV-601-1A1    0>~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~>0
POV-601-3F8  101>ccatttccgtctccaaccatataattagcatccttgtttttatcctgtatttttatcagtttttagctagttaaaacataaatagtaaagctaaaaagag>200

POV-601-1A1    0>~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~>0
POV-601-3F8  201>gagttctggaatcttgcaacgacaggatgaaggcggtgttgttgctagcgctactgggagcgttcaccaacgcagcgcctttgttaagcaaccagcgtct>300

POV-601-1A1    0>~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~>0
POV-601-3F8  301>tggcagtgcagaagaagaaaaattctgctcgacgcatcagggcgaagtgcacgccaggttctggcttcagatgcgcgtgggtgtacgacacagtccgctc>400

POV-601-1A1    0>~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~>0
POV-601-3F8  401>tacactcccagcaacatgtgcatgatggacatagaagactctacggacacagaagactccacgatggaaaaagaatacacgtctacggcaacgggtgatg>500

POV-601-1A1    1>~~~~~~~~~~~~~~~~~~~~~~~~~~~~~~~~~~~~~~ggcgtgagcataccgctaagttacataggccttagatttaacccgtcgcttacagatggcta>62
POV-601-3F8  501>cggacggattgaacgtgtccgtagcactaattggagaaggcgtgagcataccgctaagttacataggccttagatttaacccgtcgcttacagatggcta>600

POV-601-1A1   63>cctgtacgtcaacgtctcgtcacgggctccttgggatcaacagactctggacctatccgcgaacgacggctggggtatcaaacaggttctagaaaaagag>162
POV-601-3F8  601>cctgtacgtcaacgtctcgtcacgggctccttgggatcaacagactctggacctatccgcgaacgacggctggggtatcaaacaggttctagaaaaagag>700

POV-601-1A1  163>atactggccatccagatagggtgcgacaaccaaaaatttcccgaagaacccacaactacccaaccccccctcacctgtcacaacaacgctttcctcaacaa>262
POV-601-3F8  701>atactggccatccagatagggtgcgacaaccaaaaatttcccgaagaacccacaactacccaaccccccctcacctgtcacaacaacgctttcctcaacaa>800

POV-601-1A1  263>ctctagatccgaatgacgaaaacacagacactacgccgacaacccaccggcgacagtgtagacggaaagcgcaatccagatgactttgacttctcgctgat>362
POV-601-3F8  801>ctctagatccgaatgacgaaaacacagacactacgccgacaacccaccggcgacagtgtagacggaaagcgcaatccagatgactttgacttctcgctgat>900

POV-601-1A1  363>cgtggacccccgatgcgtgacctctgtaaacctgcactttgagattaaggacgcgtgcatggaccacaaagaatcgtcgccgttgtcgctgaagggggaa>462
POV-601-3F8  901>cgtggacccccgatgcgtgacctctgtaaacctgcactttgagattaaggacgcgtgcatggaccacaaagaatcgtcgccgttgtcgctgaagggggaa>1000

POV-601-1A1  463>tatggagacggcgaactagtaagaaaagaaatcaaaaacgtgggaaaggatcacaatatgtgcagtcttaacctcagccctggccattgagctgtttttta>562
POV-601-3F8 1001>tatggagacggcgaactagtaagaaaagaaatcaaaaacgtgggaaaggatcacaatatgtgcagtcttaacctcagccctggccattgagctgtttttta>1100

POV-601-1A1  563>ttcggcaatataataggtgattattgaacattaaacaaaacttatcccacaacgccgcaaca>624
POV-601-3F8 1101>ttcggcaatataataggtgattattgaacattaaacaaaacttatcccacaacgccgcaaca>1162
```

Figure 6

```
3F8 .seq        ..MKAVLLLALLGAFTNAAPLLSNQRLGSAEEEKFCSTHQGEVHARFWLQMRVGVRHSPLYTPSNMCMMDIEDSTD....   74
B029.seq        ..MKAVLLLALLGAFTNAAPLLSNQRLGSAEEEKFCSTHHDKVYARFWLQMRVGVRHSSLYAPSNMCMMDIEDSTVDIEG   78
GO.seq          ..MKVVLLLVLLGALTNAAPVGNQRIDSE.EKANFCSTHQNEVYARFRLQMRVGVRHSPLYTPSNMCMMNIEDSMMDNE.   76
NA11.seq        ..MKAVLLLALLGAFTNAAPLLESQRSNSEEKANFCSTHNNEVYARFRLQMRVGVRHSPFYTPSNMCMMDIEDSVED...   75
NA17.seq        ..MKVVVLLALLGALTNAAPVGNQRLNSK.EKEDFCSTHQNEVYARFRLQMRVGVRHSPLYVPSNMCMMDIEDSVDDIE.   76
NP.seq          ..MKVVVLLALLGALTNAAPVGNQRLNSK.EKEDFCSTHPDEVYARFRLQMRVGVRHSPLYVPSNMCMMDIEDSVDDIE.   76
NZ2.seq         ..MKAVLLLALLGAFTNAAPLLESQRSNSEEKANFCSTHNDEVYARFRLQMRVGVRHSPLYTPSNMCMLDIEDSVED...   75
OV-HN3_12.seq   ..MKAVLLLALLGAFTNAAPLLESQRSNSEEKANFCSTHNNEVYARFRLQMRVGVRHSPFYTPSNMCMMDIEDSVED...   75
OV-IA82.seq     MKAAAVLLLALLGAFTNAAPVSNQRLGSE.EKEKFCSTHHDEVYARFRLQMRVGVRHSPLYVPSNMCMMDIEDSTDD...   76
OV-SA00.seq     ..MKVVLLLVLLGAFTNAAPLVGNQRLDSEEKANFCSTHQNEVYARFRLQMRVGVRHSPLYTPSNMCMMDIEDSIMD...   75
SJ1.seq         ..MKVVVLLALLGALTNAAPVGNQRLNSK.EKEDFCLTHPDEVYARFRLHMRVGVRHSPLYTPSNMCMMNIEDSMMDNE.   76
SY17.seq        ..MKAVLLLALLGAFTNAAPVGNQRLDSG.EKEKFCSTHQDEVYARFRLQMRVGVRHSPFYTPSNMCMMDIEDSVDDIE.   76
YX.seq          ..MKVVLLLVLLGALTNAAPVGNQRIDSE.EKANFCSTHQNEVYARFRLQMRVGVRHSPLYVPSNMCMMDIEDSVMDNE.   76

3F8 .seq       ...TEDSTMEKEYTSTATGDADGLNVSVALIGEGVSIPLSYIGLRFNPSLTDGYLYVNVSSRAPWDQQTLDLSANDGWGI   151
B029.seq       STETEDSTVEKEYTSAATGDANGVNVSVALMGEGVSIPLSYIGLGFNPLLKDGYLYVNVSSRAPWDQQTLDLSANDGWGI   158
GO.seq         ....YTST........ATGDADGVNVSVALMGKGVSIPLSYIGLGFNPLLADGYLYVNVSSRAPWVQQTPDLSANGGWGI   144
NA11.seq       ....IEESTEKEYASTATGEAAGVNVSVALVGEGVSIPFSYIGLGFNPSLEDSYLYVNVSSRAPWVKQTSDLSANGGWGI   151
NA17.seq       ....EDSIIVKEFTSTATGEAAGVNVSVALVGEGVSIPFSYIGLGFNPSLEDSYLYVNVSSRAPWVQQTPDLSANDSWGI   152
NP.seq         ....EDSIIVKEFTSTATGEAAGVNVSVALVGEGVSIPFSYIGLGFNPSLEDSYLYVNVSSRAPWVQQTPDLSANDSWGI   152
NZ2.seq        ....IEESTEKEYASTATGEAAGVNVSVALVGEGVSIPFSYIGLGFNPSLEDSYLYVNVSSRAPWVKQTSDLSANGGWGI   151
OV-HN3_12.seq  ....IEESTEKEYASTATGEAAGVNVSVALVGEGVSIPFSYIGLGFNPSLEDSYLYVNVSSRAPWVKQTSDLSANGGWGI   151
OV-IA82.seq    ....IEESTEKEYTSTATGEAAGVNVSVALVGEGVKIPFSYIGLGFNPS.TDGYLYVNVSSRAPWVQQTPDLSANSGWGI   151
OV-SA00.seq    ....IENSTEKEYTSAATGDANGVNVSVALIGEGVSIPLSYIGLGFNPSLADGYLYVNVSSRAPWVQQTLDLSANGGWGI   151
SJ1.seq        ....YTST........ATGDADGVNVSVALMGKGVSIPLSYIGLGFNPLLADGYLYVNVSSRAPWVQQTPDLSANGGWGI   144
SY17.seq       ....EDSIIVKEFTSTATGEADGVNVSVALVGEDVKIPLSYIGLGFNPS.TDGYLYVNVSSRAPWVQQTLDLSANDSWGI   151
YX.seq         ....YMSA........ATGDADGVNVSVALIGEGVSIPLSYIGLGFNPSLADGYLYVNVSSRAPWVQQTQDLSANGSWGI   144

3F8.seq        KQVLEKEILAIQIGCDNQKFPEEPTTTQPPSPVTTTLSSTT.LDPNDENTDTTP.TTTGDSVDGKRNPDDFDFSLIVDPR   229
B029.seq       KQVLEKEILAIQIGCDNQKFPEEPTTTQPPSPVTTTLSPTTTLNPNNENTDTTP.TPTGASVDGKRNPDDIDFSLIVDPR   237
GO.seq         KQVLEEEILAIQIGCDNQKFLEEPTTTQPPSLVTTTLSSTT.LGLTDENTDTTP.TTTSASVDRKRNLDDIDFSLLVDTR   222
NA11.seq       KQVLEKELLAIQIGCDNQKFPEEPTTTPPSPV.TTTLSSTT.PDLNEENTNTTP.TTTSASVDRRRNLDDIDFSLLVDPR   228
NA17.seq       KQVLEKELLAIQIGCDNQKFPEEPTTTQPPSLVTTTLSSTT.LDLNDENTDTTPPTTTSASVNKKRNPDDFDFSLLVDPR   231
NP.seq         KQVLEKELLAIQIGCDNQKFPEEPTTTQPPSLVTTTLSSTT.LDLNDENTDTTPPTTTSASVNKKRNPDDFDFSLLVDPR   231
NZ2.seq        KQVLEKELLAIQIGCDNQKFPEEPTTTPPSPV.TTTLSSTT.PDLNEENTENTP.TTTGASVDRKRNPADIDFSLLVDPR   228
OV-HN3_12.seq  KQVLEKELLAIQIGCDNQKFPEEPTTTPPSPV.TTTLSSTT.PDLNEENTDTTP.TTTSASVDRRRNLDDIDFSLLVDPR   228
OV-IA82.seq    KQVLEKELLAIQIGCDNQKFPEEPTTTPSLVT..TTLSPTTTLNPNNENTDTTP.TPTGASVDGKRNPDDIDFSLIVDPR   228
OV-SA00.seq    NQILEKELLAIQIGCDNQKFPEEPTTTQPPSPVTTTLSSTT.LDLTDENTDTTPLTTTSASVNRKRNPDDFDFSLLVDPR   230
SJ1.seq        KQVLEEEILAIQIGCDNQKFLEEPTTPQPPSLVTTTLSSTT.LGLTDENTDTTP.TTTSASVDRKRNLDDIDFSLLVDTR   222
SY17.seq       KQVLEKELLAIQIGCDNQKFPEEPTTTQPPSLVTTTLSSTT.PDLNDENTDTTP.TTTGASVDRKRNPADFSFSLLVDPR   229
YX.seq         KQVLEQEMLAIQIGCDNQKFPEEPTTTQPPSPVTTTLSSTT.LDSNDENADTTPPTTTSASVNRKRNSDDFDFSLLVDPR   223

3F8 .seq       CVTSVNLHFEIKDACMDHKESSPLSLKGEYGDGELVRKEIKNVGKDHNMCSLNLSPGH      287
B029.seq       CVTSVNLHFEIKDACMDYKQESPLSLKGKYGDGELVKEEIKDVGKNHNMCSLNLSPGH      295
GO.seq         CVTSVNLHFEIKDACMDYKKESPLMMKGGYGDGELVRKEIKYVGTNHTMCSLNLSPGY      280
NA11.seq       CVTSVDLHVELRDACMDYKQESPLSLKGKYGDGELVKKEIKDVGKNYNMCSLNLNPGN      286
NA17.seq       CVTSVDLHVELRDACIDYKETSQLSLKGEYGDGELIKKEIKDVGKDHNMCSLNLNPGN      289
NP.seq         CVTSVDLHVELRDACIDYKETSQLSLKGEYGDGELIKKEIKDVGKDHNMCSLNLNPGN      289
NZ2.seq        CVTSVDLHVELRDACIDYKQESPLSLKGKYGDGELVKKEIKDVGKNHNMCSLNLNPGN      286
OV-HN3_12.seq  CVTSVDLHVELRDACMDYKQESPLSLKGKYGDGELVKKEIKDVGKNYNMCSLNLNPGN      286
OV-IA82.seq    CVTSVNLHFELKDACMDYKKESPLSLKGKYGDSELVKQEIKDVGKNHNMCSLNLSPGH      286
OV-SA00.seq    CVTSVDLHVELRDACIDYKEASQLSLKGKYGDGELVKKEIKDVGKDHNMCSLNLSPGH      288
SJ1.seq        CVTSVNLHFEIKDACMDYKKESPLMMKGGYGDGELVRKEIKYVGTNHTMCSLNLSPGY      280
SY17.seq       CVTSVDLHVELRDACMEYKETSPLSLKGEYGDGELVKKEIKDVGKNHNMCSLNLNPGN      287
YX.seq         CVTSVDLHVELRDACIDYKEASLLSLKGKYGDGELVKKEIKDVGKDHNMCSLNLSPGH      281
```

Figure 7

```
3F8.seq        TTGCTGCTGCTATGTCGTGCCCGACTCTGTGCGACAAAGA.AGCAGCTTACCAGACGACTCTTCGTTCATGTTCTCTAAGCAAGAACACTGGAGTGAGTT    99
B029.seq       ...TTGCTGCTATGTCGTACCCGACTCTGTGCTACAAAGACAGCAGCTCACCAGAC...TCTTCGTCCCTGTTCTCCAAGCAAGAACACTGGAGTGAGTT    94
GO.seq         TTGCTGC.GCTATGTCGTGCCCGACTCTGTGCGACAAAGACAGAAGCTCACCAAAC...TCTTCGTCTCTGTTCTCCAAGCAAGAACACTGGAGTGATTT    96
NA11.seq       ....................................................................................................     0
NA17.seq       TTGCTGCTGCTATATCGTACCTGACTCTGTGTTACAAAGACAGCAGCTCACCAGAC...TCTTCGTCCCTGTTCTCTAAGCAAGAACACTGGAGTGAGTT    97
NP.seq         TTGCTGC.GCTATGTCGTACCTGACTCTGTGT.ACAAAGACAGCAGCTCACCAGAC...TCTTCGTCCCTGTTCTCTAAGCAAGAACACTGGAGTGAGTT    95
NZ2.seq        ....................................................................................................     0
OV-HN3_12.seq  ....................................................................................................     0
OV-IA82.seq    TTGCTGCTGCTATGTCGTGCCCGACTCTGTGCGACAAAGACAGCGGCTAACCAGAC...TCTTCGTCCCTGTTCTCCAAGCAAAAA.ACTGGAGTGAGTT    96
OV-SA00.seq    TTGCTGC.GCTATGTCGTGCCCGACTCTGTGCGACAAAGACAGAAGCTCACCAAAC...TCTTCGTCTCTGTTCTCCAAGCAAGAACACTGGAGTGATTT    96
SJ1.seq        TTGCTGC.GCTATGTCGTGCCCGACTCTGTGCGACAAAGACAGAAGCTCACCAAAC...TCTTCGTCTCTGTTCTCCAAGTAAGAACACTGGAGTGATTT    96
SY17.seq       ....................................................................................................     0
YX.seq         TTGCTGCTGCTATATCGTACCTGACTCTGTGTTTTAAAGACAGCAGCTCACCGA.C...TCTTCGTCCCTGTTCTCCAAGCAAGAACACTGGAGTGATTT    96

3F8.seq        GCCATTTCCGTCTCCAACCATATAATTAGCATCCTTGTTTTTATCCTGTATTTTTATCAGTTTTTA.GCTAGTTAAAACATAAATAGTAAAGCTAAAAAG   198
B029.seq       GCCATTTCCGTCTCCAACCATATAATTAGCATCCTTGTTTTTATCCTGTATTTTTATCAGTTTTTATGCTAGTTAAAACATAAATAGTAAGGCTAAAAAG   194
GO.seq         GCCATTTCCGTCTCCAAC.ATATAATTAGCATCCTTGTTTTTATCTTGTCTTTTTATCAGTTTTTATGTTAGTTAAAACATAAATAGTAAAGCTAAAAAG   195
NA11.seq       .........................TCAACATCCTTGTTTTTATCCTGTCTTTTTATCAGTTTTTTAGCTAGTTAAAACATAAATAGTAAAGCTAAAAAG    75
NA17.seq       GCCATTTCCGTCTCCAAC.ATATAATTAGCATCCTTGTTTTTATCTTGTCTTTTTATCAGTTTTTATGCTAGTTAAAACATAAATAGTAAAGCTAAAAAG   196
NP.seq         GCCATTTCCGTCTCCGAC.ATATAATTAGCATCCTTGTTTTTATCCTGTCTTTTTATCAGTTTTTATGCTAGTTAAAACATAAATAGTAAAGCTAAAAAG   194
NZ2.seq        .........................TCAGCATCCTTGTTTTTATCCTGTCTTTTTATCAGTTTTTTAGCTAGTTAAAACATAAATAGTAAAGCTAAAAAG    75
OV-HN3_12.seq  .........................TCAACATCCTTGTTTTTATCCTGTCTTTTTATCAGTTTTT.AGCTAGTTAAAACATAAATAGTAAAGCTAAAAAG    74
OV-IA82.seq    GCCATTTCCGTCTCCAACCATATAATTAGCATCCTTGTTTTTATCCTGTATTTTTATCAGTTTTTATGCTAGTTAAAACATAAATAGTAAGGCTAAAAAG   196
OV-SA00.seq    GCCATTTCCGTCTCCAAC.ATATAATTAGCATCCTTGTTTTTATCTTGTCTTTTTATCAGTTTTTATGTTAGTTAAAACATAAATAGTAAAGCTAAAAAG   195
SJ1.seq        GCCATTTCCGTCTCCAAC.ATATAATTAGCATCCTTGTTTTTATCTTGTCTTTTTATCAGTTTTTATGCTAGTTAAAACATAAATAGTAAAGCTAAAAAG   195
SY17.seq       .........................TCAGCATCCTTGTTTTTATCCTGTCTTTTTATCAGTTTTTTAGCTAGTTAAAACATAAATAGTAAAGCTAAAAAG    75
YX.seq         GCCATTTCCGTCTCCAAC.ATATAATTAGCATCCTTGTTTTTATCTTGTCTTTTTATCAGTTTTTATGTTAGTTAAAACATAAATAGTAAAGCTAAAAAG   195

3F8.seq        AGGAGTTCTGGAATCTTGCAACGAC.AGGATGAAGGCGG......TGTTGTTGCTAGCGCTACTGGGAGCGTTCACCAACGCAGCGCCTTTGTTAAGCAA   291
B029.seq       AAGAGTTCTAGAATCTTGCAACAACCAAGATGAAGGCGG......TGTTGTTGCTAGCGCTACTGGGAGCGTTCACCAACGCAGCGCCTTTGTTAAGCAA   288
GO.seq         AGTA.CTCTGGAATCTTGCAACAGTCAGGATGAAGGTGG......TGTTGTTGCTAGTGCTACTGGGGGCGTTAACCAACGCAGCGCCC...GTCGGCAA   285
NA11.seq       AGGAGTTCTGGAGTCTTGCAATAACCAGGATGAAGGCGG......TGTTGTTGCTGGCGTTACTGGGAGCGTTCACCAACGCAGCGCCTTTGTTAGAAAG   169
NA17.seq       AGTA.CTCTGGAATCTTGCAACAATCAGGATGAAGGTGG......TGGTGTTGCTGGCGCTACTGGGAGCGTTAACCAACGCAGCGCCC...GTCGGCAA   286
NP.seq         AGTA.CTCTGGAATCTTGCAACAATCAGGATGAAGGTGG......TGGTGTTGCTGGCGCTACTGGGAGCGTTAACCAACGCAGCGCCC...GTCGGCAA   284
NZ2.seq        AGGAGTTCTGGAGTCTTGCAACAACCAGGATGAAGGCGG......TGTTGTTGCTGGCGTTACTGGGAGCGTTCACCAACGCAGCGCCTTTGTTAGAAAG   169
OV-HN3_12.seq  AGGAGTTCTGGAGTCTTGCAATAACCAGGATGAAGGCGG......TGTTGTTGCTGGCGTTACTGGGAGCGTTCACCAACGCAGCGCCTTTGTTAGAAAG   168
OV-IA82.seq    AAGAGTTCTAGAATCTTGCAACAACCAAGATGAAGGCGGCGGCGGTGTTGTTGCTAGCGCTACTGGGAGCGTTCACCAACGCAGCGCCCGTC...AGCAA   293
OV-SA00.seq    AGTA.CTCTGGAATCTTGCAACAGTCAGGATGAAGGTGG......TGTTGTTGCTAGTGCTACTGGGAGCGTTTACCAACGCAGCGCCTTTGGTCGGCAA   288
SJ1.seq        AGTA.CTCTGGAATCTTGCAACAGTCAGGATGAAGGTGG......TGGTGTTGCTGGCGCTACTGGGAGCGTTAACCAACGCAGCGCCC...GTTGGCAA   285
SY17.seq       AGGAGTTCTGGAGTCTTGTAACAACCAGGATGAAGGCGG......TGTTGTTGCTGGCGTTACTGGGAGCGTTCACTAACGCAGCGCCC...GTCGGCAA   166
YX.seq         AGTA.CTCTGGAATCTTGCAACAGTCAGGATGAAGGTGG......TGTTGTTGCTAGTGCTACTGGGGGCGTTAACCAACGCAGCGCCC...GTCGGCAA   285
```

```
3F8.seq          CCAGCGTCTTGGCAGTGCAGAAGAAGAAAAATTCTGCTCGACGCATCAGGGCGAAGTGCACGCCAGGTTCTGGCTTCAGATGCGCGTGGGTGTACGACAC   391
B029.seq         CCAGCGTCTTGGCAGTGCAGAGGAAGAAAAATTCTGCTCGACGCATCATGACAAAGTGTACGCCAGGTTCTGGCTTCAGATGCGTGTGGGTGTACGACAC   388
GO.seq           CCAGCGTATTGACAGTGAGGAGAAAGCAAATTTCTGCTCGACGCATCAAAATGAAGTGTACGCCAGGTTCCGGCTTCAGATGCGCGTGGGTGTACGGCAC   385
NA11.seq         CCAGCGTTCTAACAGTGAGGAGAAAGCAAATTTCTGCTCGACGCATAATAATGAAGTGTACGCCAGGTTCAGGCTTCAGATGCGCGTGGGTGTACGACAC   269
NA17.seq         CCAGCGTCTTAACAGTAAAGAGAAAGAAGATTTCTGCTCGACGCATCAAAATGAAGTGTACGCCAGGTTCCGGCTTCAGATGCGCGTGGGTGTACGACAC   386
NP.seq           CCAGCGTCTTAACAGTAAAGAGAAAGAAGATTTCTGCTCGACGCATCCGGATGAAGTGTACGCCAGGTTCCGGCTTCAGATGCGCGTGGGTGTACGACAC   384
NZ2.seq          CCAGCGTTCTAACAGTGAGGAAAAAGCAAATTTCTGCTCGACGCATAATGATGAAGTGTACGCCAGGTTCAGGCTTCAGATGCGCGTGGGTGTACGACAC   269
OV-HN3_12.seq    CCAGCGTTCTAACAGTGAGGAGAAAGCAAATTTCTGCTCGACGCATAATAATGAAGTGTACGCCAGGTTCAGGCTTCAGATGCGCGTGGGTGTACGACAC   268
OV-IA82.seq      CCAGCGTCTTGGCAGTGAGGAGAAAGAAAAATTCTGCTCGACTCATCATGACGAAGTGTACGCCAGGTTCCGGCTTCAGATGCGCGTGGGTGTACGACAC   393
OV-SA00.seq      CCAGCGTCTTGACAGTGAGGAGAAAGCAAATTTCTGCTCGACACATCAAAATGAAGTGTACGCCAGGTTCCGGCTTCAGATGCGCGTGGGTGTACGACAC   388
SJ1.seq          CCAGCGTCTTAACAGTAAAGAGAAAGAAGATTTCTGCTTGACGCATCCGGATGAAGTGTATGCCAGGTTCCGGCTTCATATGCGCGTGGGTGTACGACAC   385
SY17.seq         CCAGCGTCTCGATAGTGGGGAGAAAGAAAAATTCTGCTCGACGCATCAGGATGAAGTGTACGCTAGGTTCAGGCTTCAGATGCGCGTGGGTGTACGACAC   266
YX.seq           CCAGCGTATTGACAGTGAGGAGAAAGCAAATTTCTGCTCGACGCATCAAAATGAAGTGTACGCCAGGTTCCGGCTTCAGATGCGCGTGGGTGTACGACAC   385

3F8.seq          AGTCCGCTCTACACTCCCAGCAACATGTGCATGATGGACATAGAAGACTCTACGG...ACACAGAAGACTCCACGATGG..................AAA   470
B029.seq         AGTTCGCTCTACGCTCCCAGCAACATGTGCATGATGGACATAGAAGACTCTACGGTGGACATAGAAGGCTCCACGGAAACAGAAGACTCCACGGTGGAAA   488
GO.seq           AGTCCGCTCTACACTCCCAGCAACATGTGCATGATGAACATAGAAGACTCCATGATGGATA.......................................   446
NA11.seq         AGTCCTTTCTACACTCCCAGCAACATGTGCATGATGGACATAGAAGACTCCGTTGAGGACATAGAAGAG......TCCACAGA...............GA   348
NA17.seq         AGTCCGCTCTACGTTCCCAGCAACATGTGCATGATGGACATAGAAGACTCTGTTGATGACATAGAAGAAGACTCCATTATAGT...............AA   471
NP.seq           AGTCCGCTCTACGTTCCCAGCAACATGTGCATGATGGACATAGAAGACTCTGTTGATGACATAGAAGAAGACTCCATTATAGT...............AA   469
NZ2.seq          AGTCCGCTCTACACTCCCAGCAACATGTGCATGCTGGACATAGAAGACTCCGTTGAGGACATAGAAGAG......TCCACAGA...............AA   348
OV-HN3_12.seq    AGTCCTTTCTACACTCCCAGCAACATGTGCATGATGGACATAGAAGACTCCGTTGAGGACATAGAAGAG......TCCACAGA...............GA   347
OV-IA82.seq      AGTCCGCTCTACGTTCCCAGCAACATGTGCATGATGGACATAGAAGACTCTACGGATGACATAGAAGAGTCCACGGAGA.....................   472
OV-SA00.seq      AGTCCGCTCTACACTCCCAGCAACATGTGCATGATGGACATAGAAGACTCCATTATGGACATAGAAAA......CTCCACAGA...............AA   467
SJ1.seq          AGTCCGCTTTACACTCCCAGCAACATGTGCATGATGAACATAGAAGACTCCATGATGGATA.......................................   446
SY17.seq         AGTCCTTTCTACACTCCCAGCAACATGTGCATGATGGACATAGAAGACTCCGTTGATGACATAGAAGAAGACTCCATTATAGT...............AA   351
YX.seq           AGTCCGCTCTACGTTCCCAGCAACATGTGCATGATGGACATAGAAGACTCCGTAATGGATA.......................................   446

3F8.seq          AAGAATACACGTCTACGGCAACGGGTGATGCGGACGGATTGAACGTGTCCGTAGCACTAATTGGAGAAGGCGTGAGCATACCGCTAAGTTACATAGGCCT   570
B029.seq         AAGAATACACGTCTGCGGCAACGGGTGATGCGAACGGAGTGAACGTGTCCGTGGCACTAATGGGAGAAGGCGTGAGCATACCGCTAAGTTACATAGGCCT   588
GO.seq           ATGAATACACGTCTACGGCCACAGGTGATGCGGACGGAGTGAACGTGTCTGTGGCACTAATGGGAAAAGGCGTGAGCATACCGTTAAGTTACATAGGCCT   546
NA11.seq         AAGAATACGCGTCTACGGCCACGGGTGAGGCGGCCGGAGTGAACGTGTCAGTGGCACTAGTGGGAGAAGGCGTGAGCATACCGTTTAGTTACATAGGCCT   448
NA17.seq         AAGAATTCACGTCTACAGCCACGGGTGAGGCGGCCGGAGTGAACGTGTCCGTGGCACTAGTGGGAGAAGGCGTGAGCATACCGTTTAGTTACATAGGCCT   571
NP.seq           AAGAATTCACGTCTACAGCCACGGGTGAGGCGGCCGGAGTGAACGTGTCCGTGGCACTAGTGGGAGAAGGCGTGAGCATACCGTTTAGTTACATAGGCCT   569
NZ2.seq          AAGAATACGCGTCTACGGCCACGGGTGAGGCGGCCGGAGTGAACGTGTCAGTGGCACTAGTGGGAGAAGGCGTGAGCATACCGTTTAGTTACATAGGCCT   448
OV-HN3 12.seq    AAGAATACGCGTCTACGGCCACGGGTGAGGCGGCCGGAGTGAACGTGTCAGTGGCACTAGTGGGAGAAGGCGTGAGCATACCGTTTAGTTACATAGGCCT   447
OV-IA82.seq      AAGAATACACGTCTACGGCTACGGGTGAGGCGGCCGGAGTGAACGTGTCCGTGGCACTAGTGGGAGAAGGCGTGAAAATACCGTTTAGTTACATAGGCCT   572
OV-SA00.seq      AAGAATACACGTCTGCAGCCACGGGTGATGCGAACGGAGTGAACGTGTCCGTGGCACTAATAGGAGAAGGCGTGAGCATACCGCTAAGTTACATAGGCCT   567
SJ1.seq          ATGAATACACGTCTACGGCCACGGGTGATGCGGACGGAGTGAACGTGTCTGTGGCACTAATGGGAAAAGGCGTGAGCATACCGTTAAGTTACATAGGCCT   546
SY17.seq         AAGAATTTACGTCTACGGCCACGGGTGAGGCGGACGGAGTGAACGTGTCCGTGGCACTAGTGGGAGAAGACGTGAAAATACCGTTAAGTTACATAGGCCT   451
YX.seq           ATGAATACATGTCTGCGGCCACGGGTGATGCGGACGGAGTGAACGTGTCCGTGGCACTAATAGGAGAAGGCGTGAGCATACCGCTAAGTTACATAGGCCT   546
```

Figure 8

```
3F8.seq         TAGATTTAACCCGTCGCTTACAGATGGCTACCTGTACGTCAACGTCTCGTCACGGGCTCCTTGGGATCAACAGACTCTGGACCTATCCGCGAACGACGGC   670
B029.seq        CGGATTCAACCCATTGCTTAAAGATGGCTACCTGTACGTCAACGTCTCGTCACGGGCTCCTTGGGATCAACAGACTCTGGACCTATCCGCGAACGACGGC   688
GO.seq          CGGATTCAACCCATTGCTTGCAGATGGCTACCTGTACGTCAACGTCTCGTCACGAGCTCCTTGGGTTCAACAAACTCCGGACCTATCCGCGAACGGCGGC   646
NA11.seq        TGGATTCAACCCGTCGCTTGAAGATAGCTACCTGTACGTTAACGTCTCGTCACGAGCTCCTTGGGTTAAACAGACTTCGGACCTATCCGCGAACGGCGGC   548
NA17.seq        TGGATTCAACCCGTCGCTTGAAGATAGCTACTTGTACGTCAACGTCTCGTCACGAGCTCCTTGGGTTCAACAGACTCCAGACTTATCTGCAAACGACAGC   671
NP.seq          TGGATTCAACCCGTCGCTTGAAGATAGCTACTTGTACGTCAACGTCTCGTCACGAGCTCCTTGGGTTCAACAGACTCCAGACTTATCTGCAAACGACAGC   669
NZ2.seq         TGGATTCAACCCGTCGCTTGAAGATAGCTACCTGTACGTCAACGTCTCGTCACGAGCTCCTTGGGTTAAACAGACTTCGGACCTATCCGCGAACGGCGGC   548
OV-HN3_12.seq   TGGATTCAACCCGTCGCTTGAAGATAGCTACCTGTACGTTAACGTCTCGTCACGAGCTCCTTGGGTTAAACAGACTTCGGACCTATCCGCGAACGGCGGC   547
OV-IA82.seq     TGGATTCAACCCATC...TACAGATGGCTACCTGTACGTCAACGTCTCGTCACGAGCTCCTTGGGTTCAACAGACTCCAGACCTATCCGCGAACAGCGGC   669
OV-SA00.seq     CGGATTCAACCCATCGCTTGCAGATGGCTACCTGTACGTCAACGTCTCGTCACGAGCTCCTTGGGTTCAACAGACTCTAGACCTATCCGCGAACGGCGGC   667
SJ1.seq         AGGATTCAACCCATTGCTTGCAGATGGCTACTTGTACGTCAACGTCTCGTCACGAGCTCCTTGGGTTCAACAGACTCCGGACCTATCCGCGAACGGCGGC   646
SY17.seq        TGGATTCAACCCATC...TACAGATGGCTACCTGTACGTCAACGTCTCGTCACGAGCTCCTTGGGTTCAACAGACTCTAGACCTATCTGCGAACGACAGC   548
YX.seq          CGGATTTAACCCATCGCTTGCAGATGGCTACCTGTACGTCAACGTCTCGTCACGGGCTCCTTGGGTTCAACAGACTCAAGACTTATCTGCGAACGGCAGC   646

3F8.seq         TGGGGTATCAAACAGGTTCTAGAAAAAGAGATACTGGCCATCCAGATAGGGTGCGACAACCAAAAATTTCCCGAAGAACCCACAACTACCCAACCCCCCT   770
B029.seq        TGGGGTATTAAACAGGTTCTAGAAAAAGAGATACTGGCCATCCAGATAGGGTGCGACAACCAAAAATTTCCCGAAGAACCCACAACTACCCAACCCCCCT   788
GO.seq          TGGGGTATTAAACAGGTTCTAGAAGAAGAGATACTGGCCATCCAGATAGGGTGCGACAACCAAAAATTTCTAGAAGAACCCACAACAACCCAACCCCCCT   746
NA11.seq        TGGGGTATTAAACAGGTTCTAGAAAAAGAGTTACTGGCCATCCAAATAGGGTGCGACAACCAAAAATTTCCCGAAGAACCCACAACTAC...ACCCCCCT   645
NA17.seq        TGGGGTATTAAACAGGTTCTAGAAAAAGAGTTACTGGCCATCCAAATAGGGTGCGACAACCAAAAATTTCCCGAAGAACCCACAACCACCCAACCCCCCT   771
NP.seq          TGGGGTATTAAACAGGTTCTAGAAAAAGAGTTACTGGCCATCCAAATAGGGTGCGACAACCAAAAATTTCCCGAAGAACCCACAACCACCCAACCCCCCT   769
NZ2.seq         TGGGGTATCAAACAGGTTCTAGAAAAAGAGTTACTGGCCATCCAAATAGGGTGCGACAACCAAAAATTTCCCGAAGAACCCACAACTAC...ACCCCCCT   645
OV-HN3_12.seq   TGGGGTATTAAACAGGTTCTAGAAAAAGAGTTACTGGCCATCCAAATAGGGTGCGACAACCAAAAATTTCCCGAAGAACCCACAACTAC...ACCCCCCT   644
OV-IA82.seq     TGGGGTATTAAACAGGTTCTAGAAAAAGAGTTACTGGCCATCCAGATAGGGTGCGACAACCAAAAATTTCCCGAAGAACCCACAACTACCC......CCT   763
OV-SA00.seq     TGGGGTATTAATCAGATTCTAGAAAAAGAGTTACTGGCCATCCAAATAGGGTGCGACAACCAAAAATTTCCCGAAGAACCCACAACTACCCAACCTCCCT   767
SJ1.seq         TGGGGTATTAAACAGGTTCTAGAAGAAGAGATACTGGCCATCCAGATAGGGTGCGACAACCAAAAATTTCTAGAAGAACCCACAACTCCCCAACCCCCCT   746
SY17.seq        TGGGGTATTAAACAGGTTCTAGAAAAAGAGTTACTGGCCATCCAAATAGGGTGCGACAACCAAAAATTTCCTGAAGAACCCACAACCACCCAACCTCCCT   648
YX.seq          TGGGGTATTAAACAGGTTCTAGAACAAGAGATGCTGGCCATCCAGATAGGGTGCGACAACCAAAAATTTCCTGAAGAACCCACAACTACCCAACCTCCCT   746

3F8.seq         CACCTGTCACAACAACGCTTTCCTCAACAAC...TCTAGATCCGAATGACGAAAACACAGACACTACGCCG...ACAACCACCGGCGACAGTGTAGACGG   864
B029.seq        CACCTGTCACAACAACGCTTTCCCCAACAACGACTTTAAACCCGAATAACGAAAACACAGACACTACGCCG...ACGCCCACCGGCGCCAGTGTAGACGG   885
GO.seq          CACTTGTCACGACAACGCTTTCCTCAACAAC...GCTAGGTCTGACTGACGAGAACACAGACACTACGCCG...ACGACCACCAGCGCCAGTGTAGACAG   840
NA11.seq        CACCTGTCACGACAACGCTTTCCTCAACAAC...TCCAGATCTGAATGAAGAAAACACAAACACTACGCCG...ACGACCACCAGCGCCAGTGTAGACAG   739
NA17.seq        CACTTGTCACGACAACGCTTTCCTCAACAAC...TCTAGATCTGAATGACGAAAACACAGACACTACGCCGCCAACCACCACCAGCGCCAGCGTAAACAA   868
NP.seq          CACTTGTCACGACAACGCTTTCCTCAACAAC...TCTAGATCTGAATGACGAAAACACAGACACTACGCCGCCAACCACCACCAGCGCCAGCGTAAACAA   866
NZ2.seq         CACCTGTCACTACAACGCTTTCCTCAACAAC...TCCAGATCTGAATGAAGAAAACACAGAAAATACGCCG...ACGACCACCGGCGCCAGTGTAGACAG   739
OV-HN3_12.seq   CACCTGTCACGACAACGCTTTCCTCAACAAC...TCCAGATCTGAATGAAGAAAACACAGACACTACGCCG...ACGACCACCAGCGCCAGTGTAGACAG   738
OV-IA82.seq     CACTTGTCACGACAACGCTTTCCCCAACAACGACTTTAAATCCGAATAACGAAAACACAGACACTACGCCG...ACGCCCACCGGCGCCAGTGTAGACGG   860
OV-SA00.seq     CACCTGTCACGACAACGCTTTCCTCAACAAC...GCTAGATCTGACTGACGAAAACACAGACACTACGCCGCTGACCACCACCAGCGCCAGTGTAAACAG   864
SJ1.seq         CACTTGTCACGACAACGCTTTCCTCAACAAC...GCTAGGTCTGACTGACGAGAACACAGACACTACGCCG...ACGACCACCAGCGCCAGTGTAGACAG   840
SY17.seq        CACTTGTCACGACAACGCTTTCCTCAACAAC...TCCAGATCTGAATGACGAAAACACAGACACTACGCCG...ACGACCACTGGCGCCAGTGTAGACAG   742
YX.seq          CACCTGTCACGACAACGCTTTCCTCAACAAC...TCTAGATTCGAATGACGAAAACGCAGACACTACGCCGCCGACCACCACCAGCGCCAGTGTAAACAG   843

3F8.seq         AAAGCGCAATCCAGATGACTTTGACTTCTCGCTGATCGTGGACCCCCGATGCGTGACCTCTGTAAACCTGCACTTTGAGATTAAGGACGCGTGCATGGAC   964
B029.seq        AAAGCGCAATCCAGATGACATTGACTTCTCGCTGATCGTGGACCCCCGATGCGTGACCTCTGTAAACCTGCACTTCGAGATCAAGGACGCGTGCATGGAC   985
GO.seq          AAAGCGCAATCTAGATGACATTGACTTCTCGCTGCTCGTAGACACCCGATGCGTGACCTCGGTAAACCTGCACTTCGAGATTAAGGACGCGTGTATGGAC   940
NA11.seq        AAGGCGCAATCTAGATGACATTGACTTCTCGCTGCTCGTGGACCCCCGATGCGTGACCTCTGTAGACCTACACGTCGAGCTCAGGGACGCGTGCATGGAC   839
NA17.seq        AAAGCGCAATCCAGATGACTTTGACTTCTCGCTGCTCGTGGACCCCCGATGCGTGACCTCTGTAGACCTGCACGTCGAGCTCAGGGACGCGTGCATAGAC   968
NP.seq          AAAGCGCAATCCAGATGACTTTGACTTCTCGCTGCTCGTGGACCCCCGATGCGTGACCTCTGTAGACCTGCACGTCGAGCTCAGGGACGCGTGCATAGAC   966
NZ2.seq         AAAGCGCAATCCAGCTGACATTGACTTCTCGCTGCTCGTGGACCCCCGATGCGTGACCTCTGTAGACCTGCACGTCGAGCTCAGGGACGCGTGCATAGAC   839
OV-HN3_12.seq   AAGGCGCAATCTAGATGACATTGACTTCTCGCTGCTCGTGGACCCCCGATGCGTGACCTCTGTAGACCTACACGTCGAGCTCAGGGACGCGTGCATGGAC   838
OV-IA82.seq     AAAGCGCAATCCAGATGACATTGACTTCTCGCTGATCGTGGACCCCCGATGCGTGACCTCTGTAAACCTGCACTTCGAGCTCAAGGACGCGTGCATGGAC   960
OV-SA00.seq     AAAGCGCAATCCAGATGACTTTGACTTCTCGCTGCTCGTGGACCCCCGATGCGTGACCTCTGTAGACTTGCACGTCGAGCTCAGGGACGCGTGCATAGAC   964
SJ1.seq         AAAGCGCAATCTAGATGACATTGACTTCTCGCTGCTCGTAGACACCCGATGCGTGACCTCGGTAAACCTGCACTTCGAGATCAAGGACGCGTGTATGGAC   940
SY17.seq        AAAGCGCAATCCAGCTGACTTTAGTTTCTCGCTGCTCGTGGACCCCCGATGCGTGACTTCTGTAGACCTGCACGTCGAGCTCAGGGACGCGTGCATGGAG   842
YX.seq          AAAGCGCAATTCAGATGACTTTGACTTCTCGCTGCTCGTAGACCCCCGATGCGTGACCTCTGTAGACCTGCACGTCGAGCTCAGGGACGCGTGCATAGAC   943

3F8.seq         CACAAAGAATCGTCGCCGTTGTCGCTGAAGGGGGAATATGGAGACGGCGAACTAGTAAGAAAAGAAATCAAAAACGTGGGAAAGGATCACAATATGTGCA  1064
B029.seq        TACAAACAAGAGTCGCCGTTGTCGCTGAAGGGGAAATATGGAGACGGTGAACTAGTAAAAGAGGAGATTAAAGACGTGGGAAAGAACCACAATATGTGCA  1085
GO.seq          TACAAAAAAGAGTCGCCGTTGATGATGAAGGGGGGATATGGAGACGGCGAACTAGTAAGAAAAGAGATCAAATACGTGGGAACGAATCACACTATGTGCA  1040
NA11.seq        TACAAACAAGAGTCGCCGTTGTCGCTGAAGGGGAAATATGGAGACGGCGAGCTAGTAAAAAAGGAGATCAAAGACGTGGGAAAGAATTACAATATGTGCA   939
NA17.seq        TACAAAGAAACGTCGCAGTTGTCGCTGAAGGGGGAATATGGAGACGGCGAACTAATAAAAAAGGAGATCAAAGACGTGGGAAAGGATCACAATATGTGCA  1068
NP.seq          TACAAAGAAACGTCGCAGTTGTCGCTGAAGGGGGAATATGGAGACGGCGAACTAATAAAAAAGGAGATCAAAGACGTGGGAAAGGATCACAATATGTGCA  1066
NZ2.seq         TACAAACAAGAGTCGCCGTTGTCGCTGAAGGGGAAATATGGAGACGGCGAACTAGTAAAAAAGGAGATTAAAGACGTGGGAAAGAATCACAATATGTGCA   939
OV-HN3_12.seq   TACAAACAAGAGTCGCCGTTGTCGCTGAAGGGGAAATATGGAGACGGCGAGCTAGTAAAAAAGGAGATCAAAGACGTGGGAAAGAATTACAATATGTGCA   938
OV-IA82.seq     TACAAAAAAGAGTCGCCGTTGTCGCTGAAGGGGAAATATGGAGACAGTGAACTAGTAAAACAGGAGATTAAAGACGTGGGAAAGAATCACAATATGTGCA  1060
OV-SA00.seq     TACAAAGAAGCGTCGCAGTTGTCGCTGAAGGGGAAATATGGAGACGGCGAACTAGTAAAAAAGGAGATCAAAGACGTGGGAAAGGATCACAATATGTGCA  1064
SJ1.seq         TACAAAAAAGAGTCGCCGTTGATGATGAAGGGGGGATATGGAGACGGCGAACTAGTAAGAAAAGAGATCAAATACGTGGGAACGAATCACACTATGTGCA  1040
SY17.seq        TACAAAGAAACGTCGCCGTTGTCGCTGAAGGGGGAATATGGAGACGGCGAACTAGTAAAAAAGGAGATCAAAGACGTGGGAAAGAATCACAATATGTGCA   942
YX.seq          TACAAAGAAGCGTCGCTGTTGTCGCTGAAGGGGAAATATGGAGACGGCGAACTAGTAAAAAAGGAGATCAAAGACGTGGGAAAGGATCACAATATGTGCA  1043

3F8.seq         GTCTTAACCTCAGCCCTGGCCATTGAGCTGTTTTTATTCGGCAATATAATA.GGTGATTATTGAACATTAAACAAAACTTATCCCACAACGCCGCAACA  1162
B029.seq        GTCTTAACCTCAGCCCTGGCCATTGAGCTGTTTTTATTCGGCAATATAATA.GGTGATTATTGAACATTAAACAAAACTTATCCCACAACGCCGCAACA  1183
GO.seq          GTCTCAACCTCAGCCCTGGCTATTGATTTGTTTTTATTCGGCAATATAATA.GGTGATTATTGAACATTAAACAAAACTTATCCCACAACGCCTCAACA  1138
NA11.seq        GTCTTAACCTCAACCCTGGCAATTGAGATGTTTTTATTCGGCAATATAATA.GG.GATTATTGAACATTAAACAAAACTTATCCCACAACGCCGCAACA  1036
NA17.seq        GTCTTAACCTCAACCCTGGCAATTGAGCTGTTTTTATTCGGCAATATAATA.GGTGATTATTGAACATTAAACAAAACTTATCCCACAACGCCGCAACA  1166
NP.seq          GTCTTAACCTCAACCCTGGCAATTGAGCTGTTTTTATTCGGCG.TATAATA.GGTGATTATTGAACATTAAACAAAACTTATCCCACAACGCCGCAACA  1163
NZ2.seq         GTCTTAACCTCAACCCTGGCAATTGAGCTGTTTTTATTCGGCAATATAATA.GGTGATTATTGAACATTAAACAAAACTTATCCCACAACGCCGCAAC   1036
OV-HN3_12.seq   GTCTTAACCTCAACCCTGGCAATTGAGATGTTTTTATTCGGCAATATAATA.GG.GATTATTGAACATTAAACAAAACTTATCCCACAACGCCGCAACA  1035
OV-IA82.seq     GTCTTAACCTCAGCCCTGGCCATTGAGCTGTTTTTATTCGGCAATATAATAAGGTGATTATTGAACATTAAACAAAACTTATCCCACAACGCCGCAACA  1159
OV-SA00.seq     GTCTTAACCTCAGCCCTGGCCATTGAGATGTTTTTATTCGGCAAT.TAATA.GGTGATTATTGAACATTAAACAAAACTTATCCCACAACGCCGCAACA  1161
SJ1.seq         GTCTCAACCTCAGCCCTGGCTATTGATTTGTTTTTATTCGGCAATATAATA.GGTGATTATTGAACATTAAACAAAACTTATCCCACAACGCCGCAACA  1138
SY17.seq        GTCTTAACCTCAACCCTGGCAATTAAGCTGTTTTTATTCGGCAATATAATA.GGTGATTATTGAACATTAAACAAAACTTATCCCACAACGCCGCAACA  1040
YX.seq          GTCTTAACCTCAGCCCTGGCCATTGAGCTGTTTTTATTCGGCAATATAATA.GGTGATTATTGAACATTAAACAAAACTTATCCCACAACGCCGCAACA  1141
```

Figure 8（continued）

```
POV-601-1A1     1>aaatatactgtttgaacgcaaagacgccatgtcgcgacttcaaatactgacctcatttggacaaatctacgcacctgacgaagctcggctgcgagagatc>100
POV-601-3F8     1>aaatatactgtttgaacgcaaagacgccatgtcgcgacttcaaatactgacctcatttggacaaatctacgcacctgacgaagctcggctgcgagagatc>100

POV-601-1A1   101>gcgcgtgatttgggaatatgcaccataaaacgcgcattcggcgacatgctgtacggctttatagacttcaacccggtgcccctgacccaagtaaacatgc>200
POV-601-3F8   101>gcgcgtgatttgggaatatgcaccataaaacgcgcattcggcgacatgctgtacggctttatagacttcaacccggtgcccctgacccaagtaaacatgc>200

POV-601-1A1   201>tcatgcccaactgctacttcgcggtcaacggcaacctgcttccgtgcacggaggacttccggctcagactcccggcaacggagatctctgcggcctacct>300
POV-601-3F8   201>tcatgcccaactgctacttcgcggtcaacggcaacctgcttccgtgcacggaggacttccggctcagactcccggcaacggagatctctgcggcctacct>300

POV-601-1A1   301>gacgaaaacgggacggacgatcctgtgcggtagagacttcaacatagtagcgccgtcggggttcaagccgtccatgcggctgcgcaacctcagtcacgtg>400
POV-601-3F8   301>gacgaaaacgggacggacgatcctgtgcggtagagacttcaacatagtagcgccgtcggggttcaagccgtccatgcggctgcgcaacctcagtcacgtg>400

POV-601-1A1   401>tctgcgcttgtagagattttagagctttacgacgagtccggagagtaccaattcgtgctcggccccagcgcgcagttaatgctgcggctaatggagaagg>485
POV-601-3F8   401>tctgcgcttgtagagattttagagctttacgacgagtccggagagtaccaattcgtgctcggccccagcgcgcagttaatgctgcggctaatggagaagg>500

POV-601-1A1   485>~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~>485
POV-601-3F8   501>agaatgtctgtctgtttggcagaggttggtgcatagtggacctgcgcaagctggacgtagccatataattgctgctgctatgtcgtgcccgactctgtgc>600

POV-601-1A1   485>~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~>485
POV-601-3F8   601>gacaaagaagcagcttaccagacgactcttcgttcatgttctctaagcaagaacactggagtgagttgccatttccgtctccaaccatataattagcatc>700

POV-601-1A1   485>~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~~>485
POV-601-3F8   701>cttgtttttatcctgtatttttatcagttttagctagttaaaacataaatagtaaagctaaaaagaggagttctggaatcttgcaacgacagg>794
```

Figure 9

```
NP.seq           ...................MSRLQILTSFGQIYAPDEARLREIARDLGICTIKRAFGDML   41
YX.seq           ...................MSQLQILTSFGQIYAPDEARLREIARDLGICTIKRAFGDML   41
SJ1.seq          ...................MSQLQILTSFGQIYAPDEARLREIARDLGICTIKRAFGDML   41
GO.seq           ...................MSRLQILTSFGQIYAPDEARLREIARDLGICTIKRAFGDML   41
NA17.seq         ...................MSRLQILTSFGQIYAPDEARLREIARDLGICTIKRAFGDML   41
OV-SA00.seq      ...................MSRLQILTSFGQIYAPDEARLREIARDLGICTIKRAFGDML   41
B029.seq         ...................MSRLQILTSFGQIYAPDEARLREIARDLGICTIKRAFGDML   41
OV-IA82.seq      MHEGDARITENILFERKDAMSRLQILTSFGQIYAPDEARLREIARDLGICTIKRAFGDML   60
NA1-11.seq       ...................MSRLQILTSFGQIYAPDEARLREIARDLGVCTIKRAFGDML   41
OV-HN3_12.seq    ...................MSRLQILTSFGQIYAPDEARLREIARDLGVCTIKRAFGDML   41
SY17.seq         ...................MSRLQILTSFGQIYAPDEARLREIARDLGICTIKRAFGDML   41
NZ2.seq          ...................MSRLQILTSFGQIFAPDEARLREIARDLGICTIKRAFGDML   41
POV-601-3F8.seq  ...................MSRLQILTSFGQIYAPDEARLREIARDLGICTIKRAFGDML   41


NP.seq           YGFIDFNEVPLTCVNMLMSDFYFAVNGNLLPCTEDFRLRLPATDISAAYLTKTGRTILCG  101
YX.seq           YGFIDFNEVPLTCVNMLMSDFYFAVNGNLLPCTEDFRLRLPATDISAAYLTKTGRTILCG  101
SJ1.seq          YGFIDFNEVPLTCVNMLMSNCYFAVNGNLLPCTEDFRLRLPATEISAAYLTKTGRTILCG  101
GO.seq           YGFIDFNEVPLTCVNMLMSDCYFAVNGNLLPCTEDFRLRLPATEISAAYLTKTGRTILCG  101
NA17.seq         YGFIDFNEVPLTCVNMLMSDCYFAVNGNLLPCTEDFRLRLPATEISAAYLTKTGRTILCG  101
OV-SA00.seq      YGFIDFNEVPLTCVNMLMSNCYFAVNGNLLPCTEDFRLRLPATEISAAYLTKTGRTILCG  101
B029.seq         YGFIDFNEVPLTCVNMLMSNCYFAVNGNLLPCTEDFRLRLPATEISAAYLTKTGRTILCG  101
OV-IA82.seq      YGFIDFNEVPLTCVNMLMSNCYFAVNGNLLPCTEDFRLRLPATEISAAYLTRTGRTILCG  120
NA1-11.seq       YGFIDFDEVPLTCVNMLMPNCYFAVNGNLLPCTEDFRLRLPATEISAAYLTRTGRTILCG  101
OV-HN3_12.seq    YGFIDFDEVPLTCVNMLMPNCYFAVNGNLLPCTEDFRLRLPATEISAAYLTRTGRTILCG  101
SY17.seq         YGFIDFNEVPLTCVNMLMPNCYFAVNGNLLPCTEDFRLRLPATEISAAYLTRTGRTILCG  101
NZ2.seq          YGFIDFDEVPLTCVNMLMSNCYFAVNGNLLPCTEDFRLRLPATEISAAYLTRTGRTILCG  101
POV-601-3F8.seq  YGFIDFNEVPLTCVNMLMPNCYFAVNGNLLPCTEDFRLRLPATEISAAYLTKTGRTILCG  101


NP.seq           KDFNIVAPSGFKPSMRLRNLSEVSAIVEILEFYSESGEYQFVLGPSAQFMLRLMEKENVC  161
YX.seq           KDFNIVAPSGFKPSMRLRNLSEVSAIVEILEFYSESGEYQFVLGPSAQFMLRLMEKENVC  161
SJ1.seq          KDFNIVAPSGFKPSMRLRNLSEVSAIVEILEFYSESGEYQFVLGPSAQFMLRLMEKENVC  161
GO.seq           KDFNIIAPSGFKPSMRLRNLSEVSAIVEILEFYSESGEYQFVLGPSAQFMLRLMEKENVC  161
NA17.seq         KDFNIIAPSGFKPSMRLRNLSEVSAIVEILEFYSESGEYQFVLGPSAQFMLRLMEKENVC  161
OV-SA00.seq      KDFNIIAPLGFKPSMRLRNLSEVSAIVEILEFYSESGEYQFVLGPSAQFMLRLMEKENVC  161
B029.seq         KDFNIVAPSGFKPSMRLRDLSEVSAIVEILELYDESGEYQFVLGPSAQFMLRLMEKENVC  161
OV-IA82.seq      KDFNIVAPSGFKPSMRLRDLSEVSAIVEILELYDESGDYQFVLGPSAQFMLRLMEKENVC  180
NA1-11.seq       KDFNIVAPSGFKPSMRLRDLSEVSAIVEILEIYNESGEYQFVLGPSAQFMLRLMEKENVC  161
OV-HN3_12.seq    KDFNIVAPSGFKPSMRLRDLSEVSAIVEILEIYNESGEYQFVLGPSAQFMLRLMEKENVC  161
SY17.seq         KDFNIVAPSGFKPSMRLRDLSEVSAIVEILEIYNESGEYQFVLGPSAQFMLRLMEKENVC  161
NZ2.seq          KDFNIVAPSGFKPSMRLRDLSEVSAIVEILEVYDESGEYQFVLGPSAQFMLRLMEKENVC  161
POV-601-3F8.seq  RDFNIVAPSGFKPSMRLRNLSEVSAIVEILELYDESGEYQFVLGPSAQLMLRLMEKENVC  161


NP.seq           LFGSGWCIVDLRKLIVTI                                            179
YX.seq           LFGSGWCIVDLRKLIVTI                                            179
SJ1.seq          LFGSGWCIVDLRKLIVTI                                            179
GO.seq           LFGSGWCIVDLRKLIVTI                                            179
NA17.seq         LFGSGWCIVDLRKLIVTI                                            179
OV-SA00.seq      LFGSGWCIVDLRKLIVTI                                            179
B029.seq         LFGNGWCIVDLRKLIVTI                                            179
OV-IA82.seq      LFGNGWCIVDLRKLIVTI                                            198
NA1-11.seq       LFGSGWCIVDLRKLIVPI                                            179
OV-HN3_12.seq    LFGSGWCIVDLRKLIVPI                                            179
SY17.seq         LFGSGWCIVDLRKLIVTI                                            179
NZ2.seq          LFGSGWCIVDLRKLIVPI                                            179
POV-601-3F8.seq  LFGRGWCIVDLRKLIVAI                                            179
                                                                               179
```

**Figure 10**

```
B029.seq          .............................AAATATACTGTTTGAACGCAAAGACACCATGTCGCGACTTCAAATACTGAC   51
GO.seq            .............................AAATATACTGTTTGAACGCAAAGACGCCATGTCGCGACTTCAAATACTGAC   51
OV-HN3_12.seq     .........................CTGAAAATATACTGTTTGAACGCAAAGACGCCATGTCGCGACTTCAAATACTGAC   55
NA1_11.seq        .........................CTGAAAATATACTGTTTGAACGCAAAGACGCCATGTCGCGACTTCAAATACTGAC   55
NA17.seq          .........................CTGAAAATATACTGTTTGAACGCAAAGACGCCATGTCGCGACTTCAAATACTGAC   55
NP.seq            .........................CTGAAAATATACTGTTTGAACGCAAAGACGCCATGTCGCGACTTCAAATACTGAC   55
NZ2.seq           .........................CTGAAAATATACTGTTTGAACGCAAAGACGCCATGTCGCGACTTCAAATACTGAC   55
OV-IA82.seq       ATGCATGAAGGAGATGCACGGATAACTGAAAATATACTGTTTGAACGCAAAGACGCCATGTCGCGACTTCAAATACTGAC   80
OV-SA00.seq       .............................AAATATACTGTTTGAACGCAAAGACGCCATGTCGCGACTTCAAATACTGAC   51
POV-601-3F8..seq  .............................AAATATACTGTTTGAACGCAAAGACGCCATGTCGCGACTTCAAATACTGAC   51
SJ1.seq           .........................CTGAAAATATACTGTTTGAATGCAAAGACGCCATGTCGCAACTTCAAATACTGAC   55
SY17.seq          .........................CTGAAAATATACTGTTTGAACGCAAAGACGCCATGTCGCGACTTCAAATACTGAC   55
YX.seq            .........................CTGAAAATATACTGTTTGAATGCAAAGACGCCATGTCGCAACTTCAAATACTGAC   55

B029.seq          CTCATTTGGACAAATCTACGCACCC.ACGAAGCTCGGCTGCGCGAGATCGCGCGTGATTTGGGAATATGCACCATAAAAC  130
GO.seq            CTCATTTGGACAAATCTACGCACCCGACGAAGCTCGGCTGCGCGAGATCGCGCGTGACTTGGGAATATGCACCATAAAAC  131
OV-HN3_12.seq     CTCATTTGGACAAATCTACGCACCCGACGAAGCTCGGCTGCGCGAGATCGCGCGTGATTTGGGAGTATGCACCATAAAAC  135
NA1_11.seq        CTCATTTGGACAAATCTACGCACCCGACGAAGCTCGGCTGCGCGAGATCGCGCGTGATTTGGGAGTATGCACCATAAAAC  135
NA17.seq          CTCATTTGGACAAATCTACGCACCCGACGAAGCTCGGCTGCGCGAGATCGCGCGTGACTTGGGAATATGCACCATAAAAC  135
NP.seq            CTCATTTGGACAAATCTACGCACCCGACGAAGCTCGGCTGCGCGAGATCGCGCGTGACTTGGGAATATGCACCATAAAAC  135
NZ2.seq           CTCATTTGGACAAATCTTCGCACCCGACGAAGCTCGGCTGCGCGAGATCGCGCGTGATTTGGGAATATGCACCATAAAAC  135
OV-IA82.seq       CTCATTTGGACAAATCTACGCACCCGACGAAGCTCGGCTGCGCGAGATCGCGCGTGATTTGGGAATATGCACCATAAAAC  160
OV-SA00.seq       CTCATTTGGACAAATCTACGCACCCGACGAAGCTCGGCTGCGCGAGATCGCGCGTGACTTGGGAATATGCACCATAAAAC  131
POV-601-3F8..seq  CTCATTTGGACAAATCTACGCACCTGACGAAGCTCGGCTGCGAGAGATCGCGCGTGATTTGGGAATATGCACCATAAAAC  131
SJ1.seq           CTCATTTGGACAAATCTACGCACCCGACGAAGCTCGGCTGCGCGAGATCGCGCGTGATTTGGGAATATGCACCATAAAAC  135
SY17.seq          CTCATTTGGACAAATCTACGCACCCGACGAAGCTCGGCTGCGCGAGATCGCGCGTGATTTGGGAATATGCACCATAAAAC  135
YX.seq            CTCATTTGGACAAATCTACGCACCCGACGAAGCTCGGCTGCGCGAGATCGCGCGTGATTTGGGAATATGCACCATAAAAC  135
Consensus

B029.seq          GCGCATTCGGCGACATGCTGTACGGCTTTATAGACTTCAACCCGGTGCCCCTGACCCAAGTAAACATGCTCATGTCCAAC  210
GO.seq            GCGCATTCGGCGACATGCTGTACGGCTTTATAGACTTCAACCCGGTGCCCCTTACCCAAGTAAACATGCTCATGTCTGAC  211
OV-HN3_12.seq     GCGCATTCGGCGACATGCTGTACGGCTTTATAGACTTCGACCCGGTTCCCCTGACCCAAGTAAACATGCTCATGCCCAAC  215
NA1_11.seq        GCGCATTCGGCGACATGCTGTACGGCTTTATAGACTTCGACCCGGTTCCCCTGACCCAAGTAAACATGCTCATGCCCAAC  215
NA17.seq          GCGCATTCGGCGACATGCTGTACGGCTTTATAGACTTCAACCCGGTGCCCCTTACCCAAGTAAACATGCTCATGTCCGAC  215
NP.seq            GCGCATTCGGCGACATGCTGTACGGCTTTATAGACTTCAACCCGGTGCCCCTTACCCAAGTAAACATGCTCATGTCCGAC  215
NZ2.seq           GCGCATTCGGCGACATGCTGTACGGCTTTATAGACTTCGACCCGGTGCCCCTGACCCAAGTAAACATGCTCATGTCCAAC  215
OV-IA82.seq       GCGCATTCGGCGACATGCTGTACGGCTTTATAGACTTCAACCCGGTGCCCCTGACCCAAGTAAACATGCTCATGTCCAAC  240
OV-SA00.seq       GCGCATTCGGCGACATGCTGTACGGCTTTATAGACTTCAACCCGGTGCCCCTGACCCAAGTAAACATGCTCATGTCCAAC  211
POV-601-3F8..seq  GCGCATTCGGCGACATGCTGTACGGCTTTATAGACTTCAACCCGGTGCCCCTGACCCAAGTAAACATGCTCATGCCCAAC  211
SJ1.seq           GCGCATTCGGCGACATGCTGTACGGCTTTATAGACTTCAACCCGGTGCCCCTTACCCAAGTAAACATGCTCATGTCCAAC  215
SY17.seq          GCGCATTCGGCGACATGCTGTACGGCTTTATAGACTTCAACCCGGTGCCCCTGACCCAAGTAAACATGCTCATGCCCAAC  215
YX.seq            GCGCATTCGGCGACATGCTGTACGGCTTTATAGACTTCAACCCGGTGCCCCTTACCCAAGTAAACATGCTCATGTCCGAC  215
```

```
B029.seq          TGCTACTTCGCGGTCAACGGCAACCTGCTTCCGTGCACGGAGGACTTCCGGCTCAGACTCCCGGCAACGGAGATCTCTGC   290
GO.seq            TGCTACTTCGCGGTCAACGGCAACCTGCTTCCGTGCACGGAGGACTTCCGGCTCAGACTCCCGGCGACGGAGATCTCCGC   291
OV-HN3_12.seq     TGCTACTTCGCGGTTAACGGCAACCTGCTTCCGTGCACGGAGGACTTCAGGCTCAGACTCCCGGCAACGGAGATCTCCGC   295
NA1_11.seq        TGCTACTTCGCGGTTAACGGCAACCTGCTTCCGTGCACGGAGGACTTCAGGCTCAGACTCCCGGCAACGGAGATCTCCGC   295
NA17.seq          TGCTACTTCGCGGTCAACGGCAACCTGCTTCCGTGCACGGAGGACTTCCGGCTCAGACTCCCGGCGACGGAGATCTCCGC   295
NP.seq            TTCTACTTCGCGGTCAACGGCAACCTGCTTCCGTGCACGGAGGACTTCCGGCTCAGACTCCCGGCGACGGATATCTCTGC   295
NZ2.seq           TGCTACTTCGCGGTCAACGGCAACCTGCTTCCGTGCACGGAGGACTTCCGGCTCAGACTCCCGGCAACGGAGATCTCTGC   295
OV-IA82.seq       TGCTACTTCGCGGTCAACGGCAACCTGCTTCCGTGCACGGAGGACTTCCGGCTCAGACTCCCGGCAACGGAGATCTCTGC   320
OV-SA00.seq       TGTTACTTCGCGGTCAACGGCAACCTGCTTCCGTGCACGGAGGACTTCCGGCTCAGACTCCCGGCGACGGAGATCTCTGC   291
POV-601-3F8..seq  TGCTACTTCGCGGTCAACGGCAACCTGCTTCCGTGCACGGAGGACTTCCGGCTCAGACTCCCGGCAACGGAGATCTCTGC   291
SJ1.seq           TGCTACTTCGCAGTCAACGGCAACCTGCTTCCGTGCACGGAGGACTTCCGGCTCAGACTCCCGGCGACGGAGATCTCTGC   295
SY17.seq          TGCTACTTCGCTGTCAACGGCAACCTGCTTCCGTGCACGGAGGACTTCCGGCTTAGACTCCCGGCAACGGAGATCTCTGC   295
YX.seq            TTCTACTTCGCGGTCAACGGCAACCTGCTTCCGTGCACGGAGGACTTCCGGCTCAGACTCCCGGCGACGGATATCTCTGC   295

B029.seq          AGCCTACCTGACGAAAACGGGACGGACGATCCTGTGCGGCAAAGACTTCAACATAGTGGCGCCGTCGGGGTTCAAGCCGT   370
GO.seq            GGCCTACCTGACGAAAACGGGACGGACGATCCTGTGCGGCAAAGACTTCAACATAATAGCGCCGTCGGGTTTCAAGCCGT   371
OV-HN3_12.seq     GGCCTACCTGACGAGAACGGGACGGACGATCCTGTGCGGCAAAGACTTCAACATAGTGGCGCCGTCAGGGTTCAAGCCGT   375
NA1_11.seq        GGCCTACCTGACGAGAACGGGACGGACGATCCTGTGCGGCAAAGACTTCAACATAGTGGCGCCGTCAGGGTTCAAGCCGT   375
NA17.seq          GGCCTACCTGACGAAAACGGGACGGACGATCCTGTGCGGCAAAGACTTCAACATAATAGCGCCGTCGGGTTTCAAGCCGT   375
NP.seq            GGCCTACCTGACGAAAACGGGACGGACGATCCTGTGCGGCAAAGACTTCAACATAGTGGCGCCGTCAGGGTTCAAGCCGT   375
NZ2.seq           GGCCTACCTGACGAGAACGGGACGGACGATCCTGTGCGGCAAAGACTTCAACATAGTAGCGCCGTCGGGGTTCAAGCCGT   375
OV-IA82.seq       GGCCTACCTGACGAGAACGGGACGGACGATCCTGTGCGGCAAAGACTTCAACATAGTGGCGCCGTCAGGGTTCAAGCCGT   400
OV-SA00.seq       GGCCTACCTGACGAAAACGGGACGGACGATCCTGTGCGGCAAAGACTTCAACATAATAGCGCCGTTGGGGTTCAAGCCGT   371
POV-601-3F8..seq  GGCCTACCTGACGAAAACGGGACGGACGATCCTGTGCGGTAGAGACTTCAACATAGTAGCGCCGTCGGGGTTCAAGCCGT   371
SJ1.seq           GGCCTACCTGACGAAAACGGGACGGACGATCCTGTGCGGCAAAGACTTCAACATAGTGGCGCCGTCGGGGTTCAAGCCGT   375
SY17.seq          GGCCTACCTAACGAGAACGGGACGGACGATCCTGTGCGGCAAAGACTTCAACATAGTGGCGCCGTCAGGGTTCAAGCCGT   375
YX.seq            GGCCTACCTGACGAAAACGGGACGGACGATCCTGTGCGGCAAAGACTTCAACATAGTGGCGCCGTCAGGGTTCAAGCCGT   375

B029.seq          CCATGCGGCTGCGCGACCTCAGTCACGTGTCTGCGCTTGTAGAGATCCTGGAGCTCTACGACGAGTCCGGGGAGTACCAA   450
GO.seq            CCATGCGGCTGCGCAACCTCAGTCACGTGTCTGCGCTTGTAGAAATCTTGGAGTTTTACAGCGAGTCCGGGGAGTACCAA   451
OV-HN3_12.seq     CCATGCGGCTGCGCGACCTTAGTCACGTGTCTGCGCTTGTAGAGATCCTGGAAATCTACAACGAGTCCGGGGAGTACCAA   455
NA1_11.seq        CCATGCGGCTGCGCGACCTTAGTCACGTGTCTGCGCTTGTAGAGATCCTGGAAATCTACAACGAGTCCGGGGAGTACCAA   455
NA17.seq          CCATGCGGCTGCGCAACCTCAGTCACGTGTCTGCGCTTGTAGAGATCTTGGAGTTTTACAGCGAGTCCGGGGAGTACCAA   455
NP.seq            CCATGCGGCTGCGCAACCTCAGTCACGTGTCTGCGCTTGTAGAGATCTTGGAGTTTTACAGCGAGTCCGGGGAGTACCAA   455
NZ2.seq           CCATGCGGCTGCGCGACCTCAGTCACGTGTCTGCGCTTGTAGAGATCCTGGAAGTCTACGACGAGTCCGGGGAGTACCAA   455
OV-IA82.seq       CCATGCGGCTGCGCGACCTTAGTCACGTGTCTGCGCTTGTAGAGATCCTGGAGCTCTACGACGAGTCCGGGGATTACCAA   480
OV-SA00.seq       CCATGCGGCTGCGCAACCTCAGTCACGTGTCTGCGCTTGTAGAGATCTTGGAGTTTTACAGCGAGTCCGGGGAGTACCAA   451
POV-601-3F8..seq  CCATGCGGCTGCGCAACCTCAGTCACGTGTCTGCGCTTGTAGAGATTTTAGAGCTTTACGACGAGTCCGGAGAGTACCAA   451
SJ1.seq           CCATGCGGCTGCGCAACCTCAGTCACGTGTCTGCGCTTGTAGAGATCTTGGAGTTTTACAGCGAGTCCGGGGAGTACCAA   455
SY17.seq          CCATGCGGCTGCGCGACCTCAGTCACGTGTCTGCGCTTGTAGAGATCCTGGAAATCTACAACGAGTCCGGGGAGTACCAA   455
YX.seq            CCATGCGGCTGCGCAACCTCAGTCACGTGTCTGCGCTTGTAGAGATCTTGGAGTTTTACAGCGAGTCCGGGGAGTACCAA   455
```

Figure 11

```
B029.seq          TTCGTGCTCGGCCCCAGCGCGCAGTTCATGCTGCGGCTGATGGAGAAGGAGAATGTCTGTCTGTTCGGCAACGGGTGGTG   530
GO.seq            TTCGTGCTCGGCCCCAGCGCGCAGTTCATGCTGCGGCTGATGGAGAAGGAGAATGTCTGTCTGTTCGGCAGCGGTTGGTG   531
OV-HN3_12.seq     TTCGTGCTCGGCCCCAGCGCGCAGTTCATGCTGCGGCTGATGGAGAAGGAAAACGTCTGTCTGTTCGGCAGCGGGTGGTG   535
NA1_11.seq        TTCGTGCTCGGCCCCAGCGCGCAGTTCATGCTGCGGCTGATGGAGAAGGAAAACGTCTGTCTGTTCGGCAGCGGGTGGTG   535
NA17.seq          TTCGTGCTCGGCCCCAGCGCGCAGTTCATGCTGCGGCTGATGGAGAAGGAAAACGTCTGTCTGTTTGGCAGCGGTTGGTG   535
NP.seq            TTTGTGCTCGGCCCCAGCGCGCAGTTCATGCTGCGGCTGATGGAGAAGGAGAACGTCTGTCTGTTCGGCAGCGGTTGGTG   535
NZ2.seq           TTCGTGCTCGGCCCCAGCGCGCAGTTCATGCTGCGGCTGATGGAGAAGGAGAACGTCTGTCTGTTCGGCAGCGGGTGGTG   535
OV-IA82.seq       TTCGTGCTCGGCCCCAGCGCGCAGTTCATGCTGCGGCTGATGGAGAAGGAGAATGTCTGTCTGTTCGGCAACGGTTGGTG   560
OV-SA00.seq       TTTGTGCTCGGCCCCAGCGCGCAGTTCATGCTGCGTCTGATGGAGAAGGAGAACGTCTGTCTGTTCGGCAGCGGTTGGTG   531
POV-601-3F8..seq  TTCGTGCTCGGCCCCAGCGCGCAGTTAATGCTGCGGCTAATGGAGAAGGAGAATGTCTGTCTGTTTGGCAGAGGTTGGTG   531
SJ1.seq           TTCGTGCTCGGCCCCAGCGCGCAGTTCATGCTGCGGCTGATGGAGAAGGAGAATGTCTGTCTGTTCGGCAGCGGTTGGTG   535
SY17.seq          TTCGTGCTCGGCCCCAGCGCGCAGTTCATGCTGCGGCTGATGGAGAAGGAAAACGTCTGTCTGTTCGGCAGCGGGTGGTG   535
YX.seq            TTCGTGCTCGGCCCCAGCGCGCAGTTCATGCTGCGGCTGATGGAGAAGGAGAACGTCTGTCTGTTCGGCAGCGGATGGTG   535

B029.seq          CATAGTGGACCTGCGCAAGCTAGACGTAACCATATAATTGCTGCT...ATGTCGTACCCGACTCTGTGCTACAAAGACAG   607
GO.seq            CATAGTGGACCTGCGCAAGCTAGACGTAACCATATAATTGCTGC.GCTATGTCGTGCCCGACTCTGTGCGACAAAGACAG   610
OV-HN3_12.seq     CATAGTGGACCTGCGCAAGCTGGACGTACCCATATAATCAACATCCTTGTTTTTATCCTGTCTTTTTATCAGTTTTT...   612
NA1_11.seq        CATAGTGGACCTGCGCAAGCTGGACGTACCCATATA.TCAACATCCTTGTTTTTATCCTGTCTTTTTATCAGTTTTT...   611
NA17.seq          CATAGTGGACCTGCGCAAGCTGGACGTAACCATATAATTGCTGCTGCTATATCGTACCTGACTCTGTGTTACAAAGACAG   615
NP.seq            CATAGTGGACCTGCGCAAGCTAGACGTAACCATATAATTGCTGC.GCTATGTCGTACCTGACTCTGTGT.ACAAAGACAG   613
NZ2.seq           CATAGTGGACCTGCGCAAGCTGGACGTACCCATATAATCAGCATCCTTGTTTTTATCCTGTCTTTTTATCAGTTTTT...   612
OV-IA82.seq       CATAGTGGACCTGCGCAAGCTAGACGTAACCATATAATTGCTGCTGCTATGTCGTGCCCGACTCTGTGCGACAAAGACAG   640
OV-SA00.seq       CATAGTGGACCTGCGCAAGCTAGACGTAACTATATAATTGCTGC.GCTATGTCGTGCCCGACTCTGTGCGACAAAGACAG   610
POV-601-3F8..seq  CATAGTGGACCTGCGCAAGCTGGACGTAGCCATATAATTGCTGCTGCTATGTCGTGCCCGACTCTGTGCGACAAAGA.AG   610
SJ1.seq           CATAGTGGACCTGCGCAAGCTAGACGTAACCATATAATTGCTGC.GCTATGTCGTGCCCGACTCTGTGCGACAAAGACAG   614
SY17.seq          CATAGTGGACCTGCGCAAGCTGGACGTAACCATATAATCAGCATCCTTGTTTTTATCCTGTCTTTTTATCAGTTTTT...   612
YX.seq            CATAGTGGACCTGCGCAAGCTGGACGTAACCATATAATTGCTGCTGCTATATCGTACCTGACTCTGTGTTTTAAAGACAG   615

B029.seq          CAGCTCACCAGAC...TCTTCGTCCCTGTTCTCCAAGCAAGAACACTGGAGTGAGTTGCCATTTCCGTCTCCAACCATAT   684
GO.seq            AAGCTCACCAAAC...TCTTCGTCTCTGTTCTCCAAGCAAGAACACTGGAGTGATTTGCCATTTCCGTCTCCAAC.ATAT   686
OV-HN3_12.seq     .AGCTAGTTAAAA...CATAAATAGTAAAGCTAAAAAGAGGAGTTCTGGAGTC..TTGCAATAACCAGG...........   675
NA1_11.seq        TAGCTAGTTAAAA...CATAAATAGTAAAGCTAAAAAGAGGAGTTCTGGAGTC..TTGCAATAACCAGG...........   675
NA17.seq          CAGCTCACCAGAC...TCTTCGTCCCTGTTCTCTAAGCAAGAACACTGGAGTGAGTTGCCATTTCCGTCTCCAAC.ATAT   691
NP.seq            CAGCTCACCAGAC...TCTTCGTCCCTGTTCTCTAAGCAAGAACACTGGAGTGAGTTGCCATTTCCGTCTCCGAC.ATAT   689
NZ2.seq           TAGCTAGTTAAAA...CATAAATAGTAAAGCTAAAAAGAGGAGTTCTGGAGTC..TTGCAACAACCAGG...........   676
OV-IA82.seq       CGGCTAACCAGAC...TCTTCGTCCCTGTTCTCCAAGCAAAAA.ACTGGAGTGAGTTGCCATTTCCGTCTCCAACCATAT   716
OV-SA00.seq       AAGCTCACCAAAC...TCTTCGTCTCTGTTCTCCAAGCAAGAACACTGGAGTGATTTGCCATTTCCGTCTCCAAC.ATAT   686
POV-601-3F8..seq  CAGCTTACCAGACGACTCTTCGTTCATGTTCTCTAAGCAAGAACACTGGAGTGAGTTGCCATTTCCGTCTCCAACCATAT   690
SJ1.seq           AAGCTCACCAAAC...TCTTCGTCTCTGTTCTCCAAGTAAGAACACTGGAGTGATTTGCCATTTCCGTCTCCAAC.ATAT   690
SY17.seq          TAGCTAGTTAAAA...CATAAATAGTAAAGCTAAAAAGAGGAGTTCTGGAGTC..TTGTAACAACCAGG...........   676
YX.seq            CAGCTCACC.GAC...TCTTCGTCCCTGTTCTCCAAGCAAGAACACTGGAGTGATTTGCCATTTCCGTCTCCAAC.ATAT   690

B029.seq          AATTAGCATCCTTGTTTTTATCCTGTATTTTTATCAGTTTTTATGCTAGTTAAAACATAAATAGTAAGGCTAAAAAGAAG   764
GO.seq            AATTAGCATCCTTGTTTTTATCTTGTCTTTTTATCAGTTTTTATGTTAGTTAAAACATAAATAGTAAAGCTAAAAAGAGT   766
OV-HN3_12.seq     ................................................................................   675
NA1_11.seq        ................................................................................   675
NA17.seq          AATTAGCATCCTTGTTTTTATCTTGTCTTTTTATCAGTTTTTATGCTAGTTAAAACATAAATAGTAAAGCTAAAAAGAGT   771
NP.seq            AATTAGCATCCTTGTTTTTATCCTGTCTTTTTATCAGTTTTTATGCTAGTTAAAACATAAATAGTAAAGCTAAAAAGAGT   769
NZ2.seq           ................................................................................   676
OV-IA82.seq       AATTAGCATCCTTGTTTTTATCCTGTATTTTTATCAGTTTTTATGCTAGTTAAAACATAAATAGTAAGGCTAAAAAGAAG   796
OV-SA00.seq       AATTAGCATCCTTGTTTTTATCTTGTCTTTTTATCAGTTTTTATGTTAGTTAAAACATAAATAGTAAAGCTAAAAAGAGT   766
POV-601-3F8..seq  AATTAGCATCCTTGTTTTTATCCTGTATTTTTATCAGTTTTTA.GCTAGTTAAAACATAAATAGTAAAGCTAAAAAGAGG   769
SJ1.seq           AATTAGCATCCTTGTTTTTATCTTGTCTTTTTATCAGTTTTTATGCTAGTTAAAACATAAATAGTAAAGCTAAAAAGAGT   770
SY17.seq          ................................................................................   676
YX.seq            AATTAGCATCCTTGTTTTTATCTTGTCTTTTTATCAGTTTTTATGTTAGTTAAAACATAAATAGTAAAGCTAAAAAGAGT   770

B029.seq          AGTTCTAGAATCTTGCAACAACCAAG                                                         790
GO.seq            A.CTCTGGAATCTTGCAACAGTCAGG                                                         791
OV-HN3_12.seq     ..........................                                                         675
NA1_11.seq        ..........................                                                         675
NA17.seq          A.CTCTGGAATCTTGCAACAATCAGG                                                         796
NP.seq            A.CTCTGGAATCTTGCAACAATCAGG                                                         794
NZ2.seq           ..........................                                                         676
OV-IA82.seq       AGTTCTAGAATCTTGCAACAACCAAG                                                         822
OV-SA00.seq       A.CTCTGGAATCTTGCAACAGTCAGG                                                         791
POV-601-3F8..seq  AGTTCTGGAATCTTGCAACGACAGG                                                          794
SJ1.seq           A.CTCTGGAATCTTGCAACAGTCAGG                                                         795
SY17.seq          ..........................                                                         676
YX.seq            A.CTCTGGAATCTTGCAACAGTCAGG                                                         795
```

Figure 11（continued）

```
POV-601-1A1.seq   MSRLQILTSFGQIYAPDEARLREIARDLGICTIKRAFGDMLYGFIDFNEVPLTQVNMLMPNCYFAVNGNLIPCTEDFRLR    80
POV-601-3F8.seq   MSRLQILTSFGQIYAPDEARLREIARDLGICTIKRAFGDMLYGFIDFNEVPLTQVNMLMPNCYFAVNGNLIPCTEDFRLR    80

POV-601-1A1.seq   LPATEISAAYLTKTGRTILCGRDFNIVAPSGFKPSMRLRNLSEVSAIVEILELYDESGEYQFVLGPSAQLMLRR......   154
POV-601-3F8.seq   LPATEISAAYLTKTGRTILCGRDFNIVAPSGFKPSMRLRNLSEVSAIVEILELYDESGEYQFVLGPSAQLMLRLMEKENV   160

POV-601-1A1.seq   ...................                                                                154
POV-601-3F8.seq   CLFGRGWCIVDLRKLIVAI                                                                179
```

Figure 12

```
POV-601-3F8   MKAVLLLALLGAFTNAAPLLSNQRLGSAEEEKFCSTHQGEVHARFWLQMRVGVRHSPLYTPSNMCMMDIEDSTDTEDSTM  80
POV-601-1A1   --------------------------------------------------------------------------------

POV-601-3F8   EKEYTSTATGDADGLNVSVALIGEGVSIPLSYIGLRFNPSLTDGYLYVNVSSRAPWDQQTLDLSANDGWGIKQVLEKEIL  160
POV-601-1A1   ------------------------GVSIPLSYIGLRFNPSLTDGYLYVNVSSRAPWDQQTLDLSANDGWGIKQVLEKEIL  56

POV-601-3F8   AIQIGCDNQKFPEEPTTTQPPSPVTTTLSSTTLDPNDENTDTTPTTTGDSVDGKRNPDDFDFSLIVDPRCVTSVNLHFEI  240
POV-601-1A1   AIQIGCDNQKFPEEPTTTQPPSPVTTTLSSTTLDPNDENTDTTPTTTGDSVDGKRNPDDFDFSLIVDPRCVTSVNLHFEI  136

POV-601-3F8   KDACMDHKESSPLSLKGEYGDGELVRKEIKNVGKDHNMCSLNLSPGH  287
POV-601-1A1   KDACMDHKESSPLSLKGEYGDGELVRKEIKNVGKDHNMCSLNLSPGH  183
```

Figure 13

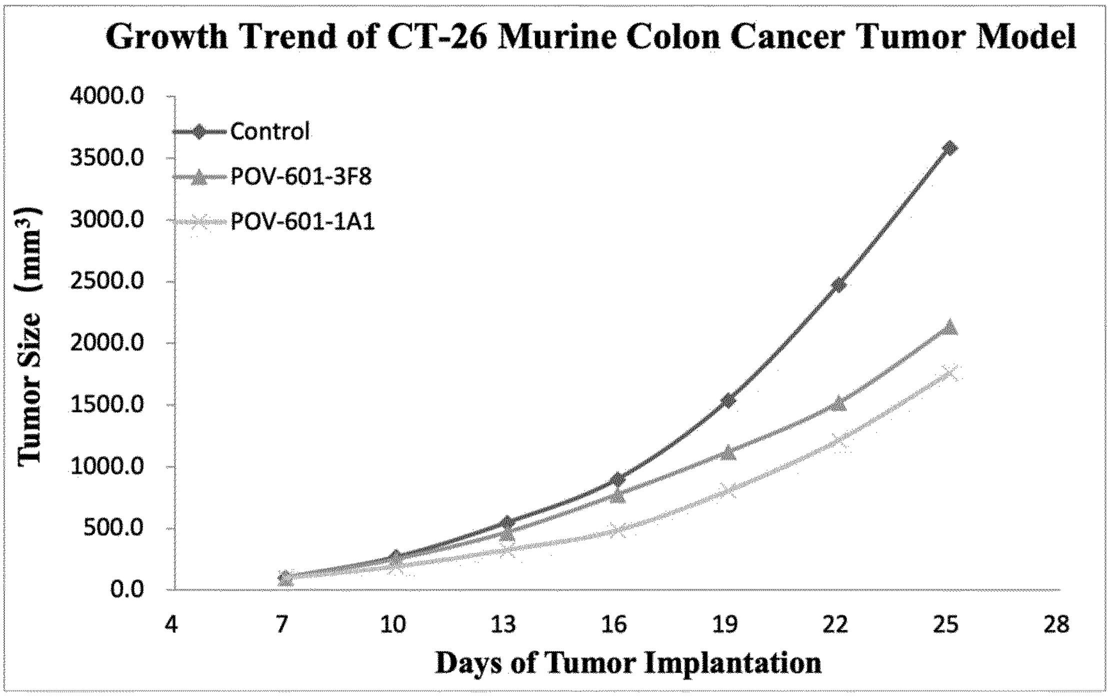


Figure 14

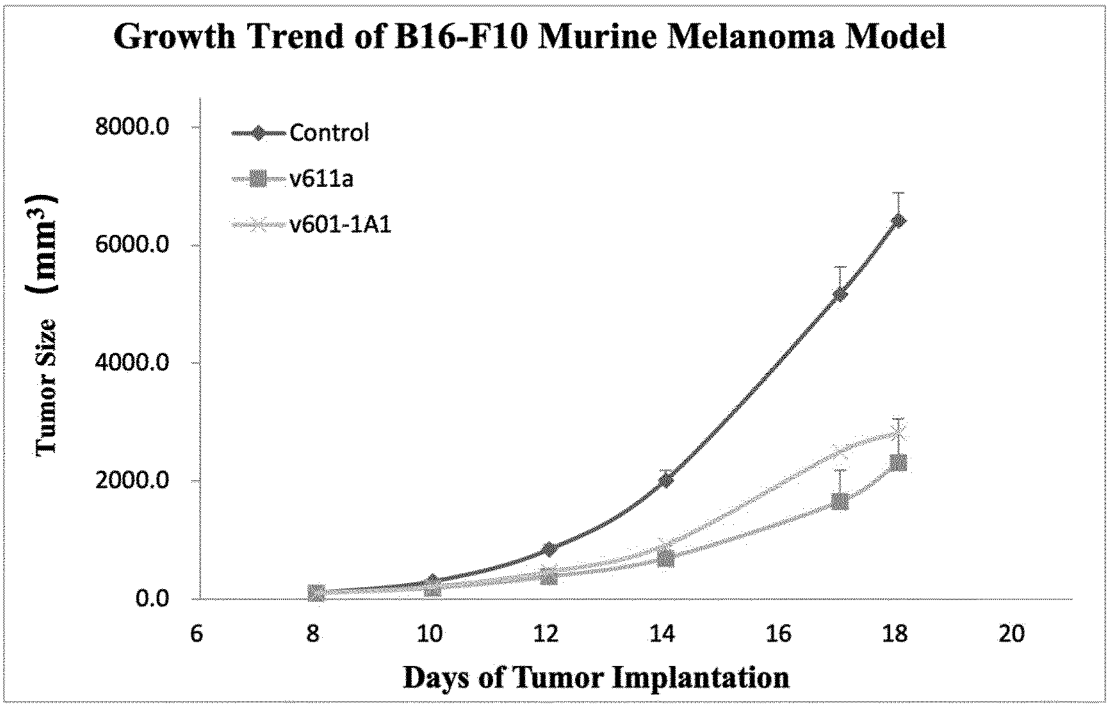


Figure 15

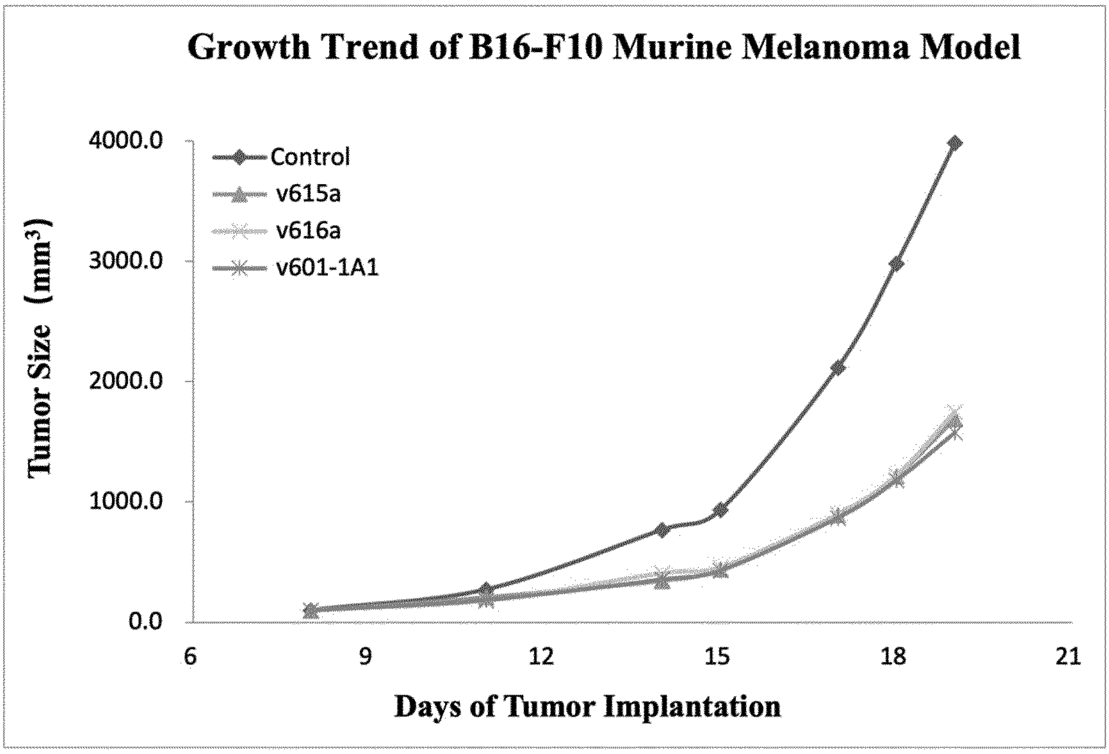


Figure 16

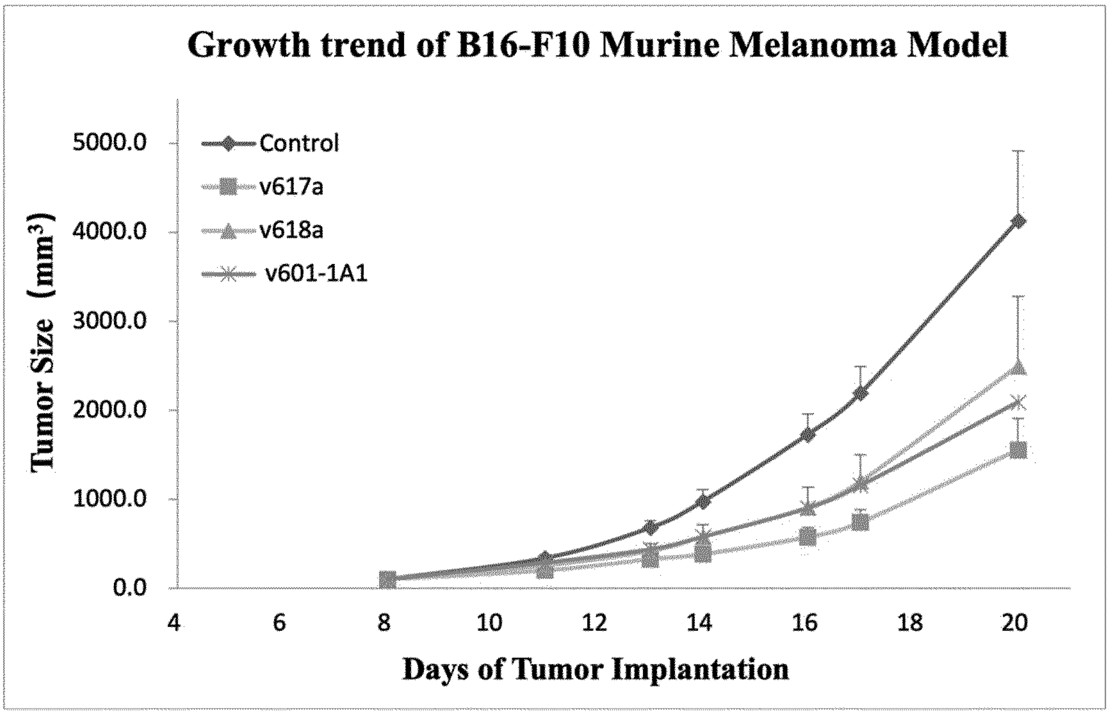
Growth trend of B16-F10 Murine Melanoma Model
Control
v617a
v618a
v601-1A1
Tumor Size (mm³)
5000.0
4000.0
3000.0
2000.0
1000.0
0.0
4
6
8
10
12
14
16
18
20
Days of Tumor Implantation

Figure 17

**INTERNATIONAL SEARCH REPORT**

International application No. **PCT/CN2021/112408**

**A. CLASSIFICATION OF SUBJECT MATTER**

C12N 15/863(2006.01)i; A61K 35/768(2015.01)i; A61P 35/00(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS, DWPI, SIPOABS, CNKI, ISI web of Science, Genbank, WANFANG: 羊传染性脓疱皮炎病毒, 羊口疮, 溶瘤病毒, 缺失, 失活, 肿瘤, 趋化因子结合蛋白, orf virus, ORFV, oncolytic virus, delete, inactive, tumor, cancer, chemokine-binding protein, CBP, orf111, orf112, applicant/inventor

**C. DOCUMENTS CONSIDERED TO BE RELEVANT**

| Category* | Citation of document, with indication, where appropriate, of the relevant passages | Relevant to claim No. |
|---|---|---|
| X | FLEMING, S.B. "Deletion of the Chemokine Binding Protein Gene from the Parapoxvirus Orf Virus Reduces Virulence and Pathogenesis in Sheep" *FRONTIERS IN MICROBIOLOGY,* Vol. 8, 24 January 2017 (2017-01-24), article number 46, abstract, main body, pages 2-4, 9-10 | 1-11, 15-18 |
| Y | FLEMING, S.B. "Deletion of the Chemokine Binding Protein Gene from the Parapoxvirus Orf Virus Reduces Virulence and Pathogenesis in Sheep" *FRONTIERS IN MICROBIOLOGY,* Vol. 8, 24 January 2017 (2017-01-24), article number 46, abstract, main body, pages 2-4, 9-10 | 12-13, 15-21 |
| Y | WO 2017165366 A1 (SOUTH DAKOTA BOARD OF REGENTS et al.) 28 September 2017 (2017-09-28) claims 1-3 | 12-13, 15-21 |
| Y | CN 108026542 A (EBERHARD KARLS UNIVERSITAET TUEBINGEN MEDIZINISCHE FAKULTAET) 11 May 2018 (2018-05-11) claims 1-2, 7, 9-13, description paragraph 94 | 12-13, 15-21 |
| Y | CN 108770344 A (HU, Minjie et al.) 06 November 2018 (2018-11-06) description, paragraphs 34-35, 41 | 13, 15-21 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 October 2021** | **11 November 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China**<br>Facsimile No. **(86-10)62019451** | Authorized officer<br><br>Telephone No. |

Form PCT/ISA/210 (second sheet) (January 2015)

INTERNATIONAL SEARCH REPORT

International application No.
PCT/CN2021/112408

C. DOCUMENTS CONSIDERED TO BE RELEVANT

| Category* | Citation of document, with indication, where appropriate, of the relevant passages | Relevant to claim No. |
|---|---|---|
| A | CN 108220251 A (SOUTHERN MEDICAL UNIVERSITY) 29 June 2018 (2018-06-29)<br>entire document | 1-21 |
| A | CN 103260644 A (AH USA 42 LLC) 21 August 2013 (2013-08-21)<br>entire document | 1-21 |
| A | 闫丰超 等 (YAN, Fengchao et al.). "羊口疮病毒分子生物学的研究进展 (Progress in Molecular Biology of Orf Virus)"<br>*中国兽医科学 (Chinese Veterinary Science),*<br>Vol. 43, No. 1, 31 December 2013 (2013-12-31),<br>pp. 103-109 | 1-21 |

Form PCT/ISA/210 (second sheet) (January 2015)

**INTERNATIONAL SEARCH REPORT**

International application No.
**PCT/CN2021/112408**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
**Information on patent family members**

International application No.
**PCT/CN2021/112408**

| Patent document cited in search report | Publication date (day/month/year) | Patent family member(s) | Publication date (day/month/year) |
|---|---|---|---|
| WO 2017165366 A1 | 28 September 2017 | EP 3432922 A1 | 30 January 2019 |
| | | US 2020188509 A1 | 18 June 2020 |
| | | US 11013798 B2 | 25 May 2021 |
| | | EP 3432922 B1 | 19 February 2020 |
| | | BR 112018069371 A2 | 22 January 2019 |
| CN 108026542 A | 11 May 2018 | MX 2018000845 A | 16 August 2018 |
| | | JP 2018523491 A | 23 August 2018 |
| | | AU 2020264302 A1 | 26 November 2020 |
| | | CL 2018000164 A1 | 24 August 2018 |
| | | AU 2016294855 B2 | 06 August 2020 |
| | | PH 12018500155 A1 | 23 July 2018 |
| | | KR 20210064427 A | 02 June 2021 |
| | | EA 201890351 A1 | 29 June 2018 |
| | | WO 2017013023 A1 | 26 January 2017 |
| | | KR 20180058705 A | 01 June 2018 |
| | | US 2018148739 A1 | 31 May 2018 |
| | | DE 102015111756 A1 | 26 January 2017 |
| | | AU 2016294855 A1 | 15 February 2018 |
| | | EP 3325631 A1 | 30 May 2018 |
| | | CA 2993263 A1 | 26 January 2017 |
| | | BR 112018001121 A2 | 11 September 2018 |
| | | NZ 739286 A | 25 October 2019 |
| | | IL 257025 D0 | 29 March 2018 |
| | | JP 2020188776 A | 26 November 2020 |
| | | KR 102259277 B1 | 01 June 2021 |
| CN 108770344 A | 06 November 2018 | US 2019000899 A1 | 03 January 2019 |
| | | EP 3436065 A1 | 06 February 2019 |
| | | CA 3019546 A1 | 05 October 2017 |
| | | AU 2017241684 A1 | 11 October 2018 |
| | | EP 3436065 A4 | 04 December 2019 |
| | | WO 2017172713 A8 | 09 November 2017 |
| | | AU 2017241684 B2 | 21 May 2020 |
| | | JP 2019515948 A | 13 June 2019 |
| | | WO 2017172713 A1 | 05 October 2017 |
| CN 108220251 A | 29 June 2018 | None | |
| CN 103260644 A | 21 August 2013 | ZA 201208264 B | 29 January 2014 |
| | | AU 2011254315 A1 | 10 January 2013 |
| | | CO 6670515 A2 | 15 May 2013 |
| | | JP 5890399 B2 | 22 March 2016 |
| | | UY 33400 A | 30 December 2011 |
| | | CL 2012003219 A1 | 05 April 2013 |
| | | KR 20130008645 A | 22 January 2013 |
| | | CA 2798055 A1 | 24 November 2011 |
| | | RU 2012149036 A | 27 June 2014 |
| | | MX 338052 B | 31 March 2016 |
| | | WO 2011145013 A1 | 24 November 2011 |
| | | BR 112012029633 A2 | 20 September 2016 |
| | | CA 2798055 C | 24 November 2015 |
| | | US 2011287051 A1 | 24 November 2011 |
| | | AR 081409 A1 | 29 August 2012 |

Form PCT/ISA/210 (patent family annex) (January 2015)

**INTERNATIONAL SEARCH REPORT**
**Information on patent family members**

International application No.
**PCT/CN2021/112408**

| Patent document cited in search report | Publication date (day/month/year) | Patent family member(s) | | | Publication date (day/month/year) |
|---|---|---|---|---|---|
| | | NZ | 603431 | A | 27 June 2014 |
| | | AU | 2011254315 | B2 | 14 May 2015 |
| | | KR | 20150015551 | A | 10 February 2015 |
| | | EP | 2571521 | A1 | 27 March 2013 |
| | | MX | 2012013451 | A | 22 January 2013 |
| | | RU | 2545694 | C2 | 10 April 2015 |
| | | JP | 2013535953 | A | 19 September 2013 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202010813096
- CN 104017776 B
- CN 108026542 A
- WO 2012122649 A1
- CN 108220251 A
- CN 104878043 B
- CN 103952377 A
- WO 2012122649 A

### Non-patent literature cited in the description


- **HANAHAN, D. ; WEINBERG, R.A.** Hallmarks of cancer: the next generation. *Cell,* 2011, vol. 144 (5), 646-74
- **MIEST, T.S. ; CATTANEO, R.** New viruses for cancer therapy: meeting clinical needs. *Nat Rev Microbiol,* 2014, vol. 12 (1), 23-34
- **BURKE, J. ; NIEVA, J. ; BORAD, M.J. ; BREITBACH, C.J.** Oncolytic viruses: perspectives on clinical development. *Curr Opin Virol,* 2015, vol. 13, 55-60
- **SEYMOUR, L.W. ; FISHER, K.D.** Oncolytic viruses: finally delivering. *Br J Cancer,* 2016, vol. 114 (4), 357-361
- **HARRINGTON, K.J. ; PUZANOV, I. ; HECHT, J.R. ; HODI, F.S. ; SZABO, Z. ; MURUGAPPAN, S. ; KAUFMAN, H.L.** Clinical development of talimogene Laherparepvec (T-VEC): a modified herpes simplex virus type-1-derived oncolytic immunotherapy. *Expert Rev Anticancer Ther,* 2015, vol. 15 (12), 1389-1403
- **YIN, Z. ; LIU, J.H.** Animal Virology. Science Press, 1997, 977-978
- **WANG, Z.J.** Research, development and quality control of Biopharmaceuticals. Science Pres, 2018
- **CDC.** Orf Virus Infection in Humans-New York, Illinois, California, and Tennessee, 2004-2005. *Morbidity and Mortality Weekly Report,* 2006, vol. 55 (3), 65-68
- **KIRN, D.H. ; THORNE, S.H.** Targeted and armed oncolytic poxviruses: a novel multimechanistic therapeutic class for cancer. *Nat Rev Cancer,* 2009, vol. 9, 64-71
- **MCFADDEN, G.** Poxvirus tropism. *Nat Rev Microbiol,* 2005, vol. 3 (3), 201-213
- **WANG, R. ; WANG, Y. ; LIU, F. ; LUO, S.** Orf virus: A promising new therapeutic agent. *Rev Med Virol,* 2018, e2013
- **RINTOUL, J.L. ; LEMAY, C.G. ; TAI, L.H. ; STANFORD, M.M. ; FALLS, T.J. ; BRIDLE, B.W. ; SOUZA, C.T. ; DANESHMAND, M. ; OHASHI, P.S. ; WAN, Y.** ORFV: a novel oncolytic and immune stimulating parapoxvirus therapeutic. *Mol Ther,* 2012, vol. 20 (6), 1148-1157
- **SEET, B.T. ; MCCAUGHAN, C.A. ; HANDEL, T.M. ; MERCER, A. ; BRUNETTI, C. ; MCFADDEN, G. ; FLEMING, S.B.** Analysis of an orf virus chemokine-binding protein: shifting ligand specificities among a family of poxvirus viroceptors. *Proceedings of the National Academy of Sciences,* 2003, vol. 100 (25), 15137-15142
- **BERGQVIST, C. ; KURBAN, M. ; ABBAS, O.** Orf virus infection. *Rev Med Virol,* 2017, vol. 27 (4
- **LIU, W. ; YANG, K.K. ; YIN, D.D. ; WANG, Y.H. ; YU, Z.R. ; JIANG, S.D. ; LI, C.F. ; LI, Y.D. ; WANG, Y.** Prokaryotic expression and subcellular localization of dUTPase gene of orf virus. *Chinese Veterinary Science,* 2018, vol. 7, 818-823
- **IRWIN, C.R. ; HITT, M.M. ; EVANS, D.H.** Targeting Nucleotide Biosynthesis: A Strategy for Improving the Oncolytic Potential of DNA Viruses. *Front Oncol,* 2017, vol. 7, 229
- **FLEMING, S.B. ; MCCAUGHAN, C. ; LATEEF, Z. ; DUNN, A. ; WISE, I.M. ; REAL, N.C. ; MERCER, A.A.** Deletion of chemokine binding protein gene from the parapoxvirus orf virus reduces virulence and pathogenesis in sheep. *Front Microbiol,* 2017, vol. 8, 46
- **TWUMASI-BOATENG, K. ; JESSICA, L.P. ; EUNICE KWOK, Y.Y. ; JOHN, C.B. ; NELSON, B.H.** Oncolytic viruses as engineering platforms for combination immunotherapy. *Nat Rev Cancer,* 2018, vol. 18, 419-432
- **DIEL, D.G. ; LOU, S. ; DELHON, G. ; PENG, Y. ; FLORES, E.F. ; ROCK, D.L.** A nuclear inhibitor of NF-kappaB encoded by a poxvirus. *J Virol,* 2011, vol. 85 (1), 264-275

- **SON, S.J. ; HARRIS, P.W. ; SQUIRE, C.J. ; BAKER, E.N. ; KENT, S.B. ; BRIMBLE, M.A.** Total Chemical Synthesis of an Orf Virus Protein, ORFV002, an Inhibitor of the Master Gene Regulator NF-κB. Biopolymers. *Peptid Science,* 2014, vol. 102 (2), 137-144
- **WANG, G.X. ; SHANG, Y.J. ; CHEN, J.T. ; LU, Z.L. ; ZHANG, K.S. ; LIU, X.T.** Isolation and identification of orf virus in Hubei Province. *Advances in Animal Medicine,* 2012, vol. 033 (011), 37-40